# EUROPEAN PATENT SPECIFICATION

(11) **EP 0 954 374 B1**
(45) Date of publication and mention of the grant of the patent: **24.11.2004**
(21) Application number: 98903445.9
(22) Date of filing: 06.01.1998
(51) Int. Cl.: A61L 2/00, B01J 20/28

(54) **ADSORBENS AND DEVICES FOR THE REDUCTION OF SMALL ORGANIC COMPOUNDS FROM BLOOD PRODUCTS**
ADSORBENTIEN UND GERÄTE ZUR VERMINDERUNG VON KLEINEN ORGANISCHEN VERBINDUNGEN AUS BLUTPRODUKTEN
ADSORBANTS ET DISPOSITIFS PERMETTANT DE REDUIRE DE PETITS COMPOSES ORGANIQUES PRESENTS DANS DES PRODUITS SANGUINS

(30) Priority: 06.01.1997 US 779885; 07.01.1997 US 779830
(43) Date of publication of application: 10.11.1999
(73) Proprietor: Cerus Corporation, Concord, CA 94520 (US)
(72) Inventor: HEI, Derek, Joseph, Concord, CA 94521 (US); CLARKE, Michael, S., San Francisco, CA 94118 (US); PHAN, Thu, Anh, Walnut Creek, CA 94596 (US)
(74) Representative: Roques, Sarah Elizabeth
(86) International application number: PCT/US1998/000531
(87) International publication number: WO 1998/030327

(56) References cited:
- EP-A- 0 099 586
- EP-A- 0 366 946
- EP-A- 0 776 668
- WO-A-96/39818
- WO-A-96/40857
- WO-A-97/37536

## Description

### TECHNICAL FIELD

The present invention relates to devices for the reduction of small organic compounds from blood products.

### BACKGROUND ART

An extensive body of research exists regarding the removal of substances from blood products. The bulk of this research is directed at white cell reduction. *See, e.g.,* M.N. Boomgaard *et al*., *Transfusion* 34:311 (1994); F. Bertolini *et al., Vox Sang* 62:82 (1992); and A.M. Joustra-Dijkhuis *et al*., *Vox Sang* 67:22 (1994). Filtration of platelets is the most common method used in white cell reduction of platelet concentrates. *See*, *e*.*g*., M. Böck *et al*., *Transfusion* 31:333 (1991) (Sepacell PL-5A, Asahi, Tokyo, Japan); J.D. Sweeney *et al*., *Transfusion* 35:131 (1995) (Leukotrap PL, Miles Inc.. Covina, CA); and M. van Marwijk *et al*., *Transfusion* 30:34 (1990) (Cellselect, NPBI, Emmer-Compascuum, The Netherlands; Immugard Ig-500, Terumo, Tokyo, Japan). These current filtration mechanisms, however are not amenable for the removal of relatively low molecular weight compounds including for example psoralens, photoaddition products and other compounds commonly used in treating biological fluids.

The process of adsorption has been used to isolate selective blood components onto phospholipid polymers. For example, several copolymers with various electrical charges have been evaluated for their interactions with blood components, including platelet adhesion and protein adsorption. K. Ishihara *et al*., *J. Biomed. Mat. Res.* 28:1347 (1994). Such polymers, however, are not designed for the adsorption of low molecular weight compounds.

Various dialysis means are able to remove low molecular weight compounds from plasma and whole blood. For example, dialysis can successfully remove low molecular weight toxins and pharmaceutical compounds. Thus, dialysis might be used to remove, for example, psoralens and psoralen photoproducts from blood products. Unfortunately, current dialysis procedures involve very complicated and expensive devices. As such, the use of dialysis machines would not be practical for the decontamination of a large volume of blood products.

The use of polystyrene divinylbenzene, silica gel, and acrylester polymers for the adsorption of methylene blue has previously been described. For example, PCT publication No. WO 91/03933 describes batch studies with free adsorbent resin (e.g., Amberlites (Rohm and Haas (Frankfurt, Germany) and Bio Beads (Bio-Rad Laboratories (Munich, Germany)). Without very careful removal of the adsorbent resins after exposure to the blood product, however, these methods create the risk of transfusion of the resin particles.

In addition, devices and processes for the removal of leukocytes and viral inactivation agents (e.g. psoralens, hypericin, and dyes such as methylene blue, toluidine blue, and crystal violet) have also been disclosed. Specifically, PCT Publication No. WO 95/18665 describes a filter comprising a laid textile web which incudes a mechanically stable polymeric substrate. The web itself comprises interlocked textile fibers forming a matrix with spaces and fibrillated particles disposed within the spaces. However, this device causes a significant decrease in the Factor XI activity.

WO 96/39818 discloses the use of a compound having a nucleic acid binding ligand and a mustard group to inactivate pathogens in a biological material. After treatment removal of the chemical product may be carried out using an adsorbent material.

Simpler, safer and more economical means for reducing the concentration of small organic compounds in a biological fluid while substantially maintaining the biological activity of the purified fluid are therefore needed.

### DISCLOSURE OF THE INVENTION

The present invention provides an adsorbent medium for reducing the concentration of a small organic compound in a blood product while substantially maintaining a desired biological activity of the blood product comprising (1) highly porous adsorbent particles capable of adsorbing the small organic compound, and (2) a sintered polymeric matrix in which the adsorbent particles are immobilized.

Immobilisation of adsorbent particles in an inert matrix for reducing the concentration of small organic compounds in blood products has several benefits and advantages over existing systems. One advantage is the reduction in leakage of loose adsorbent particles into blood products which in turn produces a safer blood product. Another advantage is the enhanced ability of the adsorbent particles to adsorb small organic compounds due in part to the elimination of clumping problems associated with non-immobilised particles when used with blood products, for example, materials comprising red blood cells. A further advantage is the reduction in problems associated with handling loose adsorbent particles; thereby, simplifying manufacturing. A surprising benefit of using adsorbent particles immobilised by an inert matrix is the reduction in damage to stored membrane containing blood products, for example platelets, red blood cells, and white blood cells.

In one embodiment, the adsorbent particles have a surface area greater than about 750 m²/g. In another embodiment, the adsorbent particles may have a surface area greater than about 1000 m²/g.

In one embodiment the device is a flow device wherein the adsorbent particles are less than about 200 µm in diameter and greater than 10 µm in diameter. In an alternative embodiment the device is a batch device wherein the adsorbent particles are less than about 1200 µm in diameter and greater than 300 µm in diameter.

The present invention contemplates that the adsorbent particles comprise polyaromatic compounds or activated carbon. In one embodiment, the polyaromatic compounds are included in hypercrosslinked, polystyrene divinylbenzene copolymer networks. In one embodiment, the activated carbon may be derived from a natural source. In another embodiment, the activated carbon may be derived from a synthetic source, resulting from treating highly sulfonated polystyrene beads with a high temperature activation process.

Further contemplated is a device which reduces the concentration of acridines, acridine derivatives, methylene blue or thiols in a blood product. Also contemplated is a device which reduces the concentration of psoralens, psoralen derivatives, including, for example 4'-(4-amino-2-oxa)butyl-4,5',8-trimethyl psoralen, isopsoralens or aminopsoralens. The blood product may be selected from the group consisting of platelets, red blood cells or plasma.

As an additional component, the invention contemplates that the device further comprises a blood bag.

The adsorbent medium and devices of the invention may be used in a method for reducing the concentration of cyclic compounds in a blood product while substantially maintaining a desired biological activity of the blood product, the method comprising the steps of: a) contacting the blood product with the devices of the present invention and b) removing the blood product from contact with the devices of the present invention.

Further contemplated is a device which reduces the concentration of acridines, acridine derivatives, methylene blue of thiols in a blood product. Also contemplated is a device which reduces the concentration of psoralens, psoralen derivatives, including, for example 4'-(4-amino-2-oxa)butyl-4,5',8-trimethyl psoralen, isopsoralens or aminopsoralens. The blood product may be selected from the group consisting of platelets, red blood cells or plasma.

As an additional component, the invention contemplates that the device further comprises a blood bag.

We also describe a method of inactivating pathogens in solution, wherein the method comprises: a) providing, in any order: i) a cyclic compound, ii) a solution suspected of being contaminated with the pathogens, and iii) fiberized resin; b) treating the solution with the cyclic compound so as to create a treated solution product wherein the pathogens are inactivated; and c) contacting the treated solution product with the fiberized resin, the improvement comprising a device for reducing the concentration of small organic compounds in a blood product while substantially maintaining a desired biological activity of the blood product, the device comprising highly porous adsorbent particles, and wherein the adsorbent particles, and wherein the adsorbent particles are immobilized by an inert matrix.

We also describe a method of reducing the concentration of cyclic compounds in solution, comprising: a) providing i) cyclic compounds in solution at a concentration, the cyclic compounds selected from the group consisting of monocyclic compounds, polycyclic compounds, and heterocyclic compounds, and ii) fiberized resin; and b) contacting the cyclic compounds with the fiberized resin, thereby reducing the concentration of the cyclic compounds.

We also describe a blood bag, comprising: a) a biocompatible housing; and b) resin immobilized in a fiber network, the immobilized resin contained within the biocompatible housing.

### BRIEF DESCRIPTION OF THE DRAWINGS

FIG. 1 diagrammatically depicts a perspective view of one embodiment of a fiber, indicating its inner core and outer sheath, that forms the fiber networks of the fiberized resin.
FIG. 2 schematically represents a portion of one embodiment of the fiberized resin of the present invention.
FIG. 3 diagrammatically represents a cross-sectional view of one embodiment of fiberized resin in which the adsorbent beads are secured to fibers that make up the fiberized resin.
FIG. 4 diagrammatically represents a cross-sectional view of one embodiment of fiberized resin in which the adsorbent beads are immobilized within the fibers of the fiberized resin and the heat seals that encompass samples of fiberized resin.
FIG. 5 is a graph showing a comparison of adsorption kinetics for removal of aminopsoralens from platelets with Dowex® XUS-43493 and Amberlite® XAD-16 HP loose adsorbent beads and fiberized resin containing Amberlite® XAD-16.
FIG. 6 is a graph showing a comparison of adsorption kinetics for removal of aminopsoralens from platelets with fiberized resin containing Amberlite® XAD-16 and fiberized resin with the two different loadings of activated charcoal.
FIG. 7 is a graph showing a comparison of the adsorption kinetics for removal of aminopsoralens from platelets with p(HEMA)-coated and uncoated Dowex® XUS-43493 beads.
FIG. 8 is a graph showing a comparison of the effect of pre-treatment solution glycerol content on relative aminopsoralens adsorption capacity for Amberlite® XAD-16 and Dowex® XUS-43493.
FIG. 9 is a graph showing a comparison of the effect of wetting solution on 4'-(4-amino-2-oxa)butyl-4,5',8-trimethyl psoralen adsorption capacities for dried adsorbent in 100% plasma for Amberlite® XAD-16 (bottom) and Dowex® XUS-43493 (top); the samples that were not wet in an ethanol solution are labeled "No Tx". Adsorbent capacities are reported as percentages relative to the capacity of optimally wet adsorbent.
FIG. 10 is a graph showing a comparison of adsorption of aminopsoralens over a 3-hour period from plasma using Amberlite® XAD-16 wet in several different solutions.
FIG. 11 is a graph showing a comparison of the kinetics of adsorption of methylene blue over a 2-hour period from plasma.
FIG. 12 depicts the chemical structures of acridine, acridine orange, 9-amino acridine, and 5-[(β-carboxyethyl)amino]acridine.
FIG. 13 is a graph showing plots the data for adenine capacity (y-axis) and 5-[(β-carboxyethyl)amino]acridine capacity (x-axis) for various resins.
FIGS. 14A and 14B is a graph showing a comparison of the adsorption kinetics for removal of 5-[(β-carboxyethyl)amino]acridine with Dowex® XUS-43493 and Purolite® MN-200 and Amberlite® XAD-16 HP.
FIG. 15 is a graph showing a comparison of the adsorption kinetics for removal of 9-amino acridine and acridine orange with Dowex® XUS-43493.
FIG. 16 is an illustration of flow and batch conFIGurations for the immobilized adsorption device (IAD).
FIG. 17 is a graph showing a comparison of the adsorption isotherms for various Ambersorbs as compared to Purolite MN-200.
FIG. 18 is a graph showing a comparison of the levels of 5-[(β-carboxyethyl)amino]acridine and GSH in the supernatant of 300 mL PRBC units with continued or terminated exposure after 24 hours to a fiberized Pica G277 IAD (500 g/m²) over 4 weeks of storage at 4°C.
FIG. 19 is a graph showing the effect of enclosure material on 5-[(β-carboxyethyl)amino]acridine in PRBC exposed to IADs.
FIG. 20 is a graph showing a comparision of percent hemolysis for the non-immobilized and immobilized adsorbent particle Purolite MN-200.
FIG. 21 is a graph showing a comparision of percent hemolysis for the non-immobilized and immobilized adsorbent particle Pica G-277 activated carbon.
FIG. 22 is a graph showing kinetics for removal 4'-(4-amino-2-oxa) butyl-4,5',8 trimethylpsoralen from platelet concentrates.

### BEST MODE FOR CARRYING OUT THE INVENTION

The present invention provides for materials and devices for reducing the concentration of small organic compounds from a treated blood product. The small organic compounds can include cyclic and acyclic compounds. Exemplary compounds include pathogen inactivating compounds, dyes and thiols. Devices are provided that comprise a three dimensional network of adsorbent particles immobilized by an inert matrix. This immobilization reduces the risk of leakage of loose adsorbent particles into the blood product. Furthermore, immobilization of the adsorbent particles by an inert matrix simplifies manufacturing by reducing problems associated with handling loose adsorbent particles. Immobilization of the adsorbent particles also enhances the ability of the adsorbent particles to adsorb small organic compounds in compositions comprising red blood cells, and or platelets. and or plasma. Finally, using adsorbent particles immobilized by an inert matrix reduces damage to compositions comprising blood products containing cellular membranes, for example platelets, red blood cells, and white blood cells.

### DEFINITIONS

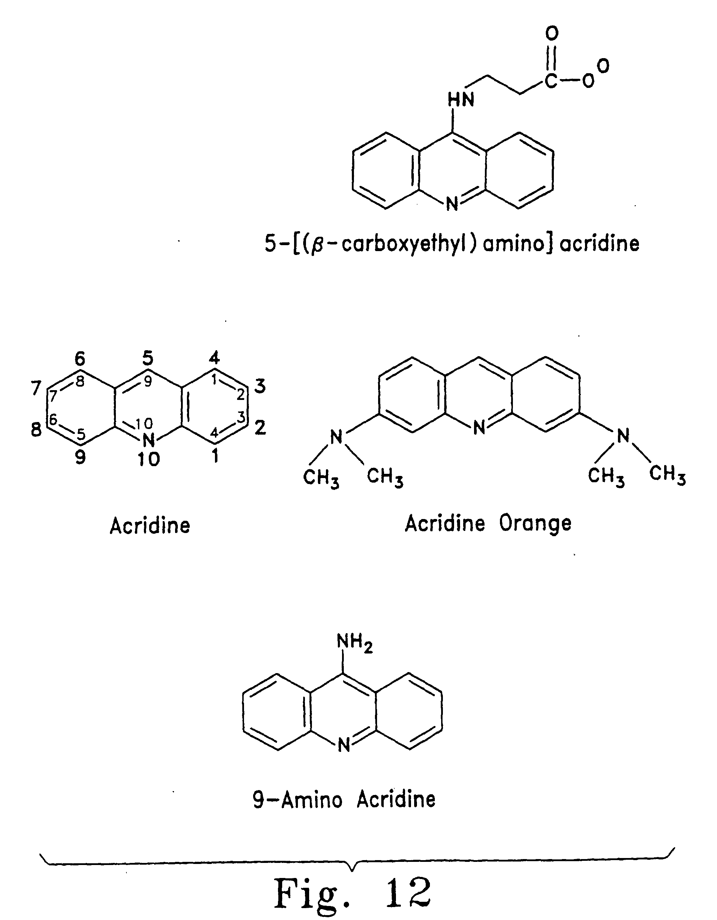

The term "acridine derivatives" refer to a chemical compound containing the tricyclic structure of acridine (dibenzo[*b*,*e*]pyridine; 10-azanthracene). The compounds have an affinity for (and can bind) to nucleic acids non-covalently through intercalation. The term "aminoacridine" refers to those acridine compounds with one or more nitrogen-containing functional groups. Examples of aminoacridines include 9-amino acridine and acridine orange {depicted in Figure 12).

The term "adsorbent particle" broadly refers to any natural or synthetic material which is capable of interacting with molecules in a liquid thus allowing the molecule to be removed from the liquid. Examples of naturally occurring adsorbents include but are not limited to activated carbon, silica, diatomaceous earth, and cellulose. Examples of synthetic adsorbents include but are not limited to polystyrene, polyacrylics, and carbonaceous adsorbents. Adsorbent particles are often porous, often possess high surface areas, and may be modified with a variety of functional groups (e.g. ionic, hydrophobic, acidic, basic) which can effect how the adsorbent interacts with molecules.

The term "aromatic," "aromatic compounds," and the like refer broadly to compounds with rings of atoms having delocalized electrons. The monocyclic compound benzene (C₆H₆) is a common aromatic compound. However, electron delocalization can occur over more than one adjacent ring (*e.g*., naphthalene (two rings) and anthracene (three rings)). Different classes of aromatic compounds include, but are not limited to, aromatic halides (aryl halides), aromatic heterocyclic compounds, aromatic hydrocarbons (arenes), and aromatic nitro compounds (aryl nitro compounds).

The term "biocompatible coating" refers broadly to the covering of a surface (*e*.*g*., the surface of a polystyrene bead) with a hydrophilic polymer that when in contact with a blood product does not result in an injurious, toxic, or immunological response and renders the surface more biocompatible by decreasing cell adhesion, protein adsorption or improves cell function. Suitable coatings are biocompatible if they have minimal, if any, effect on the biological material to be exposed to them. By "minimal" effect it is meant that no significant difference is seen compared to the control. In preferred embodiments, biocompatible coatings improve the surface hemocompatibility of polymeric structures. For example, poly(2-hydroxyethyl methacrylate) (pHEMA) is frequently used for the coating of materials used in medical devices (*e*.*g*., blood filters).

The term "biocompatible housing" refers broadly to filter housings, containers, bags, vessels, receptacles, and the like that are suitable for containing a biological material, such as whole blood, platelet concentrates and plasma. Suitable containers are biocompatible if they have minimal, if any, effect on the biological material to be contained therein. By "minimal" effect it is meant that no significant difference is seen in blood product function compared to the control as decribed herein, for red blood cells, platelets and plasma. Thus, blood products may be stored in biocompatible housings prior to transfusion to a recipient. In a preferred embodiments, a biocompatible housings are blood bags, including a platelet storage container.

The term " biological fluids" include human or non-human whole blood, plasma, platelets, red blood cells, leukocytes, serum, lymph, saliva, milk, urine, or products derived from or containing any of the above, alone or in mixture, with or without a chemical additive solution. Preferably, the fluid is blood or a blood product with or without a chemical additive solution, more preferably plasma, platelets and red blood cells, most preferably apheresis plasma and red blood cells.

The term "blood bag" refers to a blood product container.

The term "blood product" refers to the fluid and/or associated cellular elements and the like (such as erythrocytes, leukocytes, platelets, etc.) that pass through the body's circulatory system; blood products include, but are not limited to, blood cells, platelet mixtures, serum, and plasma. The term "platelet mixture" refers to one type of blood product wherein the cellular element is primarily or only platelets. A platelet concentrate (PC) is one type of platelet mixture where the platelets are associated with a smaller than normal portion of plasma. In blood products synthetic media may make up that volume normally occupied by plasma; for example, a platelet concentrate may entail platelets suspended in 35% plasma/65% synthetic media. Frequently, the synthetic media comprises phosphate.

The term "blood separation means" refers broadly to a device, machine, or the like that is able to separate blood into blood products (*e.g*., platelets and plasma). An apheresis system is one type of blood separation means. Apheresis systems generally comprise a blood separation device, an intricate network of tubing and filters, collection bags, an anticoagulant, and a computerized means of controlling all of the components.

The term "crosslinked" refers broadly to linear molecules that are attached to each other to form a two- or three-dimensional network. For example, divinylbenzene (DVB) serves as the crosslinking agent in the formation of styrene-divinylbenzene copolymers. The term also encompasses "hypercrosslinking" in which hypercrosslinked networks are produced by crosslinking linear polystyrene chains either in solution or in a swollen state with bifunctional agents. A variety of bifunctional agents can be used for cross-linking (for example, see Davankov and Tsyurupa, Reactive Polymers 13:24-42 (1990); Tsyurupa et al., Reactive Polymers 25:69-78 (1995).

The term "cyclic compounds" refers to compounds having one (*i*.*e*., a monocyclic compounds) or more than one (*i.e.*, polycyclic compounds) ring of atoms. The term is not limited to compounds with rings containing a particular number of atoms. While most cyclic compounds contain rings with five or six atoms, rings with other numbers of atoms (*e.g*., three or four atoms) are also contemplated by the present invention. The identity of the atoms in the rings is not limited, though the atoms are usually predominantly carbon atoms. Generally speaking, the rings of polycyclic compounds are adjacent to one another; however, the term "polycyclic" compound includes those compounds containing multiple rings that are not adjacent to each other.

The term "dye" refers broadly to compounds that impart color. Dyes generally comprise chromophore and auxochrome groups attached to one or more cyclic compounds. The color is due to the chromophore, while the dying affinities are due to the auxochrome. Dyes have been grouped into many categories, including the azin dyes (*e*.*g*., neutral red, safranin, and azocarmine B); the azo dyes: the azocarmine dyes; the dephenymethane dyes, the fluorescein dyes, the ketonimine dyes, the rosanilin dyes, and the triphenylmethane dyes. It is contemplated that the methods and devices of the present invention may be practiced in conjunction with any dye that is a cyclic compound.

The term "fiberized resin" generally refers to immobilization of adsorbent material, including for example, resins, in, on or entrapped to a fiber network. In one embodiment, the fiber network is comprised of polymer fibers. In another embodiment, the fibers consist of a polymer core (*e.g.*, polyethylene terephthalates [PET]) with a high melting point surrounded by a polymer sheath (*e.g*., nylon or modified PET) with a relatively low melting temperature. Fiberized resin may be produced by heating the fiber network, under conditions that do not adversely affect the adsorbent capacity of the resin to a significant degree. Where the resin comprises beads, heating is performed such that the adsorbent beads become attached to the outer polymer sheath to create "fiberized beads". By producing fiberized resin containing a known amount of adsorbent beads per defined area, samples of fiberized resin for use in the removal of cyclic compounds (*e.g.*, psoralens, and, in particular, aminopsoralens) and other products can be obtained by cutting a defined area of the fiberized resin, rather than weighing the adsorbent beads.

The term "filter" refers broadly to devices, materials, and the like that are able to allow certain components to a mixture to pass through while retaining other components. For example, a filter may comprise a mesh with pores sized to allow a blood product (*e*.*g*., plasma) to pass through, while retaining other components such as resin particles. The term "filter" is not limited to the means by which certain components are retained.

The term "flow adapter" refers to a device that is capable of controlling the flow of a particular substance like a blood product. The flow adapter may perform additional functions, such as preventing the passage of pieces of adsorbent resin material.

The term "heterocyclic compounds" refers broadly to cyclic compounds wherein one or more of the rings contains more than one type of atom. In general, carbon represents the predominant atom, while the other atoms include, for example, nitrogen, sulfur, and oxygen. Examples of heterocyclic compounds include furan, pyrrole, thiophene, and pyridine.

The phrase "high temperature activation process" refers to a high temperature process that typically results in changes in surface area, porosity and surface chemistry of the treated material due to pyrolysis and/or oxidation of the starting material.

The term "immobilization adsorption device (IAD)" refers to immobilized adsorbent material in, on or entrapped to an inert matrix. Where the inert matrix is a fiber network the term IAD can be used interchangeably with the term fiberized resin.

The term "inert matrix" refers to any synthetic or naturally occurring fiber or polymeric material which can be used to immobilize adsorbent particles without substantially effecting the desired biological activity of the blood product. The matrix may contribute to the reduction in concentration of small organic compounds although typically it does not contribute substantially to the adsorption or removal process. In addition, the inert matrix may interact with cellular or protein components resulting in cell removal (e.g. leukodepletion) or removal of protein or other molecules.

The term "in-line column" refers to a container, usually cylindrically shaped, having an input end and an output end and containing a substance disposed therein to reduce the concentration of small organic compounds from a blood product.

The term "isolating" refers to separating a substance out of a mixture containing more than one component. For example, platelets may be separated from whole blood. The product that is isolated does not necessarily refer to the complete separation of that product from other components.

The term "macropores" generally means that the diameter of the pores is greater than about 500 Å. The term micropores refers to pores with diameters less than about 20 Å. The term mesopores refers to pores with diameters greater than about 20 Å, and less than about 500 Å.

The term "macroporous" is used to describe a porous structure having a substantial number of pores with diameters greater than about 500 Å.

The term "macroreticular" is a relative term that means that the structure has a high physical porosity (*i.e.*, a large number of pores are present) a porous adsorbent structure possessing both macropores and micropores.

The term "mesh enclosure," "mesh pouch" and the like refer to an enclosure, pouch, bag or the like manufactured to contain multiple pores. For example, the present invention contemplates a pouch, containing the immobilized adsorbent particle, with pores of a size that allow a blood product to contact the immobilized adsorbent particle, but retain the immobilized adsorbent particle within the pouch.

The term "partition" refers to any type of device or element that can separate or divide a whole into sections or parts. For example, the present invention contemplates the use of a partition to divide a blood bag, adapted to contain a blood product, into two parts. The blood product occupies one part of the bag prior to and during treatment, while the adsorbent resin occupies the other part. In one embodiment, after treatment of the blood product, the partition is removed (*e*.*g*., the integrity of the partition is altered), thereby allowing the treated blood product to come in contact with the adsorbent resin. The partition may either be positioned in the bag's interior or on its exterior. When used with the term "partition," the term "removed" means that the isolation of the two parts of the blood bag no longer exists; it does not necessarily mean that the partition is no longer associated with the bag in some way.

The term "photoproduct" refers to products that result from the photochemical reaction that a psoralen undergoes upon exposure to ultraviolet radiation.

The term "polyaromatic compounds" refers to polymeric compounds containing aromatic groups in the backbone, such as polyethylene terphalate, or as pendant groups, such as polystyrene, or both.

The term "polystyrene network" refers broadly to polymers containing styrene (C₆H₅CH=CH₂) monomers; the polymers may be linear, consisting of a single covalent alkane chain with phenyl substituents, or cross-linked, generally with *m*- or *p*-phenylene residues or other bifunctional or hyper crosslinked structure, to form a two-dimensional polymer backbone.

The term "psoralen removal means" refers to a substance or device that is able to remove greater than about 70% of the psoralen from, *e.g.*, a blood product; preferably, greater than about 90% preferable; most preferably greater than about 99%. A psoralen removal means may also remove other components of the blood product, such as psoralen photoproducts.

The phrase "reducing the concentration" refers to the removal of some portion of the aromatic compounds from the aqueous solution. While reduction in concentration is preferably on the order of greater than about 70%, more preferably on the order of about 90%, and most preferably on the order of about 99%.

The phrase "removing substantially all of said portion of a small organic compound (*e.g*. a psoralen, psoralen derivative, isopsoralen, acridine, acridine derivative, or dye) free in solution" refers preferably to the removal of more than about 80% of the compound free in solution, more preferably to the removal of more than about 85%, even more preferably of more than about 90%, and most preferably to the removal of more than about 99%.

The term "resin" refers to a solid support (such as particles or beads etc.) capable of interacting and attaching to various small organic compounds. including psoralens, in a solution or fluid (*e*.*g*., a blood product), thereby decreasing the concentration of those elements in solution. The removal process is not limited to any particular mechanism. For example, a psoralen may be removed by hydrophobic or ionic interaction (*i.e.,* affinity interaction). The term "adsorbent resin" refers broadly to both natural organic substances and synthetic substances and to mixtures thereof.

The term "shaker device" refers to any type of device capable of thoroughly mixing a blood product like a platelet concentrate. The device may have a timing mechanism to allow mixing to be restricted to a particular duration.

The term "sintered medium" refers to a structure which is formed by applying heat and pressure to a porous plastic, including for example a powdered thermoplastic polymer. Porous plastics can be prepared by mixing powders of relatively low melting polymers and heating them so the plastic particles partially fuse but still allow a path for fluids to penetrate the porous mass. Sintered adsorbent media can be prepared similarly by incorporating carbon or other high or non-melting adsorbent particle with that of the low melting powder and heating. Methods of producing porous plastic materials are described in U.S. Patent Nos. 3,975,481, 4,110,391, 4,460,530, 4,880,843 and 4,925,880. The process causes fusing of the powder particles resulting in the formation of a porous solid structure. The sintered medium can be formed into a variety of shapes by placing the polymeric powder in a forming tool during the sintering process. Adsorbent particles can be introduced into the sintered medium by mixing adsorbent particles with the powdered thermoplastic polymer before subjecting to the sintering process.

The term "stabilizing agent" refers to a compound or composition capable of optimizing the adsorption capacity of certain resins. Generally speaking, acceptable stabilizing agents should be soluble in water and ethanol (or other wetting agents), nonvolatile relative to water and ethanol, and safe for transfusion in small amounts. Examples of stabilizing agents include, but are not limited to, glycerol and low molecular weight PEGs. A "wetting agent" is distinguishable from a "stabilizing agent" in that the former is believed to reopen adsorbent pores of those resins that are not hyper-crosslinked (*i*.*e*., non-macronet resins). Wetting agents generally will not prevent pores from collapsing under drying conditions, whereas stabilizing agents will. A general discussion of wetting and wetting agents is set forth in U.S. Patent No. 5,501,795 to Pall *et al*..

The phrase "substantially maintaining a desired biological activity of the blood product" refers to substantially maintaining properties of a blood product which are believed to be indicative of the potential performance of the product in a therapeutic setting. For example, where red blood cells are concerned, *in vivo* activity is not destroyed or significantly lowered if ATP levels, extracellular potassium leakage, % hemolysis are substantially maintained in red blood cells treated by the methods described herein as compared to untreated samples. For example, the change in ATP level between the treated red blood cell and the untreated red blood cells can be less than about 10%. The change in hemolysis between the treated red blood cells and the untreated red blood cells following storage can be less than about 1%, prefereably less than about 0.8%. The change in extracellular potassium leakage between the treated red blood cells and the untreated red blood cells can be less than about 15%. In the case of plasma, *in vivo* activity is not destroyed or significantly lowered if the level of clotting factors, such as Factors I, II, V, VII, X, XI, or the change in PT and PTT time are substantially maintained in plasma when treated by the methods described herein as compared to untreated samples. For example, the change in level of clotting factors, such as Factors I, II, V, VII, X, XI between the treated plasma and the untreated plasma can be less than about 20%; prefereably less than about 10%. The change in PT and PTT time for the treated plasma compared to the untreated plasma can be, for example, less than about 3 seconds and greater than 1 second; preferably 1.5 seconds. Where platelets are concerned, *in vivo* activity is not destroyed or significantly lowered if, for example, platelet yield, pH, aggregation response, shape change, GMP-140, morphology or hypotonic shock response are substantially maintained in red blood cells treated by the methods described herein as compared to untreated samples. It is further contemplated that the phrase substantially maintained for each of the properties associated with a described blood products may also includes values acceptable to those of ordinary skill in the art as described in the literature, including for example in Klein H.G., ed. Standards for Blood Banks and Transfusion Services, 17^{th} Ed., Bethesda, MD: American Association of Blood Banks, 1996.

The term "thiazine dyes" includes dyes that contain a sulfur atom in one or more rings. The most common thiazine dye is methylene blue [3,7-Bis(dimethylamino)-phenothiazin-5-ium chloride). Other thiazine dyes include, but are not limited to, azure A, azure C and thionine, as descrined *e.g.* in U.S. Patent No. 5,571,666 to Schinazi.

The term "xanthene dyes" refers to dyes that are derivatives of the compound xanthene. The xanthene dyes may be placed into one of three major categories: i) fluorenes or amino xanthenes, ii) the rhodols or aminohydroxyxanthenes, and iii) the fluorones or hydroxyxantheses. Examples of xanthene dyes contemplated for use with the present invention include rose bengal and eosin Y; these dyes may be commercially obtained from a number of sources (*e.g.*, Sigma Chemical Co., St. Louis, MI), and as described *e.g.* in U.S. Patent No. 5,571,666 to Schinazi.

### Adsorbent Particles

Provided are adsorbent particles which are useful in a device for reducing the concentration of small organic compounds in a blood product while substantially maintaining a desired biological activity of the blood product.

The adsorbent particles can be of any regular or irregular shape that lends itself to incorporation into the inert matrix but are preferably roughly spherical. When the IAD is used with a flow device, the particles are greater than about 10 µm in diameter;preferably, the particles are between about 10 µm and about 200 µm in diameter; more preferably, the particles are between about 10 µm and about 100 µm in diameter; most preferably, the particles are between about 10 µm and about 50 µm in diameter. When the 1AD is used with a batch device, the particles are greater than about 300 µm in diameter and less than about 1200 µm in diameter; preferably, the particles are between about 300 µm and about 1200 µm in diameter.

A high surface area is characteristic of the particles. Preferably, the particles have a surface area between about 750 m²/g and about 3000 m²/g. More preferably, the particles have a surface area between about 1000 m²/g and about 3000 m²/g. Most preferably, the particles have a surface area between about 1750 m²/g and about 3000 m²/g.

Adsorbent particles suitable for use in the device of the present invention can be any material on which small organic molecules are adsorbed, with the limitation that the material does not substantially adversely affect the biological activity of a fluid upon contact. The adsorbent particle can be, for example, made of materials such as activated carbon, hydrophobic resins or ion exchange resins.

In one preferred embodiment the adsorbent particles are activated carbons derived either from natural or synthetic sources. Preferably the activated carbons are derived from synthetic sources. Nonlimiting examples of activated carbons include; Picatiff Medicinal, which is available from PICA USA Inc. (Columbus, OH), Norit ROX 0.8, which is available from Norit American, Inc. (Atlanta. GA), Ambersorb 572, which is available from Rohm & Haas (Philadelphia, PA), and G-277, which is available from PICA (Columbus, OH).

In one preferred embodiment the particles are Norit A Supra, which is available from Norit Americas, Inc. (Atlanta, GA). Norit A Supra is a USP-grade activated carbon that is formed by steam activation of lignite. This activated carbon has a very high total surface area (2000 m²/g) and is very microporous in nature.

In another preferred embodiment, the particles can be hydrophobic resins. Nonlimiting examples of hydrophobic resins include the following polyaromatic adsorbents: Amberlite® adsorbents (*e*.*g*., Amberlite® XAD-2, XAD-4, and XAD-16), available from Rohm and Haas (Philadelphia, PA); Amberchrom® adsorbents available from Toso Haas (TosoHass, Montgomeryville, PA); Diaion®//Sepabeads® Adsorbents (*e*.*g*., Diaion® HP20), available from Mitsubishi Chemical America, Inc. (White Plains, NY); Hypersol-Macronet® Sorbent Resins (*e.g*., Hypersol-Macronet® Sorbent Resins MN-200, MN-150 and MN-400) available from Purolite (Bala Cynwyd, PA); and Dowex® Adsorbents (*e*.*g*., Dowex® XUS-40323, XUS-43493, and XUS-40285), available from Dow Chemical Company (Midland, MI).

Preferred particles are hydrophobic resins which are polyaromatic adsorbents comprising a hypercrosslinked polystyrene network, such as Dowex® XUS-43493 (known commercially as Optipore® L493 or V493) and Purolite MN-200.

Hypercrosslinked polystyrene networks, such as Dowex® XUS-43493 and Purolite MN-200 are non-ionic macroporous and macroreticular resins. The non-ionic macroreticular and macroporous Dowex® XUS-43493 has a high affinity for psoralens, including for example, 4'-(4-amino-2-oxa)butyl-4,5',8-trimethyl psoralen, and it possesses superior wetting properties. The phrase "superior wetting properties" means that dry (*i*.*e*. essentially anhydrous) adsorbent does not need to be wet with a wetting agent (*e*.*g*., ethanol) prior to being contacted with the blood product in order for the adsorbent to effectively reduce the concentration of small organic compounds from the blood product.

Hypercrosslinked polystyrene networks, such as Dowex® XUS-43493 and Purolite MN-200 are preferably in the form of spherical particles with a diameter range of about 100 µm to about 1200 µm. For flow devices. the particles have a diameter range from about 10 to about 100 µm. For batch devices, the particles have a diameter range from about 100 to about 1200 µm. Adsorbent particles, including for example, Dowex® XUS-43493, preferably have extremely high internal surface areas and relatively small pores (*e.g.* 46 Å). The internal surface area of the particle can be from about 300 to about 1100 m²/g; preferably 1100 m²/g. The pores size of the particle can be greater than 25Å and less than 800Å; preferably from about 25Å to about 150Å, most preferably from about 25Å to about 50Å. While it is not intended that the present invention be limited to the mechanism by which reduction of small organic compounds takes place, hydrophobic interaction is believed to be the primary mechanism of adsorption. Its porous nature confers selectively on the adsorption process by allowing small molecules to access a greater proportion of the surface area relative to large molecules (*i*.*e*., proteins) and cells. Purolite® has many similar characteristics to Dowex® XUS-43493, such as high affinity for psoralens and superior wetting properties, and is also a preferred adsorbent particle.

Polystyrene particles can be classified, based on their mechanism of synthesis and physical and functional characteristics. as i) conventional networks and ii) hypercrossiinked networks. Preferred adsorbents have a high surface area. have pores that do not collapse, do not require wetting and for materials comprising red blood cells or platelets, also have extremely low levels of small particles and foreign particles (*e.g.* dust, fibers, non-adsorbent particles, and unidentified particles). For a batch device, the small particles preferably have a diameter of less than 30µm. For a flow device, the small particles preferably have a diameter of less than 5µm. In addition, preferred adsorbents have low levels of extractable residual monomer, crosslinkers and other organic extractables.

The conventional networks are primarily styrene-divinylbenzene copolymers in which divinylbenzene (DVB) serves as the crosslinking agent (*i*.*e*., the agent that links linear polystyrene chains together). These polymeric networks include the "gel-type" polymers. The gel-type polymers are homogeneous, nonporous styrene-DVB copolymers obtained by copolymerization of monomers. The macroporous adsorbents represent a second class of conventional networks. They are obtained by copolymerization of monomers in the presence of diluents that precipitate the growing polystyrene chains. The polystyrene network formed by this procedure possess a relatively large internal surface area (up to hundreds of square meters per gram of polymer); Amberlite® XAD-4 is produced by such a procedure.

In contrast to the conventional networks described above, the preferred adsorbents of the present invention (*e.g.*, Dowex® XUS-43493) are hypercrosslinked networks. These networks are produced by crosslinking linear polystyrene chains either in solution or in a swollen state with bifunctional agents; the preferred bifunctional agents produce conformationally-restricted crosslinking bridges, that are believed to prevent the pores from collapsing when the adsorbent is in an essentially anhydrous (*i.e*., "dry") state.

The hypercrosslinked networks are believed to possess three primary characteristics that distinguish them from the conventional networks. First, there is a low density of polymer chains because of the bridges that hold the polystyrene chains apart. As a result, the adsorbents generally have a relatively large porous surface area and pore diameter. Second, the networks are able to swell; that is, the volume of the polymer phase increases when it contacts organic molecules. Finally, the hypercrosslinked polymers are "strained" when in the dry state; that is, the rigidity of the network in the dry state prevents chain-to-chain attractions. However, the strains relax when the adsorbent is wetted, which increases the network's ability to swell in liquid media. Davankov and Tsyurupa, *Reactive Polymers* 13:27-42 (1990); Tsyurupa *et al*., *Reactive Polymers* 25:69-78 (1995).

Several cross-linking agents have been successfully employed to produce the bridges between polystyrene chains, including *p*-xylene dichloride (XDC), monochlorodimethyl ether (MCDE), 1,4-bis-chloromethyldiphenyl (CMDP), 4,4'-bis-(chloromethyl)biphenyl (CMB), dimethylformal (DMF), *p*,*p*'-bis-chloromethyl-1,4-diphenylbutane (DPB), and tris-(chloromethyl)-mesitylene (CMM). The bridges are formed between polystyrene chains by reacting one of these cross-linking agents with the styrene phenyl rings by means of a Friedel-Crafts reaction. Thus, the resulting bridges link styrene phenol rings present on two different polystyrene chains. *See, e.g.,* U.S. Patent No. 3,729,457.

The bridges are especially important because they generally eliminate the need for a "wetting" agent. That is, the bridges prevent the pores from collapsing when the adsorbent is in an essentially anhydrous (*i.e.*, "dry") state, and thus they do not have to be "reopened" with a wetting agent prior to the adsorbent being contacted with a blood product. In order to prevent the pores from collapsing, conformationally-restricted bridges should be formed. Some bifunctional agents like DPB do not result in generally limited conformation: for example, DPB contains four successive methylene units that are susceptible to conformation rearrangements. Thus, DPB is not a preferred bifunctional agent for use with the present invention.

Some of the structurally-related characteristics of the above-described adsorbent particles are summarized in Table A.

**TABLE A**

| **Resin** | **Chemical Nature** | **Mean Surface** **Area (m**^{**2**}**/g)** | **Mean Pore** **Diam. (A)** | **Mesh** **Size (µm)** |
|---|---|---|---|---|
| **Amberlite® Adsorbents - Rohm and Haas** | | | | |
| XAD-2 | polyaromatic | 300 | 90 | 20-60 |
| XAD-4 | polyaromatic | 725 | 40 | 20-60 |
| XAD-7 | polymethacrylate | 450 | 90 | 20-60 |
| XAD-16 | polyaromatic | 800 | 100 | 20-60 |
| XAD-1180 | polyaromatic | 600 | 300 | 20-60 |
| XAD-2000 | polyaromatic | 580 | 42 | 20-60 |
| XAD-2010 | polyaromatic | 660 | 280 | 20-60 |

| **Amberchrom® Adsorbents - Toso Haas** | | | | |
|---|---|---|---|---|
| CG-71m | polymethacrylate | 450-550 | 200-300 | 50-100 |
| CG-71c | polymethacrylate | 450-550 | 200-300 | 80-160 |
| CG-161m | polyaromatic | 800-950 | 110-175 | 50-100 |
| CG-161c | polyaromatic | 800-950 | 110-175 | 80-160 |

| **Diaion®//Sepabeads® Adsorbents - Mitsubishi Chemical** | | | | |
|---|---|---|---|---|
| HP20 | polyaromatic | 500 | 300-600 | 20-60 |
| SP206 | brominated styrenic | 550 | 200-800 | 20-60 |
| SP207 | brominated styrenic | 650 | 100-300 | 20-60 |
| SP850 | polyaromatic | 1000 | 50-100 | 20-60 |
| HP2MG | polymethacrylate | 500 | 200-800 | 25-50 |
| HP20SS | polyaromatic | 500 | 300-600 | 75-150 |
| SP20MS | polyaromatic | 500 | 300-600 | 50-100 |

| **Dowex® Adsorbents - Dow Chemical Company** | | | | |
|---|---|---|---|---|
| XUS-40285 | functionalized | 800 | 25 | 20-50 |
| XUS-40323 | polyaromatic | 650 | 100 | 16-50 |
| XUS-43493 | polyaromatic | 1100 | 46 | 20-50 |

### Processing the Adsorbent Particles

The adsorbent particles may be further processed to remove fine particles, salts. potential extractables, and endotoxin. The removal of these extractable components is typically performed by treatment with either organic solvents. steam, or supercritical fluids. Preferably the particles are sterilized.

Several companies currently sell "cleaned" (*i*.*e*., processed) versions of commercially available adsorbent particles. In addition to processing the adsorbent particles (*e*.*g*. resins), these companies test the adsorbents, and the final adsorbent is certified sterile (USP XXI), pyrogen-free (LAL), and free of detectable extractables (DVB and total organics).

Thermal processing (*e.g*., steam) is an effective method for processing adsorbent particles. F. Rodriguez, *Principles Of Polymer Systems,* (Hemisphere Publishing Corp.), pp. 449-53 (3rd. Ed., 1989). Supelco, Inc. (Bellefonte, PA) uses a non-solvent, thermal proprietary process to clean the Dowex® XUS-43493 and Amberlite adsorbents. The main advantage of using steam is that it does not add any potential extractables to the adsorbent. One big disadvantage, however, is that this process can strip water from the pores of the resin beads; effective performance of some adsorbents requires that the beads be re-wet prior to contacting the illuminated blood product.

One advantage of the cleaned/processed adsorbent is an extremely low level of particles with diameters less than 30 µm. Preliminary testing on adsorbents (Dowex® XUS-43493 and Amberlite® XAD-16) processed by Supelco was performed to determine particle counts. The results of these tests indicated that foreign particles (*e.g.*, dust, fibers, non-adsorbent particles, and unidentified particles) were absent and that fine particles (< 30 µm) were essentially absent.

### The Use of Wetting Agents and Stabilizing Agents with Adsorbent Resins

Methods may be used for preventing drying and loss of adsorption capacity of particles, such as Amberlite® which lose some of their adsorption capacity under certain conditions (*e.g*., drying).

In one method, particles, materials or devices may be manufactured in a wet state which is sealed and not capable of drying. This method is associated with several important drawbacks. The shelf-life of the products could be reduced since levels of extractables from the materials could increase over time. Sterilization may be limited to a steam process because γ-irradiation of wet polymers is typically not performed. Manufacturing a device that requires that a component be maintained in a wet state is, in general. more difficult than manufacturing a dry device; for example, bioburden and endotoxin may become of concern if there is a long lag time between device assembly and terminal sterilization.

A second method for preventing loss of adsorption capacity involves the use of an adsorbent which is not adversely affected by drying. As previously set forth, macroreticular adsorbents possessing highly crosslinked porous structures (*e.g*., Dowex® XUS-43493 and Purolite® MN-200) generally do not require a wetting agent because the crosslinks prevent the pores from collapsing. Unlike Amberlite® XAD-16, these macroreticular adsorbents retain a very high proportion of their initial activity when they are dried.

In a third method, loss of adsorption capacity upon drying may be prevented by hydrating Amberlite® XAD-16 and related adsorbents (*e.g.*, Amberlite® XAD-4) in the presence of a non-volatile wetting agent. For example, when using Amberlite® XAD-16 as the adsorbent, the adsorbent beads may partially dry prior to use during handling, sterilization, and storage. When the water content of these adsorbents drops below a critical level, a rapid loss in adsorption capacity occurs (probably due to "collapse" of the pores); thus, for optimum effectiveness, the pores have to be "reopened" with a wetting agent prior to use.

Stabilizing agents are effective in maintaining adsorption capacity near its maximum when certain adsorbent resins arc subjected to drying conditions. It is believed that the use of stabilizing agents serves to prevent the adsorbent pores from collapsing.

An acceptable stabilizing agent should be soluble in water and ethanol, nonvolatile relative to ethanol and water, and safe for transfusion in small amounts. Glycerol and low molecular weight polyethylene glycol (*e.g.*, PEG-200 and PEG-400) are examples of stabilizing agents possessing these characteristics. Glycerol has a positive hemocompatibility history. It is frequently added to blood as a cryo-preservative agent in the frozen storage of red blood cell preparations. *See*, *e*.*g*., Chaplin *et al*., Transfusion 26:341-45 (1986); Valeri *et al*., Am. J. Vet. Res. 42(9)1590-94 (1981). Solutions containing up to 1% glycerol are routinely transfused, and glycerol solutions are commercially available (*e.g*., Glyerolite 57 Solution, Fenwal Laboratories, Deerfield, IL). Adsorbent beads like Amberlite® XAD-16 may stabilized in ethanol and glycerol.

Low molecular weight polyethylene glycols, commonly used as pharmaceutical bases, may also be used as stabilizing agents. PEGs are liquid and solid polymers of the general chemical formula H(OCH₂CH₂)_{*n*}OH, where *n* is greater than or equal to 4. PEG formulations are usually followed by a number that corresponds to its average molecular weight; for example, PEG-200 has a molecular weight of 200 and a molecular weight range of 190-210. PEGs are commercially available in a number of formulations (*e.g.*, Carbowax, Poly-G, and Solbase).

### Inert Matrices for Paricle Immobilization

The adsorbent particles are immobilized by an inert matrix. The inert matrix can be made of a synthetic or natural polymer. For example, the inert matrix can be a synthetic or natural polymer fiber, for example, a fiber network. The inert matrix can be sintered polymers. The inert matrix, as with the other components of the device, preferably is biocompatible and does not substantially adversely affect the biological activity of a material upon contact.

Most preferably, the synthetic fibers are polyethylene fibers (Air Quality Filtration (AQF), a division of Hoechst Celanese (Charlotte, N.C.)). Other preferred examples of synthetic fibers are polyolefin, polyester or polyamide fibers. Other exemplary synthetic fibers include polypropylene, polyvinyl alcohol and polysulfone fibers.

In a preferred embodiment, the synthetic polymer fiber includes a first polymer core with a high melting point surrounded by a sheath with a lower melting temperature. The polymer core can be a (polyethylene polyester terephthalate) core. The sheath can be a nylon sheath, or a modified polyester sheath. Fibers are commercially available from Unitika (Osaka, Japan) and Hoechst Trevira GmbH & Co. (Augsberg, Germany).

Exemplary natural polymer fibers include cellulose fibers derived from a variety of sources, such as jute, kozu, kraft and manila hemp. Networks of synthetic or natural polymer fibers have been used to make filters as described in U.S. Patent Nos. 4,559,145 and 5,639,376.

Synthetic polymers suitable for the construction of sintered particles are high density polyethylene, ultra high molecular weight polyethylene, polypropylene, polyvinyl fluoride, polytetrafluoroethylene, nylon 6. More preferably the sintered particles are polyolefins, such as polyethylene.

Fibers without adsorbent particles are contemplated by the present invention; such fibers preferably contain a large, porous, adsorptive surface area or other adsorptive means to facilitate reduction in concentration of small organic compounds.

### Immobilization of Particles

In one embodiment, the adsorbent particles are immobilized by an inert matrix to produce an adsorption medium for reducing the concentration of small organic compounds in a material. The inert matrix can be a three dimensional network including a synthetic or natural polymer fiber network with adsorbent particles immobilized therein.

Preferably, the adsorption medium comprises small porous adsorbent particles with highly porous structures and very high internal surface areas, as described above, immobilized by the inert matrix. Preferably, when a biological material is brought into contact with the adsorption medium, the adsorption medium does not substantially adversely affect the biological activity or other properties of the material.

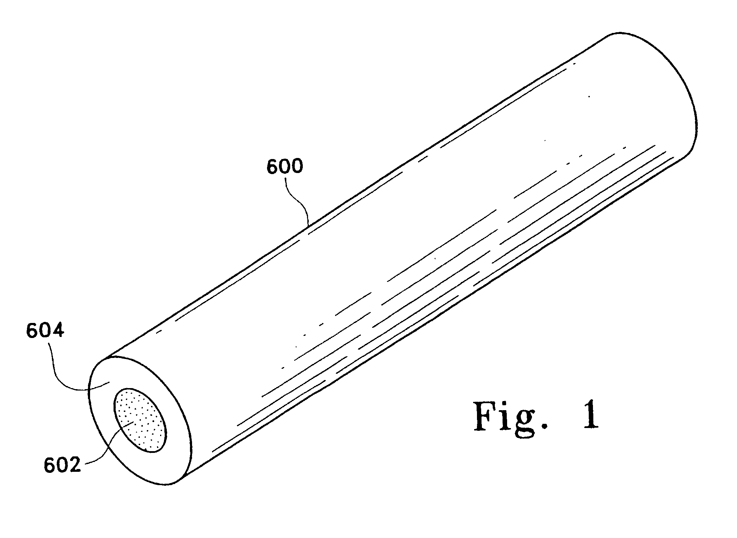

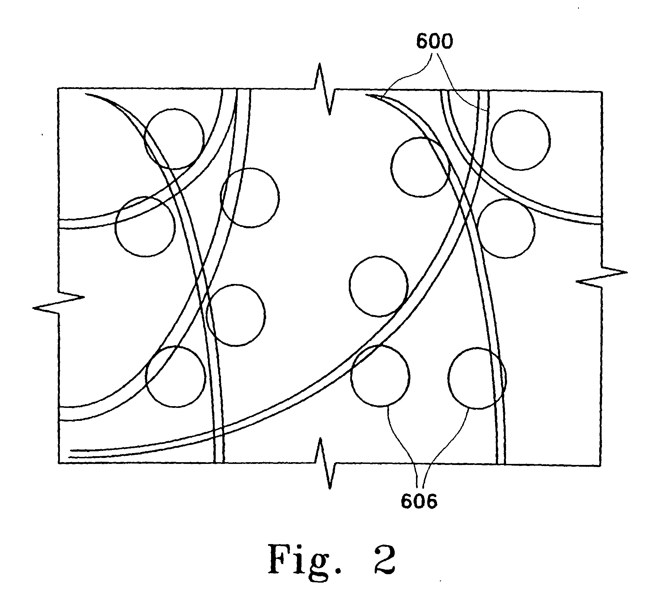

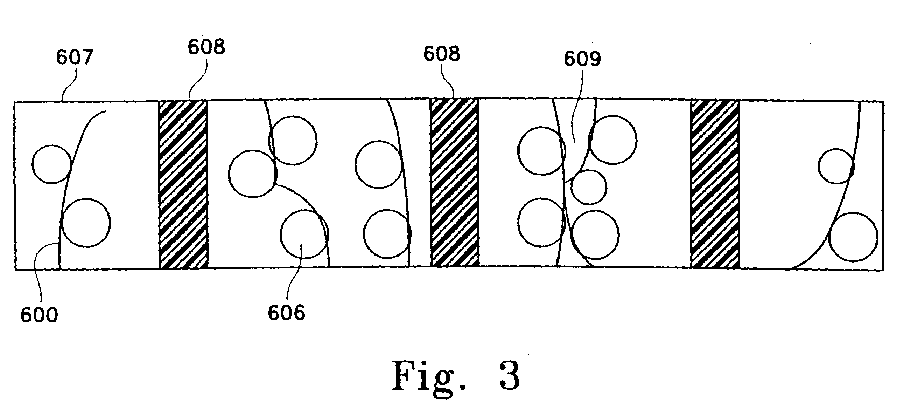

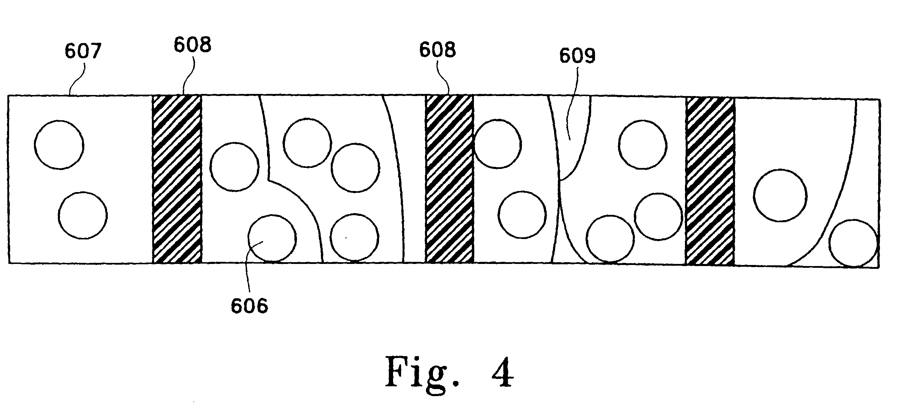

Technology for immobilization of adsorbent beads on a fiber network to construct air filters has been described in U.S. Patent No. 5,486,410 and U.S. Patent No. 5,605,746. As depicted in Figure 1, the polymer fibers 600 of the fiber network consist of a polymer core 602 (*e*.*g*., polyethylene terephthalates (PET)) with a high melting point surrounded by a polymer sheath 604 (*e*.*g*., nylon) with a relatively low melting temperature. *See* U.S. Patent No. 5,190,657 to Heagle *et al*.. The fiberized resin is prepared by first evenly distributing the adsorbent beads in the fiber network. Next, the network is rapidly heated (*e.g*., 180°C x 1 min.) causing the polymer sheath of the fibers 600 to melt and bond to the adsorbent beads 606 and other fibers, forming a cross-linked fiber network, represented in Figure 2. As depicted in Figure 3 and Figure 4 (not to scale), generally speaking, the fiber networks contain three layers; two outer layers 607 that are densely packed with fibers 600 and a less dense inner layer 609 that contains the adsorbent beads 606 and fewer fibers 600. In a preferred embodiment, the edges of the fiberized resin may be sealed with polyurethane or other polymers. Alternatively, as depicted in Figure3 and Figure 4, heat seals 608 may be made in the resulting fiberized resin at predetermined intervals; for example, heat seals can be made in the fiberized resin in a pattern of squares. Thereafter, the fiberized resin can be cut through the heat seals to form samples of resin containing a desired mass (*e.g*., preferably less than 5.0 g and more preferably less than 3.0 g) of adsorbent beads and of a size suitable for placement within a blood product container. The heat seals serve to prevent the cut fiberized resin from fraying and help to immobilize the adsorbent beads. However, the use of such heat seals in not required in order to practice the present invention. In an alternative embodiment, depicted in Figure 4, the adsorbent beads 606 are not secured to the fibers themselves, but rather are immobilized between the denser outer layers 607 of fibers and with the heat seals 608; this embodiment may also result in samples of fiberized media containing a defined amount of adsorbent after being cut through the heat seals.

The present invention also contemplates the use of an adhesive (*e.g.,* a bonding agent) to secure the adsorbent resin to the fibers. Moreover, while it is preferable that the adsorbent beads be chemically attached to the fiber network, the beads may also be physically trapped within the fiber network; this might be accomplished, for example, by surrounding the beads with enough fibers so as to hold the beads in position.

Other ways that the adsorbent particles may be immobilized in a fiber network are also contemplated. The particles can be immobilized using a dry-laid process, as described in U.S. Pat. Nos. 5,605,746 and 5,486,410 (AQF patents). The particles can be immobilized using a wet-laid process, as described in U.S. Pat. Nos. 4,559,145 and 4,309,247. The particles can be immobilized using a melt-blown process, as described in U.S. Pat. No. 5,616,254, which is herein incorporated by reference. Where a wet-laid process is used to construct a matrix from natural polymer fibers, the inert matrix preferably includes a binding agent to bond the adsorbent particles to the fibers. Nonlimiting examples of binding agents include melamine, polyamines and polyamides. The matrix typically contains 1% or less of such binding agents.

Where the inert matrix is constructed from particles of synthetic polymers which are sintered with adsorbent particles, it is important that the adsorbent particle have a higher melting temperature than the matrix.

Preferably, the resulting adsorption medium comprises known amounts of adsorbent per area. For batch devices the adsorbent per area is from about 100 g/m² to about 500 g/m², preferably from about 250 g/m² to about 350 g/m². For flow devices the adsorbent per area is from about 300 g/m² to about 1100 g/m², preferably from about 500 g/m² to about 700 g/m². Thus, the appropriate amount of adsorbent contemplated for a specific purpose can be measured simply by cutting a predetermined area of the fiberized resin (*i.e.*, there is no weighing of the fiberized resin).

The adsorption medium preferably is biocompatible *(i.e.,* not producing a toxic, injurious, or immunologic response); has a minimal impact on the properties of the material such as blood product (*e.g.*, platelets and clotting factors); and is not associated with toxic extractables. The immobilized adsorbent particles of the adsorption medium preferably have high mechanical stability (*i.e.*, no fine particle generation). The adsorption medium for a batch device contains about 25-85% adsorbent by weight, preferably about 50-80% adsorbent at a loading of about 100-500 g/m², more preferably about 50-80% adsorbent at a loading of about 250-350 g/m².

The adsorption medium for a flow device contains about 20-70% adsorbent by weight, preferably 30-50% by weight. Preferably the adsorption medium contains about 30% by weight of the adsorbent particle where a fibrous matrix is used. Where a sintered particulate matrix is used with a ground polymeric adsorbent particle, the adsorption medium preferably contains about 50% by weight of the adsorbent particle.

### Coating the Adsorbent Particles

The surface hemocompatibility of the particles, matrices or adsorption medium can be improved by coating their surfaces with a hydrophilic polymer. Exemplary hydrophilic polymers include poly(2-hydroxyethyl methacrylate) (pHEMA), which may be obtained from, *e.g.*, Scientific Polymer Products, Inc. (Ontario, NY) and cellulose-based polymers, *e*.*g*., ethyl cellulose, which may be obtained from Dow Chemical (Midland, MI). *See*, *e*.*g*., Andrade *et al*., *Trans*. *Amer*. *Soc*. *Artif*. *Int*. *Organs* XVII:222-28 (1971). Other examples of coatings include polyethylene glycol and polyethylene oxide, also available from Scientific Polymer Products, Inc.. The polymer coating can increase hemocompatibility and reduce the risk of small particle generation due to mechanical breakdown.

The adsorbent surface may also be modified with immobilized heparin. In addition, strong anion exchange polystyrene divinylbenzene adsorbents may be modified via heparin adsorption. Heparin, a polyanion, will adsorb very strongly to the surfaces of adsorbents which have strong anion exchange characteristics. A variety of quaternary amine-modified polystyrene divinyl benzene adsorbents are commercially available.

The coating can be applied in a number of different methods, including radio frequency glow discharge polymerization, as described in U.S. Patent number 5,455,040 and the Wurster coating process (performed by International Processing Corp. (Winchester. KY).

In one embodiment, the Wurster coating process can be applied by suspending the adsorbent particles (generally via air pressure) in a chamber such that the hydrophilic polymer, such as pHEMA, can be sprayed evenly onto all surfaces of the adsorbent particle. As illustrated in Example 3, Dowex® XUS-43493 sprayed evenly with pHEMA demonstrated an increase in platelet yield as well as a dramatic effect on platelet shape change with increasing amounts of coating. It was found that the Wurster coating process selectively coated the outside surface of the adsorbent surface, leaving the inside porous surface nearly unaffected.

In a preferred embodiment, the coating can be applied by soaking the immobilized adsorption medium in the hydrophilic polymer (see Example 3). This process is simpler and less expensive than spraying the adsorbent particles with the hydrophilic polymer.

The process is not limited to a process that applies the coating of the adsorption medium at any particular time. For example, in one embodiment, the pHEMA coating is applied after production of the adsorption medium, but prior to heat sealing the adsorption medium. In another embodiment, the adsorption medium is first heat sealed, and then the pHEMA coating is applied. In addition to coating the adsorption medium, the rinsing process associated with pHEMA application serves to remove loose particles and fibers.

As the amount of coating is increased, it becomes more difficult for some small organic compounds to cross the coating to reach the particle surface, resulting in a decrease in adsorption kinetics. Thus, as the amount of coating is increased, an increased mass of adsorbent must generally be used to achieve the same removal kinetics as coated adsorbent. In one embodiment, the optimum level of pHEMA coating is the minimum coating at which a protective effect on platelet yield and *in vitro* platelet function is observed (0.1-0.5%).

The coatings may be sensitive to sterilization. For example, gamma sterilization may result in cross-linking and/or scission of the coating. Therefore, the type (E-beam vs. gamma irradiation) and dose of sterilization may influence the properties of the coated adsorbent. Generally, E-beam sterilization is preferred.

### Devices

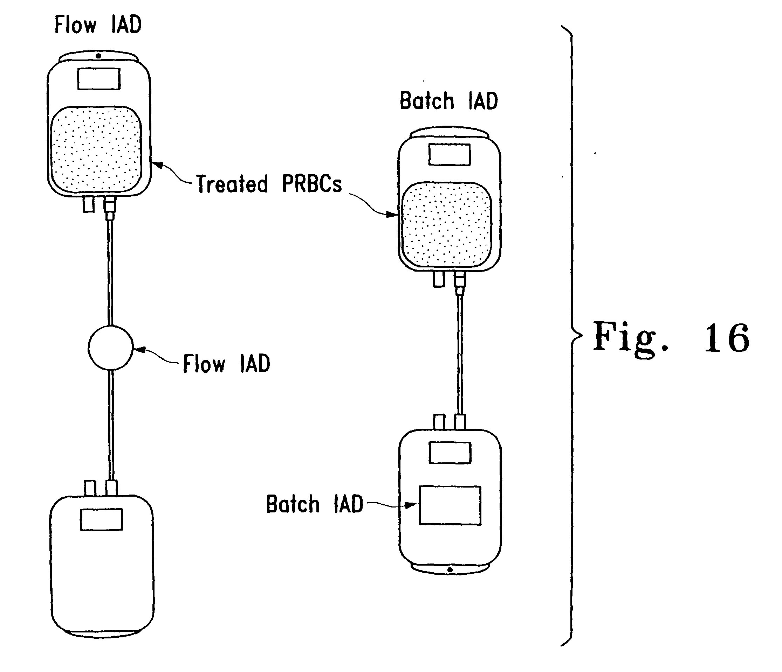

Devices are provided for reduction of concentration of small organic compounds from materials such as blood products. The device may be, for example, a flow device or a batch device. Examples of flow and batch devices are shown in Figure 16. Flow and batch devices are known in the literature and are described, for example, in PCT publication WO 96/40857.

Batch devices of the invention comprise a container, such as a blood bag, including the adsorbent medium containing immobilized particles. In one embodiment a blood product is added to a blood bag containing the adsorbent medium and the bag is agitated for a specified period of time.

For example, in one embodiment, an adsorption medium, *e.g.,* immobilized Dowex® XUS-43493, is placed inside a blood product container (*e.g.*, a PL 146 Plastic container (BaxterHealthcare Corp. (Deerfield, IL)) kept on either a platelet shaker (Helmer Laboratories (Novesvill, IN)) or rotator (Helmer Laboratories (Novesvill, IN)) for about 24 hours at room temperature and stored under various temperature conditions. The size of the blood product container can be from about 600 to about 1000 mL. The storage temperature can be from about 4°C to about 22°C.

Methods can be used to reduce the presence of adsorbent particles that may come loose from the adsorbent medium.

The present invention contemplates a batch device including the immobilized adsorbent medium retained in a container such as a mesh bag/pouch. The mesh/pouch can be constructed of a woven, non-woven or membranous enclosure. In one embodiment, the woven mesh pouch can be constructed of medical-grade polyester or nylon. The preferred embodiment is polyester. Commercially-available membranes include, but are not limited to, Supor® 200, 800, 1200 hydrophilic polyethylene sulfonate (PES) membranes (Gelman Sciences (Ann Arbor, MI)); Durapore® hydrophilic modified polyvinylidene difluoride (PVDF) (Mantee America Corp. (San Diego, CA)) and hydrophilic modified polysulfone membranes with integrated hydrophobic vents, for *e.g.* Gemini membranes, (Millipore (Marlborough, MA)); and membranes comprising polycarbonate with a polyvinylidene coating (Poretics (Livermore, CA)). The containers can be sterilized after addition of the adsorption medium.

Flow devices permit reduction of concentration of small organic compounds from materials such as blood products by perfusing the blood product through the flow device.

Where the device is a flow device, the adsorption medium is preferably about 3 to 30 mm thick to promote an even flow of biological fluid without a substantial pressure drop. Preferably the medium is about 3 to 15 mm thick. More preferably, the medium is about 5 to 8 mm thick. Where the device a batch device, the adsorption medium can be from about 2 to 30 mm thick. Preferably the medium is about 2 to 10 mm thick.

### Preferred Embodiment for Platelets

Preferably, a batch or flow device for reducing the concentration of small organic compounds in a material comprising platelets while substantially maintaining the biological activity of the platelets is provided. A batch device is preferred. The adsorption medium comprises adsorbent particles immobilized by an inert matrix. Preferred particles are highly porous and have a surface area greater than about 750 m²/g.

Particularly preferred particles are polyaromatic adsorbents comprising a hypercrosslinked polystyrene network, such as Dowex® XUS-43493 or Purolite MN-200. The preferred inert matrix includes a synthetic or natural polymer fiber. In a preferred embodiment the inert matrix includes a synthetic polymer fiber which includes a first polymer core with a high melting point surrounded by a sheath with a lower melting temperature. The polymer core can be a polyethylene terephthalate core. The sheath can be a nylon sheath or a modified polyester sheath. Staple fibers are commercially available from Unitika (Osaka, Japan) and Hoechst Trevira.

In one embodiment, the batch device comprises an adsorption medium, a particle retention device (*e*.*g*., woven mesh, nonwoven material, or membrane) and a housing (*e*.*g*., blood storage container).

Exemplary small organic compounds that are reduced by the devices, materials and methods of the present are psoralens, psoralen derivatives, isopsoralens, psoralen photoactivation products, acridines and acridine derivatives.

Platelets are typically used within 3 days of donation but may be stored for up to 5 days at room temperature, therefore, it would be advantageous to allow the platelets to remain in contact with the adsorption medium for the entire storage period. Preferably, the procedure would result in an acceptable platelet yield (*e.g*., less than 10% loss). One method contemplated by the present invention allows extended storage by improving the hemocompatibility of the adsorbent surface.

The use of an adsorption medium comprising adsorbent particles immobilized by an inert matrix permits the concentration of small organic compounds to be reduced without a substantial loss in platelet count. The phrase "without a substantial loss" refers to a loss of platelet count of less than about 10%, preferably less than about 5% over a period of time of 1 day, more preferably 5 days. Furthermore, the time that the platelets may be contacted with the adsorption medium without substantial loss in platelet count is greater than the amount of time that the platelets can be contacted with the adsorbent particles alone. The immobilization of the particles unexpectedly permits both a longer contact time and a reduction in loss of platelet count. The platelets typically cannot be contacted with non-immobilized adsorbent particles for more than about 20 hours without a significant loss of platelet count e.g. about 80% yield. In contrast, platelets may be contacted with the adsorption medium comprising adsorbent particles immobilized by an inert matrix for more than 20 hours, *e.g.* about 1 to 5 days without a substantial loss in platelet count

Additionally *in vitro* platelet function (*e*.*g*., shape change, GNP-140, pH) is improved for platelets stored in the presence of the adsorption medium in comparison to the storage of the platelets without the adsorption medium over time. Platelets stored in the presence of the adsorption medium can have a pH from greater than about 6 to less than about 7.5.

### Preferred Embodiment for Plasma

Preferably, a batch or flow device for reducing the concentration of small organic compounds in a material comprising plasma while substantially maintaining the biological activity of the plasma is provided. A flow device is preferred. The adsorption medium comprises adsorbent particles immobilized by an inert matrix. Preferred particles are highly porous and have a surface area greater than about 750 m²/g.

Particularly preferred particles are Norit A Supra, which is available from Norit Americas, Inc. (Atlanta, GA). Norit A Supra is a USP-grade activated carbon that is formed by steam activation of lignite. This activated carbon has a very high total surface area (2000 m²/g) and is very microporous in nature.

Additionally, the particles may be selected from any of the following particles wherein the particles preferably posses a size range of 10 µm to 100 µm in diameter, either by grinding direct synthesis, or some other means, and are activated carbons, such as Picactif Medicinal (Pica USA, Columbus, OH), synthetic carbonaceous adsorbents, such as Ambersorb 572 (Rohm and Haas, Philadelphia, PA), hydrophobic resins, such as Amberlite adsorbents (*e.g.*, Amberlite® XAD-2, XAD-4, and XAD-16), available from Rohm and Haas (Philadelphia, PA); Amberchrom® adsorbents available from Toso Haas (TosoHass, Montgomeryville, PA); Diaion®//Sepabeads® Adsorbents (*e.g*., Diaion® HP20). available from Mitsubishi Chemical America, Inc. (White Plains, NY); Hypersol-Macronet® Sorbent Resins (*e.g*., Hypersol-Macronet® Sorbent Resins MN-150 and MN-400) available from Purolite (Bala Cynwyd, PA) and Dowex® Adsorbents (*e.g.*, Dowex® XUS-40323, XUS-43493. and XUS-40285), available from Dow Chemical Company (Midland, Ml).

The inert matrix may be a synthetic or natural polymer fiber. In a preferred embodiment the inert matrix is sintered particles or cellulose.

Exemplary small organic compounds that are reduced by the devices, materials and methods of the present arc psoralens, psoralen derivatives, isopsoralens, psoralen photoactivation products, methylene blue, phenothiazine, acridine.

Where the biological fluid containing small molecules whose concentration is to be reduced is plasma, the device is preferably a flow device that maintains adequate levels of clotting activity. Measures of clotting activity include prothrombin time, activated partial thromboplastin time, and functional measures of clotting factors I, II, V, VII, VIII, IX, X, XI, and XII. An adequate functional measure of clotting factor activity is greater than 80% of the level prior to passing through the device, or in the case of clotting times, one that remains in the normal range established for each laboratory that does this type of testing. Preferred measures of clotting activity include PT and PTT, as they are measures of the overall ability of the plasma to clot, and factors I, II, V, VII, X, XI, and XII, as these factors are not commonly replaced by recombinant proteins. It is preferred to retain more than 90% of the clotting activity of these factors relative to the level prior to passing through the device and have changes in PT and PTT of less than about 1.5 seconds.

In one embodiment the flow device may comprise an adsorption medium and a housing. The housing should promote even flow of the plasma to promote good media utilization and as it primes, allow the plasma to push air ahead of it, thereby eliminating bubbles that would reduce the contact area between the plasma and the adsorption media, thereby reducing the media's utilization. The housing can be flat or have substantial depth to accommodate adsorption media or particle retention media that is not flat, for example cylindrically shaped adsorption media. In a preferred embodiment, the housing is flat. The housing can have inlets and outlets in various orientations, for example inlet top/outlet top or inlet bottom/outlet bottom. In a preferred embodiment, the outlet is at the bottom to promote good drainage and the inlet is at the top to promote media utilization.

In another embodiment, the flow device comprising an adsorption medium and a housing may also include a particle retention medium. In a preferred embodiment the device includes a particle retention medium downstream of the adsorption medium to retain particles that are shed from the adsorption medium while maintaining a high fluid flow rate and high recovery of proteins. The particle retention medium can be a membrane, a dry or wet laid matrix of fibers, a sintered polymer matrix, a woven material, a nonwoven material (polyester nonwoven), or a combination thereof.

The device housing holds the particle retention medium in an approximately parallel orientation downstream of the adsorption medium. (U.S. Patent No. 5,660,731 discloses examples of filter housings.) The housing can be constructed from any suitably rigid, impervious, material that does not substantially adversely affect the biological activity of a fluid. Preferably the housing is constructed from a synthetic polymer. Nonlimiting examples of such polymers include polyacrylic, polyethylene, polypropylene, polystyrene and polycarbonate plastics.

Where the device is a flow device, the adsorption medium containing particles immobilized by an inert matrix should be between 3 and 30 mm thick to promote an even flow of biological fluid without a substantial pressure drop. Preferably the medium should be between 3 and 15 mm thick. More preferably, the medium should be between 5 and 8 mm thick.

Where the device is a flow device, gravity flow is preferred. More preferably, the device is a gravity flow device that is constructed to permit flow rates of 10-80 mL/min with differential pressures of 12-72 inches water. More preferably the device permits flowrates of 30-60 mL/min with differential pressures of 24-48 inches of water.

### Preferred Embodiment for Red Blood Cells

Preferably, a batch or flow device for reducing the concentration of small organic compounds in a material comprising red blood cells while substantially maintaining the biological activity of the red blood cells is provided. The adsorption medium comprises adsorbent particles immobilized by an inert matrix. Preferred particles are highly porous and have a surface area greater than about 750 m²/g.

Preferably, the device is a flow or batch device that substantially maintains the biological activity of the red blood cells after reduction of the concentration of small organic compounds. The flow device embodiment comprises two components: an adsorption medium and a housing, and optionally, a particle retention medium. Embodiments for the particle retention medium and housing are as described above for the plasma device embodiment. In another embodiment, the red blood cell device is a batch device that does not substantially adversely affect the biological activity of a fluid upon contact. The batch device embodiment comprises an adsorption medium containing particles immobilized by an inert matrix and optionally a particle retention device.

In one embodiment the particles are activated charcoal. Preferably the activated carbons are derived from synthetic sources. Nonlimiting examples of activated carbons include Picactif Medicinal, which is available from Pica U.S.A. (Columbus, OH); Norit ROX 0.8, which is available from Norit Americas Inc. (Atlanta, GA); and G-277, which is available from Pica U.S.A. (Columbus, OH).

Where the red blood cell device is a batch device, the adsorbent is preferably an activated carbon derived from a synthetic source, such as Ambersorb 572. The Ambersorbs are synthetic activated carbonaceous (*e.g,* rich in carbon) adsorbents that are manufactured by Rohm & Haas (Philadelphia, PA). Ambersorbs are generally large spherical (300 - 900 µm) particles that are more durable than typical activated carbons. The Ambersorbs are synthetically manufactured by treating highly sulfonated porous polystyrene beads with a proprietary high temperature activation process. These adsorbents do not require pre-swelling to achieve optimal adsorption activity.

In another preferred embodiment, the particles can be hydrophobic resins. Nonlimiting examples of hydrophobic resins include the following polyaromatic adsorbents: Amberlite® adsorbents (*e*.*g*., Amberlite® XAD-2, XAD-4, and XAD-16), available from Rohm and Haas (Philadelphia, PA); Amberchrom® adsorbents available from Toso Haas (TosoHass, Montgomeryville, PA); and Diaion®//Sepabeads® Adsorbents (*e*.*g*., Diaion® HP20), available from Mitsubishi Chemical America, Inc. (White Plains, NY). In a particularly preferred embodiment the particles are Hypersol-Macronet® Sorbent Resins (*e*.*g*., Hypersol-Macronet® Sorbent Resins MN-200, MN-150 and MN-400) available from Purolite (Bala Cynwyd, PA) or Dowex® Adsorbents (*e.g.*, Dowex® XUS-43493, and XUS-40285), available from Dow Chemical Company (Midland, MI).

The preferred inert matrix includes a synthetic or natural polymer fiber. In a preferred embodiment the inert matrix includes a synthetic polymer fiber which includes a first polymer core with a high melting point surrounded by a sheath with a lower melting temperature. The polymer core can be a polyethylene terephthalate or polyester core. The sheath can be a nylon sheath or a modified polyester sheath. Fibers are commercially available from Unitika (Osaka, Japan) and Hoechst Trevira (Augsberg, Germany).

In some embodiments, the adsorption medium is in an enclosure. In one embodiment, the batch device comprises an adsorption medium, and a housing. In another embodiment, the batch device comprising an adsorption medium and a housing may also include a particle retention medium. In a preferred embodiment, the housing comprises a blood bag of a volume between 600 ml and 1L. The particle retention medium may comprise a polyester woven, polyester non-woven, or membranous enclosure, It is preferable that the batch device contact the red blood cells at 4°C or 22°C, in the presence of agitation, over a time period of 1 to 35 days.

The use of an adsorption medium comprising adsorbent particles immobilized by an inert matrix permits the concentration of small organic compounds to be reduced without a substantial loss in red blood cell function. The phrase "without a substantial loss" refers to less than about 1% change in hemolysis; preferably less than about 0.8% change in hemolysis, greater than about 90% recovery of red blood cells; preferably greater than 80%, recovery of red blood cells, less than about 10% difference from no-device control red blood cells in change in ATP concentration, and less than about 15% difference from no-device control red blood cells in change in extracellular potassium concentration. At 35 days, the change in hemolysis is at least 10% higher for the IAD compared to the non-immobilized particles, preferably, 20% higher and more preferably 50%.

Red blood cell function can be assayed using standard kits. In particular, hemolysis may be determined by measuring the adsorbance at 540nm of a red blood cell supernatant sample in Drabkin's reagent ((Sigma Chemical Company), St. Louis, MO). Potassium leakage can be assayed using a Na+/K+ analyzer (Ciba-Corning Diagnostics, Medfield, MA). Quantitative enzymatic determination of ATP in total Red Blood Cell samples is possible using a standard kit (Sigma Diagnostics, St. Louis, MO) and measuring absorbance at 340 nm compared to a water background.

Where the biological fluid to be purified is red blood cells, the device can reduce the concentration of both cyclic and acyclic small organic molecules in a red blood cell sample. Preferably the device can reduce the concentration of both acridine derivatives and thiols in a red blood cell sample. More preferably, the device can reduce the concentration of both 5-[(β-carboxyethyl)amino]acridine and glutathione in a red blood cell sample. Standard HPLC assays can be used to determine concentrations of 5-[(β - carboxyethyl) amino] acridine and glutathione in red blood cells contacted with the device. Assay mobile phases are 10 mM H₃ PO₄ in HPLC water and 10 mM H₃PO₄ in acetonitrile. Zorbax SB-CN and YMC ODSAM-303 columns are available from MacMod Analytical, Inc. (Chadds Ford, PA) and YMC, Inc. (Wilmington, NC).

### Applications

The present invention contemplates reducing the concentration of small organic compounds. Small organic compounds include pathogen-inactivating agents such as photoactivation products, aminoacridines, organic dyes and phenothiazines. Exemplary pathogen inactivating agents include furocoumarins, such as psoralens and acridines. Following treatment of a blood product with a pathogen inactivating compound as described for example in U.S. Patent Numbers 5,459,030 and 5,559,250 the concentration of pathogen inactivating compounds in the blood product can be reduced by contacting the treated blood product with a device of the invention.

In one embodiment the present invention contemplates a method of inactivating pathogens in solution, wherein the method comprises: a) providing, in any order: i) a cyclic compound, ii) a solution suspected of being contaminated with said pathogens, and iii) fiberized resin; b) treating said solution with said cyclic compound so as to create a treated solution product wherein said pathogens are inactivated; and c) contacting said treated solution product with said fiberized resin, and further comprising a device for reducing the concentration of small organic compounds in a blood product while substantially maintaining a desired biological activity of the blood product, the device comprising highly porous adsorbent particles, wherein the adsorbent particles are immobilized by an inert matrix.

In addition to the pathogen inactivating compound, reactive degradation products thereof, can be reduced from the material such as a blood product, for example prior to transfusion.

The materials and devices disclosed herein can be used in apheresis methods. Whole blood can be separated into two or more specific components (*e*.*g*., red blood cells, plasma and platelets). The term "apheresis" refers broadly to procedures in which blood is removed from a donor and separated into various components, the component(s) of interest being collected and retained and the other components being returned to the donor. The donor receives replacement fluids during the reinfusion process to help compensate for the volume and pressure loss caused by component removal. Apherersis systems are described in PCT publication WO96/40857.

### Small Organic Compounds

A diverse set of small organic compounds can be adsorbed by the device of the present invention. The molecules can be cyclic or acyclic. In one embodiment the compounds are preferably, cyclic compounds such as psoralens, acridines or dyes. In another embodiment the compounds are thiols.

Nonlimiting examples of cyclic compounds include actinomycins, anthracyclinones, mitomyacin, anthramycin, and organic dyes and photoreactive compounds such as benzodipyrones, fluorenes, fluorenones, furocoumarins, porphyrins, protoporphrins, purpurins, phthalocyanines. Monostral Fast Blue, Norphillin A, phenanthridines, phenazathionium salts, phenazines, phenothiazines, phenylazides, quinolines and thiaxanthenones. Preferably the compounds are furocoumarins or organic dyes. More preferably the compounds are furocoumarins.

Nonlimiting examples of furocoumarins, include psoralens and psoralen derivatives. Specifically contemplated are 4'-aminomethyl-4,5',8-trimethylpsoralen, 8-methoxypsoralen, halogenated psoralens, isopsoralens and psoralens linked to quaternary amines, sugars, or other nucleic acid binding groups. Also contemplated are the following psoralens: 5'-bromomethyl-4,4',8-trimethylpsoralen, 4'-bromomethyl-4,5',8-trimethylpsoralen, 4'-(4-amino-2-aza)butyl-4,5',8-trimethylpsoralen, 4'-(4-amino-2-oxa)butyl-4,5',8-trimethylpsoralen, 4'-(2-aminoethyl)-4,5',8-trimethylpsoralen, 4'-(5-amino-2-oxa)pentyl-4,5',8-trimethylpsoralen, 4'-(5-amino-2-aza)pentyl-4,5',8-trimethylpsoralen, 4'-(6-amino-2-aza)hexyl-4,5',8-trimethylpsoralen, 4'-(7-amino-2,5-oxa)heptyl-4,5',8-trimethylpsoralen, 4'-(12-amino-8-aza-2,5-dioxa)dodecyl-4,5',8-trimethylpsoralen, 4'-(13-amino-2-aza-6,11-dioxa)tridecyl-4,5',8-trimethylpsoralen, 4'-(7-amino-2-aza)heptyl-4,5',8-trimethylpsoralen, 4'-(7-amino-2-aza-5-oxa)heptyl-4,5',8-trimethylpsoralen, 4'-(9-amino-2,6-diaza)nonyl-4,5',8-trimethylpsoralen, 4'-(8-amino-5-aza-2-oxa)octyl-4,5',8-trimethylpsoralen, 4'-(9-amino-5-aza-2-oxa)nonyl-4,5',8-trimethylpsoralen, 4'-(14-amino-2,6,11-triaza)tetradecyl-4,5',8-trimethylpsoralen, 5'-(4-amino-2-aza)butyl-4,4',8-trimethylpsoralen, 5'-(6-amino-2-aza)hexyl-4,4',8-trimethylpsoralen and 5'-(4-amino-2-oxa)butyl-4,4',8-trimethylpsoralen. Preferably, the psoralen is 4'-(4-amino-2-oxa)butyl-4,5',8-trimethylpsoralen.

### Acridines

Nonlimiting examples of acridines include acridine orange, acriflavine, quinacrine, N1,N1-bis(2-hydroxyethyl)-N4-(6-chloro-2-methoxy-9-acridinyl)-1,4-pentanediamine, 9-(3-hydroxypropyl)aminoacridine, N-(9-acridinyl)glycine, S-(9-acridinyl)-glutathione. In a preferred embodiment the acridine is N-(9-acridinyl)-β-alanine, alternatively, named 5-[(β-carboxyethyl)amino]acridine.

### Dyes

Nonlimiting examples of dyes include phenothiazines such as methylene blue, neutral red, toluidine blue, crystal violet and azure A and phenothiazones such as methylene violet Bemthsen. Preferably, the dye is methylene blue or toluidine blue. More preferably, the dye is methylene blue.

### Other Organic Compounds

The concentration of a variety of organic compounds may be reduced. Other exemplary compounds include quenching compounds. Methods for quenching undesired side reactions of pathogen inactivating compounds that include a functional group which is, or which is capable of forming, an electrophilic group, are described in the co-filed U.S. Patent Application, "Methods for Quenching Pathogen Inactivators in Biological Systems", Docket Number 282173000600, filed January 6, 1998. In this method, a material, such as a blood product, is treated with the pathogen inactivating compound and a quencher, wherein the quencher comprises a nucleophilic functional group that is capable of covalently reacting with the electrophilic group. In one embodiment, the pathogen inactivating compound includes a nucleic acid binding ligand and a functional group, such as a mustard group, which is capable of reacting *in situ* to form the electrophilic group. Examples of quenchers include, but are not limited to, compounds including nucleophilic groups. Exemplary nucleophilic groups include thiol, thioacid, dithoic acid, thiocarbamate, dithiocarbamate, amine, phosphate, and thiophosphate groups. The quencher may be, or contain, a nitrogen heterocycle such as pyridine. The quencher can be a phosphate containing compound such as glucose-6-phosphate. The quencher also can be a thiol containing compound, including, but not limited to, glutathione, cysteine, N-acetylcysteine, mercaptoethanol, dimercaprol, mercaptan, mercaptoethanesulfonic acid and salts thereof, *e*.*g*., MESNA, homocysteine, aminoethane thiol, dimethylaminoethane thiol, dithiothreitol, and other thiol containing compounds. Exemplary aromatic thiol compounds include 2-mercaptobenzimidazolesulfonic acid, 2-mercapto-nicotinic acid, napthalenethiol, quinoline thiol. 4-nitro-thiophenol, and thiophenol. Other quenchers include nitrobenzylpyridine and inorganic nucleophiles such as selenide salts or organoselenides, thiosulfate, sulfite, sulfide, thiophosphate, pyrophosphate, hydrosulfide, and dithionitrite. The quencher can be a peptide compound containing a nucleophilic group. For example, the quencher may be a cysteine containing compound, for example, a dipeptide, such as GlyCys, or a tripeptide, such as glutathione.

Other organic compounds may include thiols such as methyl thioglycolate, thiolactic acid, thiophenol, 2-mercaptopyridine, 3-mercapto-2-butanol, 2-mercaptobenzothiazole, thiosalicylic acid and thioctic acid.

### EXAMPLES

The following examples serve to illustrate certain preferred embodiments and aspects of the present invention and are not to be construed as limiting the scope thereof.

In the experimental disclosure which follows, the following abbreviations apply: eq (equivalents); M (Molar); µM (micromolar); N (Normal); mol (moles); mmol (millimoles); µmol (micromoles); nmol (nanomoles); g (grams); mg (milligrams); µg (micrograms); Kg (kilograms); L (liters); mL (milliliters); µL(microliters); cm (centimeters); mm (millimeters); µm (micrometers); nm (nanometers); min. (minutes); s and sec. (seconds); J (Joules, also watt second); °C (degrees Centigrade); TLC (Thin Layer Chromatography); HPLC (high pressure liquid chromatography); pHEMA and p(HEMA) (poly[2-hydroxyethyl methacrylate]); PC(s) (platelet concentrate(s)); PT (prothrombin time); aPTT (activated partial thromboplastin time); TT (thrombin time); HSR (hypotonic shock response); FDA (United States Food and Drug Administration); GMP (good manufacturing practices); DMF (Drug Masterfiles); SPE (Solid Phase Extraction); Aldrich (Milwaukee, WI); Asahi (Asahi Medical Co., Ltd., Tokyo, Japan); Baker (J.T. Baker, Inc., Phillipsburg, NJ); Barnstead (Barnstead/Thermolyne Corp.. Dubuque, IA); Becton Dickinson (Becton Dickinson Microbiology Systems; Cockeysville, MD); Bio-Rad (Bio-Rad Laboratories, Hercules, CA); Cerus (Cerus Corporation; Concord, CA); Chrono-Log (Chrono-Log Corp.; Havertown, PA); Ciba-Corning (Ciba-Corning Diagnostics Corp.; Oberlin, OH); Consolidated Plastics (Consolidated Plastics Co., Twinsburg, OH); Dow (Dow Chemical Co.; Midland, MI); Eppendorf (Eppendorf North America Inc., Madison, WI); Gelman (Gelman Sciences, Ann Arbor, MI); Grace Davison (W.R. Grace & Co., Baltimore, MD); Helmer (Helmer Labs, Noblesville, IN); Hoechst Celanese (Hoechst Celanese Corp., Charlotte, NC); International Processing Corp. (Winchester, KY); Millipore (Milford, MA); NIS (Nicolet, a Thermo Spectra Co., San Diego, CA); Poretics (Livermore, CA); Purolite (Bala Cynwyd, PA) Rohm and Haas (Chauny, France); Saati (Stamford, CT); Scientific Polymer Products (Ontario, NY); Sigma (Sigma Chemical Company, St. Louis, MO); Spectrum (Spectrum Chemical Mfg. Corp., Gardenia, CA); Sterigenics (Corona, CA); Tetko, Inc. (Depew, NY); TosoHaas (TosoHass, Montgomeryville, PA); Wallac (Wallac Inc., Gaithersburg, MD); West Vacco (Covington, VA); YMC (YMC Inc., Wilmington, NC); DVB (divinyl benzene); LAL (Limulus Amoebocyte Lystate); USP (United States Pharmacopeia); EAA (ethyl-acetoacetate); EtOH (ethanol); HOAc (acetic acid); W (watts); mW (milliwatts); NMR (Nuclear Magnetic Resonance; spectra obtained at room temperature on a Varian Gemini 200 MHz Fourier Transform Spectrometer); ft³/min (cubic feet per minute); m.p. (melting point); g/min and gpm (gallons per minute); UV (ultraviolet light); THF (tetrahydrofuran); DMEM (Dulbecco's Modified Eagles Medium); FBS (fetal bovine serum); LB (Luria Broth); EDTA (ethelene diamine tetracidic acid); Phorbol Myristate Acetate (PMA); phosphate buffered saline (PBS); AAMI (Association for the Advancement of Medical Instruments); ISO (International Standards Organization); EU (endotoxin units); LVI (large volume injectables); GC (gas chromatography); M (mega-); kGy (1000 Gray = 0.1 MRad); MΩ (Mohm); PAS III (platelet additive solution III); dH₂O (distilled water); IAD (immobilization adsorption device); SCD (sterile connection [connect] device).

One of the examples below refers to HEPES buffer. This buffer contains 8.0 g of 137 mM NaCl, 0.2 g of 2.7 mM KCl, 0.203 g of 1 mM MgCl₂(6H₂0), 1.0 g of 5.6 mM glucose, 1.0 g of 1 mg/ml Bovine Serum Albumin (BSA) (available from Sigma, St. Louis, MO), and 4.8 g of 20 mM HEPES (available from Sigma, St. Louis, MO).

### EXAMPLE 1

### Fiberized Resin With Amberlite® XAD-16

This example compares both the kinetics of removal of aminopsoralens from platelets and platelet function and morphology utilizing fiberized resin and devices containing non-immobilized adsorbent beads. More specifically, fiberized resin comprising immobilized Amberlite® XAD-16 was compared with devices containing free (*i*.*e*., not immobilized) Amberlite® XAD-16 HP and Dowex® XUS-43493.

### Preparation Of Fiberized Resin And Adsorbent Beads

Hoechst Celanese prepared fiberized resin containing Amberlite® XAD-16 obtained from Rohm and Haas in a cleaned and hydrated state. The fibers of Hoechst Celanese's fiber network consisted of a polyethylene terephthalate core and a nylon sheath, the sheath having a lower melting temperature than the core. The fiberized resin was prepared by first evenly distributing the adsorbent beads in the fiber network. Next, the fiber network was rapidly heated causing the polymer sheath of the fibers to melt and bond to the adsorbent beads and other fibers, forming a cross-linked fiber network. The fiberized resin formed contained the Amberlite® XAD-16 at a loading of 130 g/m² *(i.e.,* each square meter of fiber contained 130 g of adsorbent beads).

The fiberized resin was cut into squares (14 cm x 14 cm), and the resulting sections contained approximately 2.5 g of dry Amberlite® XAD-16. The Amberlite® XAD-16 beads were then pre-wet by soaking the fiberized resin in 30% ethanol for approximately 10 minutes. The residual ethanol was then removed by rinsing twice in saline for 10 minutes. Alternative methods of wetting the Amberlite® XAD-16 and other adsorbents are also effective and are contemplated by the present invention. It should be noted that fiberized resin containing other types of beads (*e.g.*, bridged or hypercrosslinked resins like Dowex® XUS-43493) do not require a wetting step for effective psoralen removal.

Amberlite® XAD-16 HP (High Purity) beads were also obtained directly from Rohm and Haas in a cleaned and hydrated state. No pre-wetting was required for the loose (*i.e.,* not immobilized) Amberlite® XAD-16 HP beads prior to incorporation into a mesh pouch; however, the mass of adsorbent was corrected to account for the water content of the beads (2.5 g dry = 6.8 g with 62.8% moisture). The Dowex® XUS-43493 beads were obtained from Dow, and the dry beads did not require wetting nor did the mass of the beads require correction for water. Polyester mesh pouches (7 cm x 7 cm square; 30 µm openings) were then filled with 2.5 g (dry weight) of either the loose Amberlite® XAD-16 HP or Dowex® XUS-43493 beads.

The fiberized resin and adsorbent-containing pouches were sterilized by autoclaving on "wet" cycle for 45 minutes at 121°C. Thereafter, the fiberized resin and the adsorbent-containing pouches were inserted into separate, sterile, 1-liter PL 2410 Plastic containers (Baxter). Following insertion, the PL 2410 Plastic containers were heat sealed in a laminar flow hood, using sterile scissors, hemostats. and an impulse sealer.

Contacting Fiberized Resin And Adsorbent Beads With Psoralen-Containing Platelet Concentrate (PC)

Pools of platelet concentrate were prepared by combining 2-3 units of single donor apheresis platelets in 35% autologous plasma/65% Platelet Additive Solution (*i*.*e*., synthetic media). To this solution was added the aminopsoralen 4'-(4-amino-2-oxa)butyl-4,5',8-trimethyl psoralen in an amount to achieve a final concentration of 150 µM 4'-(4-amino-2-oxa)butyl-4,5',8-trimethyl psoralen. The resulting PC solution was divided into 300 mL units. and the units were then placed in PL 2410 Plastic containers (Baxter) and illuminated with 3 J/cm² of UVA. Following illumination, the treated PCs were transferred into the PL 2410 Plastic containers containing either fiberized resin with immobilized Amberlite® XAD-16, loose Amberlite® XAD-16 HP or loose Dowex® XUS-43493, or into an empty PL 2410 Plastic container as a control. The PL 2410 Plastic containers (Baxter) were then placed on a Helmer platelet incubator at 22°C and agitated at approximately 70 cycles/minute.

Samples of each PC were removed at 1-hour intervals during the first 8 hours of storage for analysis by HPLC of residual 4'-(4-amino-2-oxa)butyl-4,5',8-trimethyl psoralen. Each sample of PC was diluted 5-fold with sample diluent (final concentration = 35% methanol, 25 mM KH₂PO₄, pH = 3.5) containing trimethylpsoralen (TMP) as the internal standard. Proteins and other macromolecules were precipitated by incubating the samples at 4°C for 30 minutes. The samples were then centrifuged and the supernatant was filtered (0.2 µmeter) and analyzed on a C-18 reversed phase column (YMC ODS-AM 4.6 mm x 250 mm) by running a linear gradient from 65% solvent A (25 mM KH₂PO₄, pH = 3.5), 35% B (methanol) to 80% B in 20 minutes.

Platelet yield during a 5-day storage period with the fiberized resin or one of the loose beads was monitored daily by counting platelets on a Baker System 9118 CP (Baker Instrument Co.; Allentown, PA). Blood gases and pH were evaluated using a Ciba-Corning 238 pH/Blood Gas Analyzer. *In vitro* platelet function following 5 days of contact with the fiberized resin or the device containing free adsorbents was evaluated using assays for morphology, shape change, hypotonic shock response, aggregation, and GMP-140 (p-selectin) expression. Shape change, aggregation. and hypotonic shock response were evaluated using a Lumi-Aggregometer (Chrono-Log ), while GMP-140 was determined by flow cytometry using a Becton-Dickinson FACScan Fluorescence Analyzer (Becton Dickinson).

### Psoralen Removal and Platelet Yield and Function

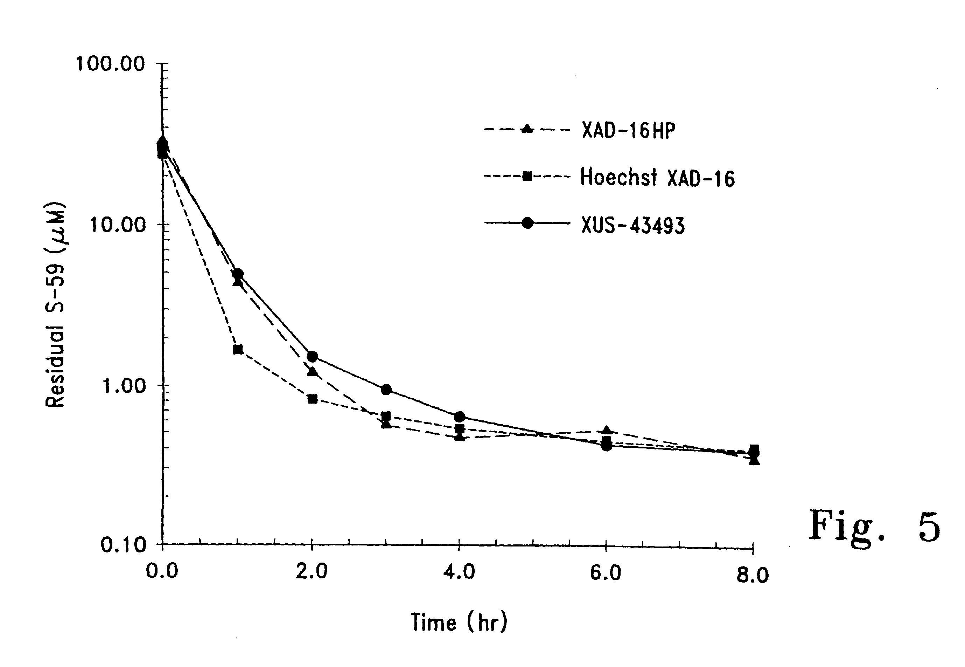

FIG. 5 compares the adsorption kinetics for removal of 4'-(4-amino-2-oxa)butyl-4,5',8-trimethyl psoralen from platelets in 35% plasma/65% synthetic media (PAS III) with XUS-43493, XAD-16 HP, and fiberized resin containing XAD-16. Specifically, the data indicated by the circles connected by the solid line represents the device containing the non-immobilized adsorbent XUS-43493 (2.5 g beads; <5% moisture); the data indicated by the triangles connected by the dashed line represents the device containing non-immobilized XAD-16 HP (6.8 g beads; 62.8% moisture); and the data indicated by the squares connected by the dashed line represents the fiberized resin (Hoechst fibers with XAD-16 beads wet in 30% ethanol; 14 cm x 14 cm). As the data in FIG. 5 indicate, the kinetics of 4'-(4-amino-2-oxa)butyl-4,5',8-trimethyl psoralen adsorption are very comparable for both the device containing non-immobilized adsorbents and device containing the fiberized resin. Thus, the fiberization process does not appear to have a significant impact on the removal kinetics.

In addition, platelet yield and function of the fiberized resin compared to the loose beads were studied. Specifically, the experiments of this study used i) 6.8 g XAD-16 HP (62.8% moisture); ii) 14 cm x 14 cm fiberized XAD-16 (130 g resin/cm²) wet in 30% ethanol; and iii) 2.5 g XUS-43493 (< 5% moisture). Duplicate platelet units were prepared for the XAD-16 HP and the fiberized resin samples, but only a single platelet unit was prepared for the XUS-43493 sample. The results are set forth in Table 1.

As indicated in Table 1, the day-5 pH and pO₂ values were slightly elevated relative to the day 5 control for samples containing non-immobilized beads (XAD-16 HP and XUS-43493). The experiment with the fiberized resin had pH and pO₂ values which were more comparable to the control. Platelet counts indicated a 9-22% platelet loss following 5 days of contact with the fiberized media and the device containing non-immobilized adsorbents. As set forth in Table 1, the fiberized resin gave better yields (9% loss on day 5) and performed better in all *in vitro* assays when compared to device containing non-immobilized XAD-16 or XUS-43493 adsorbent particles.

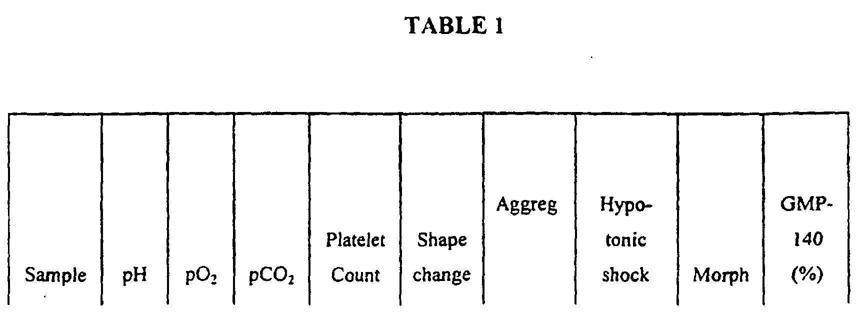

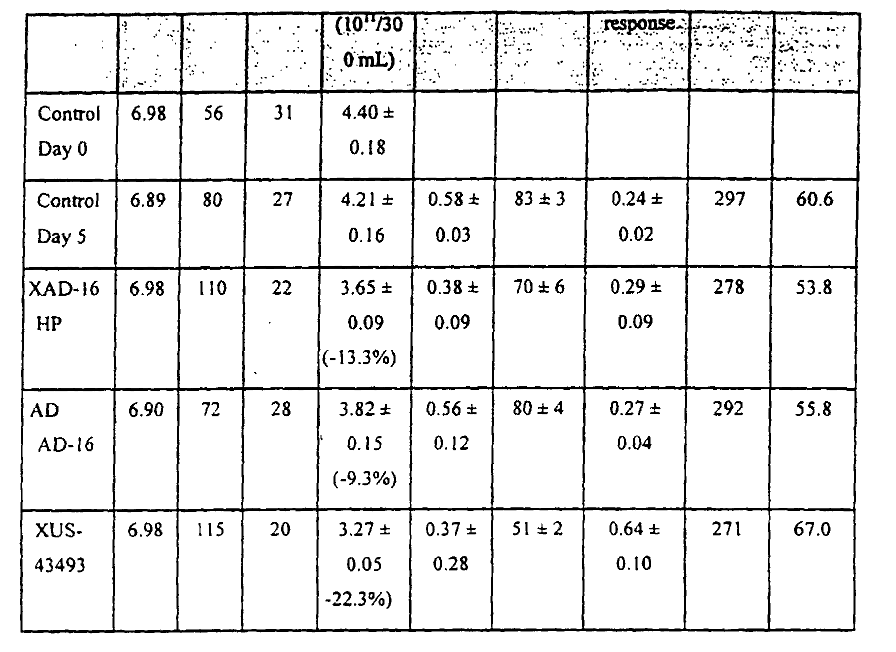

Though an understanding of the mechanism underlying the higher pH and pO₂ values observed for the device containing non-immobilized XUS-43493 and XAD-16 HP is not required in order to practice the present invention, the higher values are believed to be caused by a slight decrease in the metabolism of the platelets in the presence of the device containing non-immobilized adsorbent. By comparison, the fiberized media consistently gave day-5 pH and pO₂ values which were more comparable to the control than the XUS-43493 or XAD-16 beads.

The day-5 platelet yields were also better for the fiberized media relative to the XUS-43493 and XAD-16 HP adsorbent beads. The 22-28% loss which was observed for the XUS-43493 media was observed on several occasions. However, it should be noted that the current preferred embodiment for a device containing non-immobilized adsorbent with XUS-43493 involves transfer of the platelets from this device after 8 hours of exposure; this procedure results in < 5% loss in platelets.

The data presented in Tables 1 indicates that the fiberized media gives a higher platelet yield relative to devices containing non-immobilized adsorbents. Surprisingly, the fiberized media also results in better day-5 platelet function as indicated by pH/pO₂, shape change, aggregation, morphology and GMP-140. While an understanding of the rationale for the enhanced performance of the fiberized media is not required to practice the present invention, several hypotheses can be proposed. First, the fibers which are attached to the surface of the adsorbent beads may hinder interaction between platelets and the surface of the beads. Second, immobilizing the beads may prevent the beads from interacting and eliminate mechanical effects that are detrimental to platelets. Third, immobilizing the beads may enhance fluid shear at the bead surface, thereby decreasing interaction between platelets and the surface of the beads; by comparison, non-immobilized beads are free to flow with the fluid resulting in low flow of fluid relative to the surface of the bead.

### Platelet Loss

When reviewing the above data, it appears that there is some variability in platelet loss from one study to the next. However, the platelet loss expressed as a percentage of the day-5 control count is smaller for studies where the initial platelet count is higher. A study was performed in order to confirm whether the *number* of platelets that are lost is constant for a given area of material available for platelet adhesion. For this study, two platelet units were pooled and the pool was divided into two samples. One sample was diluted in half with 35% autologous plasma/65% synthetic media (PAS) so that the platelet count was half of the other unit. The platelet mixtures were treated with 4'-(4-amino-2-oxa)butyl-4,5',8-trimethyl psoralen + UVA and were contacted with a device containing non-immobilized adsorbent (2.5 g XUS-43493) for 5 days under the previously discussed standard platelet storage conditions.

The total number of platelets that was lost was virtually identical for the two units, while the losses calculated as a percent differed greatly. Thus, the results indicate that total platelet loss appears to be essentially constant after a period of time; that is, while the percentage of platelet loss varies with the initial platelet count, the total number of platelets lost will be approximately constant when equilibrium is reached. Based on the results set forth in this example, the fiberized resin does not have a negative effect on *in vitro* platelet function.

### EXAMPLE 2

### Fiberized Resin With Activated Charcoal

This example compares the kinetics of removal of 4'-(4-amino-2-oxa)butyl-4,5',8-trimethyl psoralen from platelets and platelet function and morphology for fiberized resin comprising Amberlite® XAD-16 and for fiberized resin comprising immobilized activated charcoal.

### Preparation Of Fiberized Resin

Hoechst Celanese prepared fiberized resin containing Amberlite® XAD-16 HP (Rohm and Haas). The fiberized resin containing the Amberlite® XAD-16 was prepared as described in the preceding example, including the 30% ethanol wetting step. Hoechst Celanese also prepared fiberized resin containing immobilized activated charcoal (WestVaco [(Luke, W. Va.)] at a loading of 375 g/m² (AQF-375-B) and 500 g/m² (AQF-500-B). #In a preferred embodiment, the adsorbent particles is a synthetic activated carbon, including for example, Ambersorb and A-Supra. Synthetic activated carbons are preferred due to their ability to eliminate the amount of particulate material shed from the immobilized adsorption medium, this fiberized resin was prepared in a method analogous to that for the fiberized resin containing Amberlite® XAD-16. The composition of the fibers for each fiberized resin was the same.

The fiberized resin was cut into squares (14 cm x 14 cm); the resulting sections contained approximately 2.5 g of dry Amberlite® XAD-16. Next. the fiberized resin was sterilized by autoclaving on "wet" cycle for 45 minutes at 121°C. Thereafter, the fiberized resin were inserted into separate sterile, 1-liter PL 2410 Plastic containers (Baxter), and the containers were heat sealed in a laminar flow hood, using sterile scissors, hemostats, and an impulse sealer.

### Contacting Fiberized Resin With Psoralen-Containing PC

Pools of platelet concentrate were prepared by combining 2-3 units of single donor apheresis platelets in 35% autologous plasma/65% synthetic media (PAS III). 4'-(4-amino-2-oxa)butyl-4,5',8-trimethyl psoralen was added in an amount to achieve a final concentration of 150 µM 4'-(4-amino-2-oxa)butyl-4,5',8-trimethyl psoralen. The resulting PC solution was divided into 300 mL units, and the units were placed in PL 2410 Plastic containers (Baxter) and illuminated with 3 J/cm² of WA. Following illumination, the treated PCs were transferred into the PL 2410 Plastic containers containing fiberized resin with either XAD-16, AQF-375-B, AQF-500-B or into an empty PL 2410 Plastic container as a control. The PL 2410 Plastic containers were then placed on a Helmer platelet incubator at 22°C and agitated at approximately 70 cycles/minute.

As performed in the preceding example, samples of each PC were removed at 1-hour intervals during the first 8 hours of storage for analysis of residual 4'-(4-amino-2-oxa)butyl-4,5',8-trimethyl psoralen by HPLC. Each sample of PC was diluted 5-fold with sample diluent (final concentration = 35% methanol, 25 mM KH₂PO₄, pH = 3.5) containing trimethylpsoralen (TMP) as the internal standard. Proteins and other macromolecules were precipitated by incubating the samples at 4°C for 30 minutes. The samples were then centrifuged and the supernatant was filtered (0.2 µmeter) and analyzed on a C-18 reversed phase column (YMC ODS-AM 4.6 mm x 250 mm) by running a linear gradient from 65% solvent A (25 mM KH₂PO₄, pH = 3:5), 35% B (methanol) to 80% in 20 minutes.

Platelet yield during a 5-day storage period with the fiberized resin or the device containing non-immobilized adsorbent was monitored daily by counting platelets on a Baker System 9118 CP. Blood gases and pH were evaluated using a Ciba-Corning 238 pH/Blood Gas Analyzer. *In vitro* platelet function following 5 days of contact with the fiberized resin or the device containing non-immobilized adsorbent was evaluated using assays for morphology, shape change, hypotonic shock response, aggregation, and GMP-140 (p-selectin) expression. Shape change, aggregation, and hypotonic shock response were evaluated using a Chrono-Log Lumi-Aggregometer, while GMP-140 was determined by flow cytometry using a Becton-Dickinson FACScan Fluorescence Analyzer.

### Psoralen Removal and Platelet Yield and Function

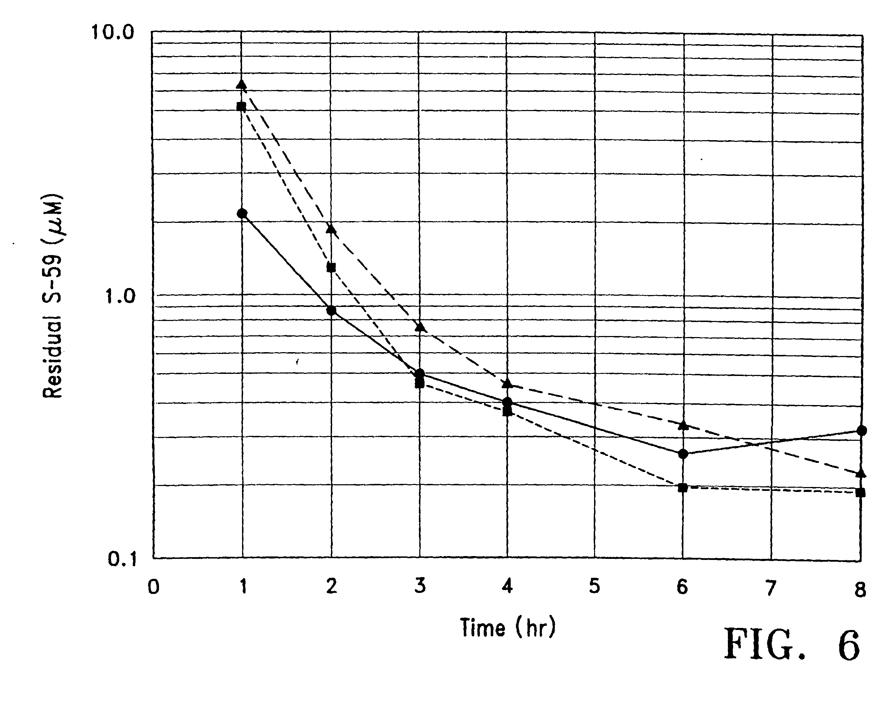

FIG. 6 compares the adsorption kinetics for removal of 4'-(4-amino-2-oxa)butyl-4,5',8-trimethyl psoralen from platelets in 35% plasma/65% synthetic media (PAS III) with fiberized resin containing XAD-16 and fiberized resin with the two different loadings of activated charcoal. Specifically, the data indicated by the circles represents 4'-(4-amino-2-oxa)butyl-4,5',8-trimethyl psoralen removal with fiberized XAD-16 beads; the data indicated by the squares represents removal with fiberized AQF-500-B; and the data indicated by the triangles represents removal with fiberized AQF-375-B. As the data in FIG. 6 indicate, the kinetics of 4'-(4-amino-2-oxa)butyl-4,5',8-trimethyl psoralen adsorption are very comparable for the different fiberized resin, and the fiberized resin all showed very good kinetics of removal (≤ 0.5 µM residual 4'-(4-amino-2-oxa)butyl-4,5',8-trimethyl psoralen after 4 hours).

Platelet yield and *in vitro* platelet function for each of the fiberized resin were also evaluated and the data are summarized in Table 2.

**TABLE 2**

| **Sample** | **pH** | **pO**_{**2**} | **pCO**_{**2**} | **Platelet Count** **(10**^{**11**}**/300 mL)** | **Shape Change** | **Aggregation** |
|---|---|---|---|---|---|---|
| Control Day 0 | 7.07 | 59 | 23 | 3.34 ± 0.13 | 1.17 ± 0.07 | 90 ± 5 |
| Control Day 5 | 6.88 | 112 | 21 | 3.09 ± 0.22 | 0.52 ± 0.02 | 72 ± 8 |
| Fiberized Resin with XAD-16 | 6.93 | 117 | 20 | 2.55 *±* 0.16 (-17%) | 0.39 ± 0.13 | 69 ± 6 |
| Fiberized Resin with AQF-500-B | 7.07 | 100 | 20 | 2.83 ± 0.04 (-8%) | 0.74 ± 0.05 | 61 ± 0 |
| Fiberized Resin with AQF-375-B | 6.99 | 107 | 20 | 2.82 ± 0.16 (-9%) | 0.46 ± 0.06 | 65 ± 2 |

Referring to Table 2, the charcoal-based fiberized resin gave good platelet yields with losses of less than 10%; as in the studies of the preceding example, the fiberized resin containing XAD-16 had a slightly higher platelet loss (about 17%). Regarding pO₂, the day 5 values for the charcoal fiberized resin are comparable to the control. Though an understanding of why the charcoal fiberized resin had slightly elevated pH values is not required to practice the present invention, it may be an artifact caused by residual extractables (*e*.*g*., phosphate) from the activation process; the rapid rise in pH (pH = 7.3-7.4) observed after 8 hours of storage of the PC with the charcoal-based fiberized resin supports that idea. Experiments with USP charcoals, which are associated with fewer extractables, may eliminate the observed initial rise in pH.

The charcoal-based fiberized resin provided good results in both the shape change and aggregation assays. Although the shape change result for the AQF-500-B fiberized resin is better than that of the control, the platelets associated with AQF-500-B performed slightly poorer in the aggregation assay.

### EXAMPLE 3

### Effect of pHEMA Coating On Adsorbent Hemocompatibility

This example compares the kinetics of removal of 4'-(4-amino-2-oxa)butyl-4,5',8-trimethyl psoralen from platelets and platelet function and morphology for both Dowex® XUS-43493 and fiberized resin containing Amberlite® XAD-16 coated with pHEMA.

### Preparation Of pHEMA-Coated Adsorbent Beads And Fiberized Resin

Dowex® XUS-43493 (commercially known as Optipore® L493) containing approximately 50% water by weight was obtained from Dow, and polymerized HEMA with a viscosity average molecular weight of 300 kD was obtained from Scientific Polymer Products. Prior to coating, the adsorbent beads were dried to a water content of < 5%. A stock solution of pHEMA was prepared by dissolving the polymer in 95% denatured ethanol/5% water to achieve a pHEMA concentration of 50 mg/ml.

The coating process was performed by International Processing Corp. in a 9-inch Wurster fluidized bed coater with a charge of approximately 4 kg (dry) of adsorbent. The coating process involved a pHEMA flow rate of 60-70 g/min, an inlet temperature of 50°C, and an air flow rate of approximately 200 ft³/min. Samples (50 g) of coated adsorbent were removed during the coating process so that coating levels ranging from 3-18% (w/w) pHEMA were obtained; adsorbent beads coated with 3.7%, 7.3%, and 10.9% pHEMA (w/w) were used in the studies described below.

A device containing non-immobilized dry (uncoated) Dowex® XUS-43493 (2.5 g) and pHEMA-coated Dowex® XUS-43493 (3.0 g or 5.0 g) were prepared by placing the desired mass of adsorbent into a square 30 µm polyester mesh pouch (7 cm x 7 cm). The adsorbent-filled pouches were inserted into separate sterile, 1-liter PL 2410 Plastic containers (Baxter) and heat sealed with an impulse sealer. Thereafter, the adsorbent-filled pouches containing PL-2410 Plastic containers were sterilized by either E-beam (NIS) or gamma irradiation (SteriGenics) to 2.5 MRad; as previously alluded to, E-beam sterilization is generally preferred.

Hoechst Celanese prepared fiberized resin containing Amberlite® XAD-16 according to the method described in Example 1. The fiberized resin was cut into squares (14 cm x 14 cm); the resulting sections contained approximately 2.5 g of dry Amberlite® XAD-16. The Amberlite® XAD-16 of the fiberized resin was simultaneously wet and coated with pHEMA by soaking in a solution containing 50 mg/mL pHEMA in 95% ethanol/5% distilled water. Residual ethanol was removed by rinsing twice in saline for 10 minutes. This procedure resulted in a coating of approximately 6% (w/w) pHEMA. The fiberized resin was then sterilized by autoclaving on "wet" cycle for 45 minutes at 121°C. Thereafter, the fiberized resin was inserted into separate sterile, 1-liter PL 2410 Plastic containers (Baxter) and heat sealed in a laminar flow hood, using sterile scissors, hemostats, and an impulse sealer.

### Contacting pHEMA-Coated Adsorbent Beads And Fiberized Resin With Psoralen-Containing PC

Pools of platelet concentrate were prepared by combining units of single donor apheresis platelets in 35% autologous plasma/65% synthetic media (PAS III). 4'-(4-amino-2-oxa)butyl-4,5',8-trimethyl psoralen was added in an amount to achieve a final concentration of 150 µM 4'-(4-amino-2-oxa)butyl-4,5',8-trimethyl psoralen. The resulting PC solution was then divided into 300 mL units, and the units were placed in PL 2410 Plastic containers (Baxter) and illuminated with 3 J/cm² of UVA. Following illumination, the treated PCs were transferred into the PL 2410 Plastic containers containing either the device containing non-immobilized pHEMA-coated Dowex® XUS-43493, the fiberized pHEMA-coated fiberized resin with Amberlite® XAD-16, or into an empty PL 2410 Plastic container as a control. The PL 2410 Plastic containers were then placed on a Helmer platelet incubator at 22°C and agitated at approximately 70 cycles/minute.

Samples of each PC were removed at 1-hour intervals during the first 8 hours of storage for analysis of residual 4'-(4-amino-2-oxa)butyl-4,5',8-trimethyl psoralen by HPLC. Each sample of PC was diluted 5-fold with sample diluent (final concentration = 35% methanol, 25 mM KH₂PO₄, pH = 3.5) containing trimethylpsoralen (TMP) as the internal standard. Proteins and other macromolecules were precipitated by incubating the samples at 4°C for 30 minutes. The samples were then centrifuged and the supernatant was filtered (0.2 µmeter) and analyzed on a C-18 reversed phase column (YMC ODS-AM 4.6 mm x 250 mm) by running a linear gradient from 65% solvent A (25 mM KH₂PO₄, pH = 3.5), 35% B (methanol) to 80% in 20 minutes.

Platelet yield after a 5-day storage period with the fiberized resin or the device containing non-immobilized adsorbent was determined by counting platelets on a Baker System 9118 CP. Blood gases and pH were evaluated using a Ciba-Corning 238 pH/Blood Gas Analyzer. *In vitro* platelet function following 5 days of contact with the fiberized resin or the device containing non-immobilized adsorbent was evaluated using assays for morphology, shape change, hypotonic shock response, aggregation, and GMP-140 (p-selectin) expression. Shape change, aggregation, and hypotonic shock response were evaluated using a Chrono-Log Lumi-Aggregometer, while GMP-140 was determined by flow cytometry using a Becton-Dickinson FACScan Fluorescence Analyzer.

### Effect Of pHEMA Coating On Psoralen Removal

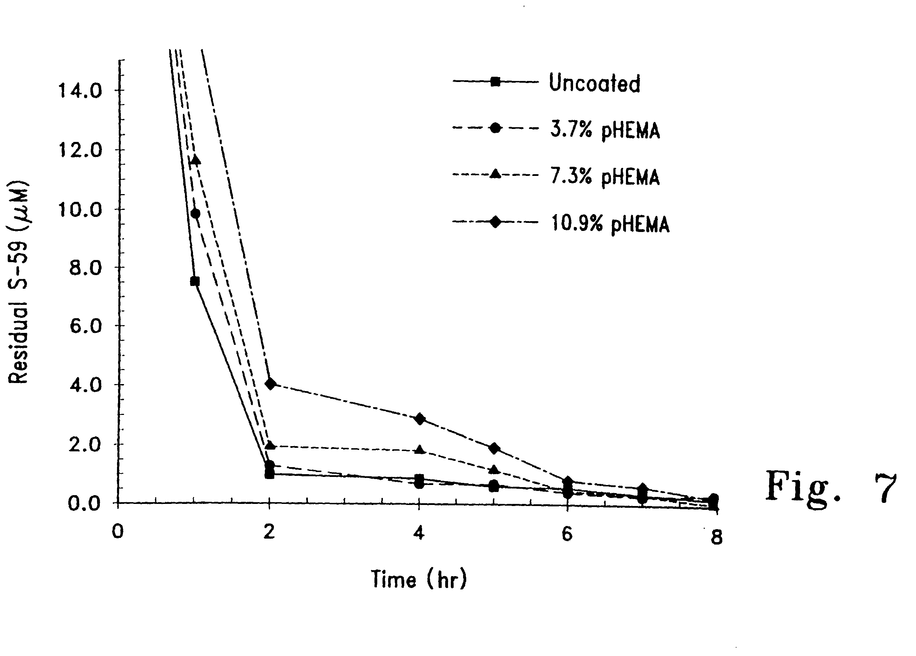

FIG. 7 compares the adsorption kinetics for removal of 4'-(4-amino-2-oxa)butyl-4,5',8-trimethyl psoralen from platelets in 35% plasma/65% synthetic media (PAS III) with pHEMA-coated and uncoated Dowex® XUS-43493 beads. Specifically, 4'-(4-amino-2-oxa)butyl-4,5',8-trimethyl psoralen removal with 3.0 g Dowex® XUS-43493 coated with 3.7% (w/w) pHEMA is represented by the circles, with 7.3% (w/w) pHEMA is represented by the triangles, and with 10.9% (w/w) pHEMA is represented by the diamonds; the squares represent 4'-(4-amino-2-oxa)butyl-4,5',8-trimethyl psoralen removal with 2.5 g (dry) uncoated Dowex® XUS-43493. As the data in FIG. 7 indicate, the kinetics of 4'-(4-amino-2-oxa)butyl-4,5',8-trimethyl psoralen adsorption decreased as the level of pHEMA coating was increased. While the mechanism need not be understood in order to practice the invention, this decrease is believed to be due to an increase in resistance to diffusion of 4'-(4-amino-2-oxa)butyl-4,5',8-trimethyl psoralen to the interior of the adsorbent particles.

The adsorption kinetics of 4'-(4-amino-2-oxa)butyl-4,5',8-trimethyl psoralen removal were also determined with 5.0 g Dowex® XUS-43493 coated with 3.7%, 7.3%, and 10.9% (w/w) pHEMA. The results (not shown) indicated that the removal kinetics for the beads coated with 10.9% pHEMA were comparable to that with the uncoated (control) beads.

### Effect Of pHEMA Coating On Platelet Yield

The effect of pHEMA on platelet yield was determined in two studies using different amounts of adsorbent (3.0 and 5.0 g) but the same levels of pHEMA coating (3.7%, 7.3%, and 10.9% [w/w]) in each. Platelet yields were calculated relative to the platelet count on day 5 for treated PC which was not contacted with a device containing non-immobilized adsorbents. As the results in Table 3 indicate, when 3.0 g of XUS-43493 were tested, there was a nominal dose response on day 5 platelet yield with increasing pHEMA coating levels; those results suggest that a low level of pHEMA coating may be most effective since it has a smaller effect on 4'-(4-amino-2-oxa)butyl-4,5',8-trimethyl psoralen removal kinetics while still inhibiting platelet adhesion to the adsorbent surface. In contrast to the nominal effect seen with 3.0 g of XUS-43493, a dose response was observed when 5.0 g were tested - increasing pHEMA coating levels did increase the day 5 platelet yield. However, yields were still lower than those observed when 3.0 g of adsorbent beads were used.

**TABLE 3**

| **Polymer Coating** | **Coating Level (%)** | **Adsorbent Mass (g)** | **Platelet Yield, Day 5(%)*** |
|---|---|---|---|
| None | 0 | 2.5 | 73.1 |
| pHEMA | 3.7 | 3.0 | 91.4 |
| pHEMA | 7.3 | 3.0 | 87.7 |
| pHEMA | 10.9 | 3.0 | 89.0 |
| pHEMA | 3.7 | 5.0 | 71.8 |
| pHEMA | 7.3 | 5.0 | 80.2 |
| pHEMA | 10.9 | 5.0 | 86.6 |

The results presented in Table 3 suggest that the use of a lower mass of adsorbent (*e*.*g*., 2.5 - 3.0 g) along with a low level of pHEMA coating (*e*.*g*., ≤ 3.0% w/w) will provide the best platelet yield. As previously indicated, the optimum level of pHEMA coating is the minimum coating at which a protective effect on platelet yield and *in vitro* platelet function is observed.

### Effect Of pHEMA Coating And Sterilization On Platelet Function

It was previously indicated that methods of sterilization may have a substantial effect on adsorbent function since the pHEMA coating can be crosslinked or cleaved by the radiation. In order to evaluate this effect, a study was performed with devices containing non-immobilized pHEMA-coated (3.7% w/w) and uncoated XUS-43493 sterilized with either 2.5 MRad E-beam or 2.5 MRad gamma irradiation. Each device contained 2.5 g (dry) of non-immobilized coated or uncoated XUS-43493 housed in a square 30 µm polyester mesh pouch (7 cm x 7 cm); the control comprised treated PC stored in a PL 2410 Plastic container alone. The results are summarized in Table 4.

**TABLE 4**

| **Sample** | **pH** | **pO**_{**2**} | **Platelet Count** **(10**^{**11**}**/300 L)** | **Shape Change** | **Aggregation** | **HSR** | **Morphology** | **CM-140 Activation (%)** |
|---|---|---|---|---|---|---|---|---|
| Control Day 0 | 7.03 | 67 | 4.94 ± 0.24 | - | - | - | - | - |
| Control Day 5 | 6.87 | 84 | 4.58 ± 0.08 (-0%) | 0.96 ± 0.05 | 76 ± 2 | 0.35 ± 0.03 | 295 | 70.0 |
| Uncoated XUS-43493 | 6.95 | 128 | 3.49 ± 0.08 (-23.8%) | 0.38 ± 0.00 | 44 ± 0 | 0.47 ± 0.12 | 260 | 58.3 |
| 3.7% pHEMA XUS-43493 Gamma | 6.93 | 108 | 4.19 ± 0.09 (-8.5%) | 0.59 ± 0.03 | 59 ± 5 | 0.32 ± 0.04 | 240 | 58.5 |
| 3.7% pHEMA XUS-43493 E-beam | 6.88 | 92 | 4.35 ± 0.11 (-5.0%) | 0.71 ± 0.06 | 69 ± 9 | 0.40 ± 0.04 | 264 | 54.8 |

Referring to Table 4, the day-5 pH values appear to be very stable compared to the control. The pO₂ value measured with the device containing non-immobilized uncoated adsorbent is slightly elevated, suggesting a mild decrease in metabolism; coating with pHEMA appeared to reduce this effect, with the results for the E-beam sterilized device containing non-immobilized adsorbent closer to the control than those for the gamma sterilized device.

Platelet yields were very good for both of the pHEMA-coated samples, the E-beam sterilized sample performing slightly better. Shape change and aggregation exhibited a pattern similar to that for yield, with the device containing non-immobilized uncoated adsorbent giving the lowest values and the pHEMA-coated/E-beam sterilized sample providing higher values similar to the control. The samples treated with a device containing non-immobilized adsorbents performed as well as or better than the control in the hypotonic shock response (HSR) assay. Samples that were treated with the devices containing non-immobilized adsorbents gave lower morphology scores than the control, but showed lower levels of activation as indicated by the GMP-140 assay.

Another hemocompatibility study was performed comparing a device containing non-immobilized uncoated XUS-43493 (2.5 g beads; 30 µm polyester mesh pouch; 7 cm x 7 cm), uncoated fiberized resin (14 cm x 14 cm) containing Amberlite® XAD-16, and fiberized resin containing pHEMA-coated Amberlite® XAD-16. The favorable effect of pHEMA on the fiberized resin is indicated by the results presented in Table 5

**TABLE 5**

| **Sample** | **pH** | **pO**_{**2**} | **Platelet Count** **(10**^{**11**}**/300 L)** | **Shape Change** | **Aggregation** | **HSR** | **Morphology** | **GM-140 Activation (%)** |
|---|---|---|---|---|---|---|---|---|
| Control Day 0 | 7.11 | 50 | 3.01 ± 0.10 | - | - | - | - | - |
| Control Day 5 | 6.91 | 122 | 2.85 ± 0.16 (-0%) | 0.60 ± 0.09 | 78 ± 4 | 0.31 ± 0.01 | 302 | 72.5 |
| Uncoated XUS-43493 | 7.00 | 151 | 2.11 ± 0.07 (-26.0%) | 0.35 ± 0.03 | 55 ± 4 | 0.57 ± 0.03 | 267 | 68.7 |
| Uncoated Fiberized Resin with XAD-16 | 6.94 | 126 | 2.23 ± 0.13 (-21.8%) | 0.52 ± 0.03 | 82 ± 4 | 0.45 ± 0.01 | 297 | 65.3 |
| pHEMA-Fiberized Resin with XAD-16 | 6.95 | 120 | 2.44 ± 0.11 (-14.4%) | 0.59 ± 0.01 | 86 ± 5 | 0.46 ± 0.02 | 305 | 63.0 |

As indicated by the data regarding *in vitro* platelet function and platelet yield in Table 5, coating with pHEMA brought the pO₂ values for the fiberized resin closer to that observed for the control. The pHEMA-coated fiberized resin also exhibited higher platelet yield than the uncoated fiberized resin. The results indicate that the pHEMA-coated fiberized resin performed better than the uncoated XUS-43493 beads in all *in vitro* platelet function assays. Moreover, the uncoated fiberized resin performed better than the uncoated XUS-43493 beads in most *in vitro* platelet function assays.

### EXAMPLE 4

### Effect Of Glycerol And Polyethylene Glycol On Adsorbent Capacity

This example examines the effect of glycerol and polyethylene glycol as stabilizing agents on adsorbent capacity and kinetics of removal of aminopsoralens from plasma. Free (*i.e.*, not fiberized) Amberlite® XAD-16 and Dowex® XUS-43493 adsorbent beads were used in the experiments of this example.

### Methodology

Amberlite® XAD-16 HP (Rohm & Haas (Philadelphia, PA)) and Dowex® XUS-43493 (Supelco, Bellefonte, PA) were dried to < 5% water in a 80°C oven. Known masses of adsorbent were soaked in ethanol solutions containing 0-50% glycerol, 50% PEG-200 or 50% PEG-400 (glycerol, PEG-200, and PEG-400 from Sigma). Following a 15 minute incubation period at room temperature, the excess solvent was removed and the samples were dried overnight in a 80°C oven; drying the adsorbent at temperatures > 120°C was avoided since changes in adsorbent properties (*e.g.,* pore melting) were previously observed at higher temperatures. After drying, adsorbent samples were weighed to determine the mass of stabilizing agent per mass of adsorbent.

Several individual studies were performed. Control samples of "non-wet" adsorbent and "optimally wet" adsorbent were included in the studies as described below. The non-wet samples of adsorbent were dried adsorbent which was not subjected to any pre-treatment, while the optimally wet samples of adsorbent were prepared by wetting the adsorbent with 30% ethanol/70% dH₂O. The optimally-wet adsorbent was rinsed with dH₂O to remove residual ethanol. The adsorbent was prepared just prior to the adsorption study to assure that drying did not occur.

Each of the adsorption studies was performed using 100% human plasma containing 150 µM 4'-(4-amino-2-oxa)butyl-4,5',8-trimethyl psoralen spiked with ³H-4'-(4-amino-2-oxa)butyl-4,5',8-trimethyl psoralen. Plasma (6.0 mL) was added to vials containing adsorbent treated with different stabilizing agents. Masses of adsorbent were corrected for glycerol or PEG content to give 0.2 g of adsorbent. The vials were placed on a rotator and agitated at room temperature. Plasma samples were removed at various times and levels of residual ³H-4'-(4-amino-2-oxa)butyl-4,5',8-trimethyl psoralen were determined. Samples (200 µL) were diluted in 5.0 mL of Optiphase HiSafe Liquid Scintillation Cocktail (Wallac) and were counted on a Wallac 1409 Liquid Scintillation Counter (Wallac).

### Adsorption Capacities Of Amberlite® XAD-16 And Dowex® XUS-43493 Treated With Glycerol

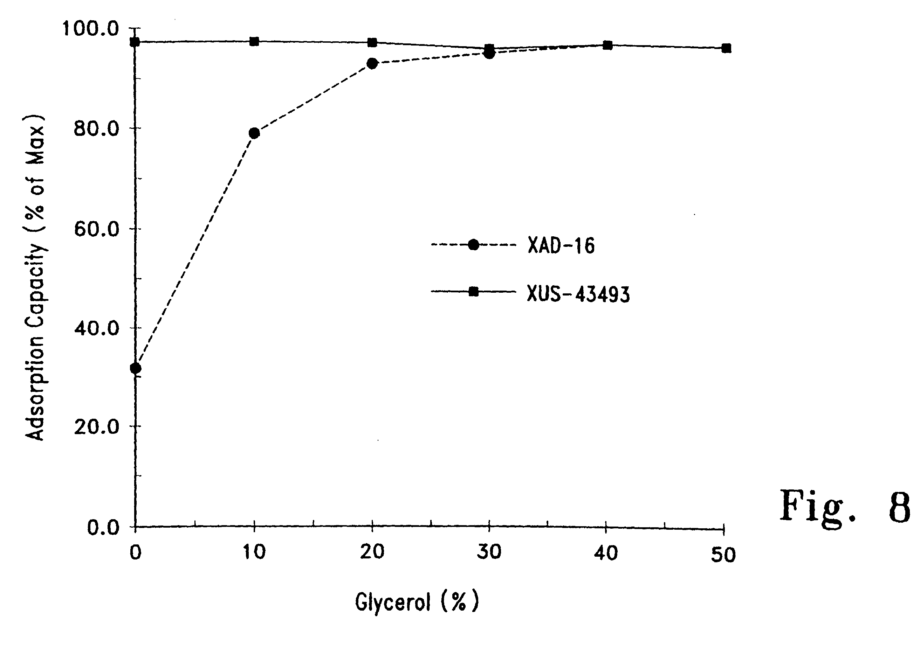

FIG. 8 compares the effect of pre-treatment with ethanol solutions containing various levels of glycerol on relative 4'-(4-amino-2-oxa)butyl-4,5',8-trimethyl psoralen adsorption capacity in 100% plasma for Amberlite® XAD-16 and Dowex® XUS-43493. Adsorbent samples were wet in the ethanol/glycerol solutions for 15 minutes prior to drying for 48 hours at 80°C. Single measurements of adsorption capacity were made after 4 hours of contact. Referring to FIG. 8, glycerol content shown on the x-axis is weight/volume percent of glycerol in ethanol. Adsorption capacities shown on the y-axis are percentages relative to the adsorption capacity of the optimally wet adsorbent sample. The adsorption capacity of XUS-43493 is represented by the squares, while that of XAD-16 is represented by the circles.

As the data in FIG. 8 indicate, the capacity of XAD- 16 increased from about 30% in the dry sample to over 90% in the sample wet in a 20% glycerol solution. These results indicate that very low levels of glycerol are required for maintaining high adsorbent capacity after drying. Control samples that were wet in 50% ethanol/50% dH₂O (no glycerol) prior to drying demonstrated adsorption capacities which were similar to untreated samples that were dried. In contrast, the XUS-43493 samples did not show any effect of glycerol on adsorption capacity; adsorption capacity approached 100% at all levels of glycerol. While not critical to the practice of the present invention, this observation supports the hypothesis that glycerol acts to prevent the adsorbent pores from collapsing during drying; because XUS-43493 has a highly crosslinked structure, it is not subject to pore collapse upon drying.

Samples that were treated with glycerol appeared to be very stable to drying. No changes were observed in adsorption capacity for samples that were stored for 7 days in a laminar flow hood (data not shown).

In a preferred embodiment of the present invention, 2.5 g dry of adsorbent are used for the removal of psoralen and psoralen photoproducts from each unit of platelets. Soaking the adsorbent in 30% glycerol/70% ethanol, followed by drying, results in adsorbent which contains approximately 50% glycerol. A 5.0 g sample of adsorbent would therefore contain 2.5 g dry adsorbent and 2.5 g of glycerol. Thus, a typical 300 mL unit of platelets would contain 0.8% glycerol, a level thought to be acceptable for transfusion.

### Adsorption Capacities Of Amberlite® XAD-16 And Dowex® XUS-43493 Treated With Glycerol Or PEG

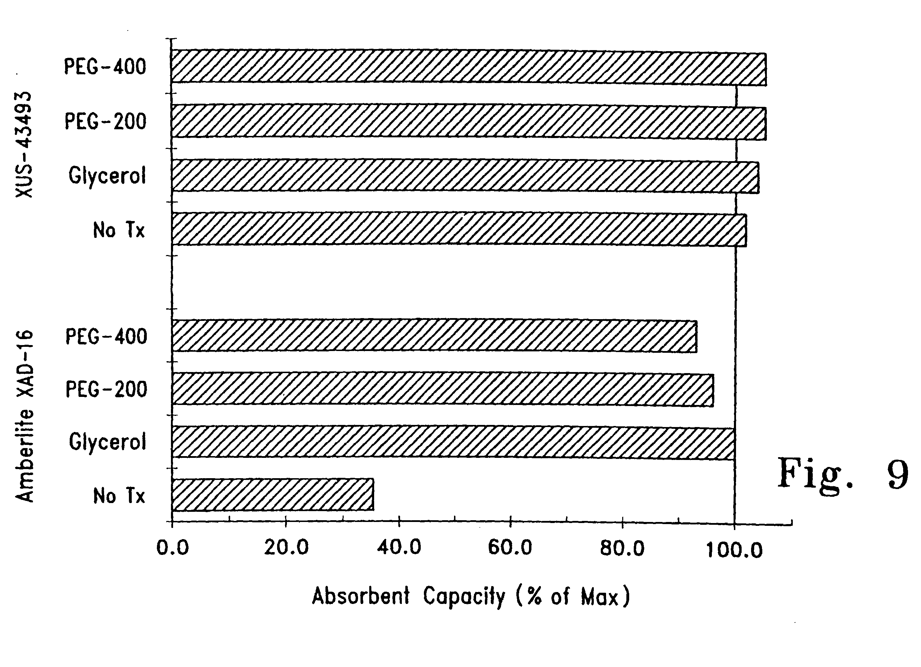

Additional studies were performed with the low molecular weight polyethylene glycols PEG-200 and PEG-400, low-toxicity agents that are nonvolatile and are soluble in ethanol and water. Samples of adsorbent were treated for 15 minutes in 50% solutions of PEG-400, PEG-200 or glycerol in ethanol. FIG. 9 compares the effect of the stabilizing agents on 4'-(4-amino-2-oxa)butyl-4,5',8-trimethyl psoralen adsorption capacities with dried adsorbent in 100% plasma for Amberlite® XAD-16 (bottom) and Dowex® XUS-43493 (top); the samples that were not wet are labeled "No Tx". Adsorbent capacities are reported as percentages relative to the capacity of optimally wet adsorbent.

As indicated by the data in FIG. 9 and predictable based on the its "macronet" structure, the capacity of Dowex® XUS-43493 was not affected by drying ("No Tx" sample). Conversely, the Amberlite® XAD-16 had approximately 35% of the maximum capacity when dried. Treating XAD-16 with glycerol, PEG-200, and PEG-400 all improved the capacity of the dried adsorbent; the adsorbent capacities with each were all greater than 90%, with glycerol>PEG-200>PEG-400. Though an understanding of the precise mechanism of action is not required to practice the present invention, differences in capacity between the glycerol and the two PEG solutions may be caused by decreasing penetration of the stabilizing agent with increasing molecular weight. That is, during the 15 minute application procedure, the glycerol (MW = 92.1) may be able to penetrate the adsorbent pores more completely than either PEG-200 (MW = 190-210) or PEG-400 (MW = 380-420), which diffuse more slowly because of their larger size.

### Adsorption Kinetics Of Amberlite® XAD-16 Treated With Glycerol Or PEG

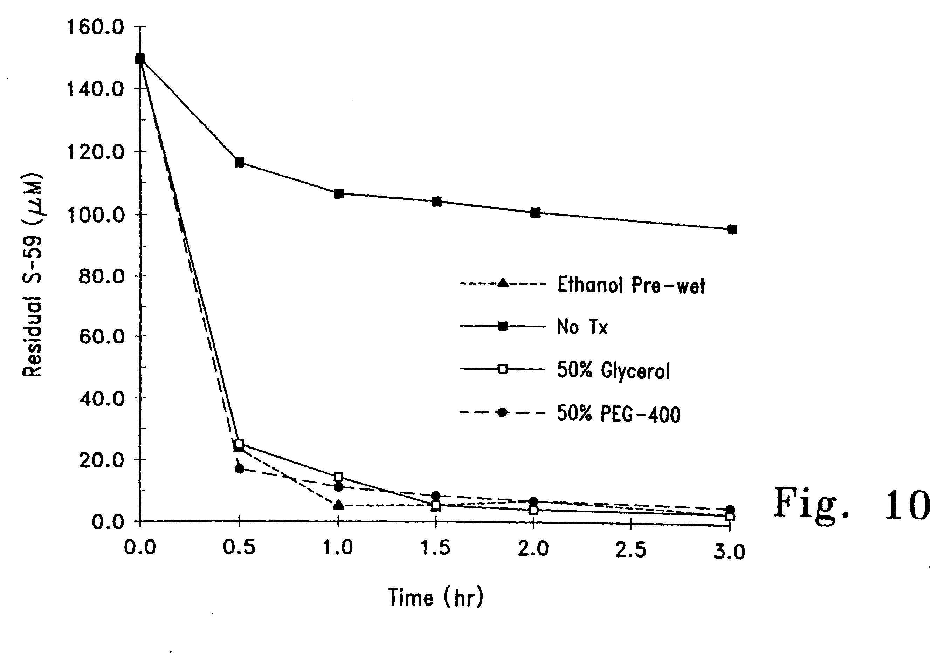

A study was also performed to determine whether filling the pores of the adsorbent with glycerol or PEG results in reduced adsorption kinetics. FIG. 10 compares adsorption of 4'-(4-amino-2-oxa)butyl-4,5',8-trimethyl psoralen over a 3-hour period from 100% plasma using Amberlite® XAD-16 wet in several different solutions. Specifically, the data in FIG. 10 represents XAD-16 i) wet prior to drying with a 50% solution of glycerol (open squares connected by solid lines), ii) wet prior to drying with a 50% solution of PEG-400 (shaded circles connected with dashed lines), iii) pre-wet, *i.e.*, just prior to initiating the study, with 50% ethanol/50% dH₂O (shaded triangles connected by dashes), and iv) not subjected to any treatment (shaded squares connected by solid lines; "No Tx"). The data in FIG. 10 demonstrate that Amberlite® XAD-16 samples that were wet in 50% glycerol/50% ethanol or 50% PEG-400/50% ethanol solutions had adsorption kinetics which were very close to the sample that was optimally wet in ethanol (*i*.*e*., the sample pre-wet with ethanol). The XAD-16 sample that was dried but not treated (No Tx) achieved only about 30% removal by 3 hours.

The data presented in this example indicate that treating Amberlite® XAD-16 with stabilizing agents in the form of solutions containing 50% ethanol and 50% glycerol, PEG-200, or PEG-400 can prevent loss of adsorption capacity associated with drying. The results obtained with these stabilizing agents suggest that low molecular weight wetting agents represent viable methods for enhancing adsorbent function.

### EXAMPLE 5

### Removal Of Methylene Blue From FFP

This example is directed at the ability of a variety of different polymeric adsorbent materials to remove methylene blue from fresh frozen plasma.

The experiments of this example evaluated "free" adsorbent resin (*i*.*e*., not incorporated into device containing non-immobilized adsorbents) and fiberized resin. The free adsorbent resins tested were Amberlite® XAD-16 HP (Rohm and Haas), MN-200 (Purolite), and Dowex® XUS-43493 (Dow Chemical Co.). The XAD-16 HP came in a hydrated state so that no pre-treatment (*i*.*e*., no wetting) was necessary, and the MN-200 was also supplied in a fully hydrated state; the XUS-43493 was dry.

Fiberized resin containing XAD-16 was prepared as generally described in Example 1. Briefly, a 2 cm x 7 cm (*i.e.*, 14 cm²) strip of fiberized resin containing 130 g/m² XAD-16 was first wet in 70% ethanol and then rinsed exhaustively in distilled water.

A stock solution of methylene blue (10 mM) was prepared by dissolving U.S.P. methylene blue (Spectrum) in distilled water. The stock solution of methylene blue was added to a sample of 100% plasma to give a final concentration of 10 µM. Samples of the "free" adsorbent resin (*i*.*e*., XAD-16 HP, MN-200, and XUS-43493) were weighed into 50 mL polypropylene tubes for adsorption studies. The water content of each adsorbent was determined by measuring mass loss upon drying. The mass of each adsorbent was corrected for water content so that the equivalent of 0.25 g dry adsorbent was used for each.

A 30 mL sample of the 100% plasma containing 10 µM methylene blue was added to each vial. The vials were placed on a rotator at room temperature. Samples (200 µL) were removed from each vial at 15 minute intervals and assayed for residual methylene blue by HPLC. Each sample of plasma was diluted 5-fold with sample diluent (final concentration = 35% methanol, 25 mM KH₂PO₄, pH = 3.5). Proteins and other macromolecules were precipitated by incubating the samples at 4°C for 30 minutes. Samples were centrifuged and the supernatant was filtered (0.2 µm) and analyzed on a C-18 reversed phase column (YMC ODS-AM, 4.6 mm x 250 mm) by running a linear gradient from 65% solvent A (25 mM KH₂PO₄, pH = 3.5), 35% B (Methanol) to 80% B in 20 minutes. The limit of detection for the HPLC assay was approximately 0.5 µM methylene blue.

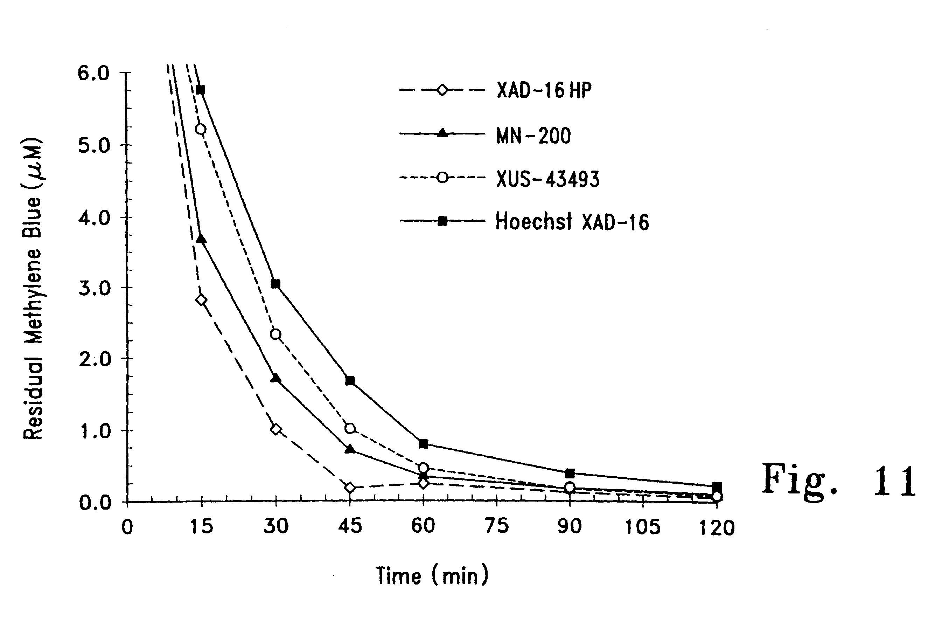

FIG. 11 compares the kinetics of adsorption of methylene blue over a 2-hour period from 100% plasma. Referring to FIG. 11, XAD-16 HP data is represented by open diamonds connected by dashed lines, the MN-200 data is represented by shaded triangles connected by solid lines, the XUS-43493 data is represented by open circles connected by dashed lines, and the fiberized resin containing XAD-16 is represented by shaded squares connected by solid lines. As the data indicate, the XAD-16 HP and MN-200 gave the fastest adsorption kinetics, followed by XUS-43493. The slightly slower kinetics of the XUS-43493 may be a result of slower wetting, as it was used in the dry state. Finally, the fiberized resin containing XAD-16 had the slowest adsorption kinetics. This may have resulted from poor contacting between the fiberized resin and plasma during the batch incubation, as a portion of the 14 cm² strip of fiberized resin was not completely submersed in the plasma throughout the adsorption study, thereby reducing the effective contact area between the adsorbent and plasma.

The data indicate that non-psoralen pathogen-inactivating compounds like the phenothiazine dyes can be removed from blood products using the resins and fiberized resin contemplated for use with the present invention.

### EXAMPLE 6

### Removal Of Acridine Compounds From Packed Red Blood Cells

This example is directed at the ability of a variety of different resin materials to remove acridine compounds from packed red blood cells (PRBCs). More specifically, the experiments of this example evaluate the removal of the acridine compound, 5-[(β-carboxyethyl)amino]acridine, from PRBCs.

The chemical structures of several acridines are depicted in FIG. 12. As indicated in FIG. 12, 9-amino acridine and 5-[(β-carboxyethyl)amino]acridine are aminoacridines.

### Resin Selectivity

Equilibrium adsorption of compound 5-[(β-carboxyethyl)amino]acridine was studied with several types of resins. The polymeric adsorbent resins evaluated were Amberlite® XAD-2, XAD-4, XAD-7, and XAD-16 HP (Rohm and Haas); Purolite® MN-150, MN-170, MN-200, MN-300, MN-400, MN-500, and MN-600; and Dowex® XUS-43493 and XUS-40285 (Dow Chemical Co.). In addition, several Amberlite® anion exchange resins (IRA-958, IRA-900, IRA-35, IRA-410 and IRA-120; Rohm and Haas) and an Amberlite® weak cation exchange resin (DP-1; Rohm and Haas) were tested. Moreover, several charcoals were evaluated, including Hemosorba® AC (Asahi), PICA G277 and Norit A Supra (both commercially available from American Norit). Finally, Porapak® RDx (Waters), a styrene vinyl pyrrolidone copolymer which has affinity for nitro aromatic compounds, was also tested.

Initially, an equilibrium adsorption study was performed with samples of each resin to evaluate capacity for 5-[(β-carboxyethyl)amino]acridine and adenine (6-aminopurine). Approximately 0.1 g of resin was weighed and transferred into a 6 mL polypropylene tube. A 5.0 mL aliquot of 25% plasma/75% Adsol® (Baxter) containing 100 µM of 5-[(β-carboxyethyl)amino]acridine in distilled water was then added to each tube. Cellular products such as red blood cells are typically stored in a medium containing a low percentage of plasma (10-35%) with a balance of synthetic media; Adsol® is one example of a synthetic media that consists of adenine, dextrose, and mannitol in a saline solution. Of course, the present invention contemplates the use of concentrations of acridines other than 100 µM in mixtures of plasma and other synthetic media. Next, the tubes were placed on a tumbling agitator and incubated for 3 hours at room temperature. Following incubation, aliquots of each sample were removed for analysis of residual 5-[(β-carboxyethyl)amino]acridine and adenine by HPLC.

For the HPLC procedure, each sample was diluted 2-fold with sample diluent (50% methanol, 25 mM KH₂PO₄, pH = 3.5), and proteins and other macromolecules were precipitated by incubating the samples at 4°C for 30 minutes. The samples were then centrifuged and the supernatant was filtered (0.2 µmeter) and analyzed on a C-18 reversed phase column (YMC ODS-AM 4.6 mm x 250 mm) by running a linear gradient from 75% solvent A (25 mM KH₂PO₄, pH = 3.5), 25% B (methanol) to 80% B in 20 minutes. For 5-[(β-carboxyethyl)amino]acridine removal, estimated capacities (µmole/g) at C_{f}= 1 µM were determined from single adsorption measurements with Cₒ = 100 µM; the results are set forth in the second column of Table 6 (ND = not detectable). For adenine removal, estimated capacities (mmole/g) at C_{f} = 1 mM were estimated from single adsorption measurements with Cₒ = 1.5 mM. The results arc set forth in the third column of Table 6 (ND = not detectable).

**TABLE 6**

| Resin | Estimated 5-[(β-carboxyethyl)amino]acridine Capacity (µmole/g) at C_{f} = 1µM | Adenine Capacity (mmole/g) at C_{f} = 1 mM |
|---|---|---|
| XAD-2 | 0.1 | 0.00 |
| XAD-4 | 3.2 | 0.02 |
| XAD-7 | 0.1 | 0.01 |
| XAD-16HP | 4.3 | 0.03 |
| XUS-43493 | 17.5 | 0.46 |
| XUS-40285 | 6.8 | 0.27 |
| MN-150 | 1.6 | 0.24 |
| MN-170 | 4.2 | 0.43 |
| MN-200 | 8.9 | 0.46 |
| MN-300 | 5.1 | 0.33 |
| MN-400 | 4.4 | 0.22 |
| MN-500 | 8.0 | 1.17 |
| MN-600 | 5.8 | 0.36 |
| IRA-958 | 0.0 | 0.00 |
| IRA-900 | 0.0 | 0.01 |
| DP-1 | 0.0 | 0.00 |
| IRA-35 | 0.0 | 0.01 |
| IRA-120 | 4.7 | 0.41 |
| Hemosorba AC | ND | ND |
| PICA G277 | 5.0 | ND |
| Norit A Supra | ND | ND |
| Porapak RDx | 0.1 | 0.01 |
| IRA-410-D | 0.0 | 0.00 |

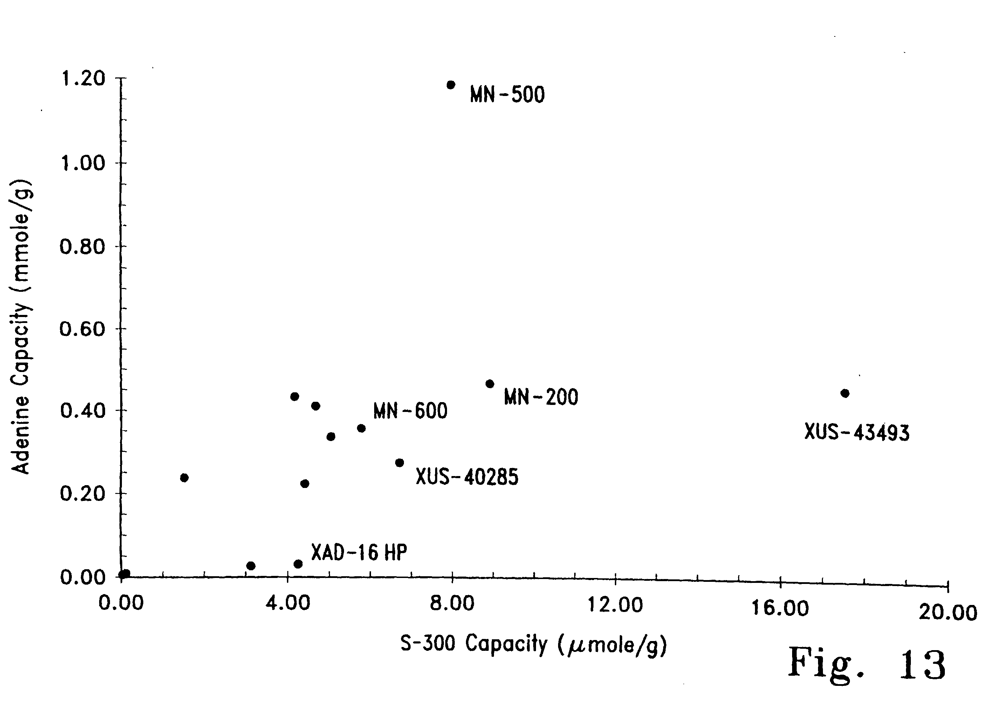

While the use of any resin capable of adsorbing acridine compounds is contemplated, preferred resins selectively adsorb 5-[(β-carboxyethyl)amino]acridine over adenine and exhibit low hemolysis. FIG. 13 plots the data for adenine capacity (y-axis) and 5-[(β-carboxyethyl)amino]acridine capacity (x-axis) for various resins. As indicated by the data in Table 6 and FIG. 13, the Dowex® XUS-43493 and Purolite® MN-200 resins had the highest 5-[(β-carboxyethyl)amino]acridine capacity; moreover, when both high 5-[(β-carboxyethyl)amino]acridine capacity and low adenine capacity were considered, the Amberlite® XAD-16 HP performed well.

The results from the *in vitro* experiments described in this example suggest that Dowex® XUS-43493 and the related resin Purolite® MN-200 are preferred resins for the removal of acridine compounds from PRBCs.

### EXAMPLE 7

### Removal Of Acridine Compounds From Packed Red Blood Cells With Styrene-divinylbenzene Adsorbents

This example is directed at the ability of a variety of different styrene-divinylbenzene (styrene-DVB) adsorbents to remove aminoacridine compounds from blood preparations. More specifically, the experiments of this example evaluate the removal of acridine orange and 9-amino acridine (depicted in FIG. 12) from a plasma/Adsol® solution.

### A. Experimental Procedures

For the experiments of this example, stock solutions (10 mM) of 5-[(β-carboxyethyl)amino]acridine (Cerus) and acridine orange (Aldrich) were prepared in distilled water, and a stock solution (10 mM) of 9-amino acridine (Aldrich) was prepared in ethanol. The 5-[(β-carboxyethyl)amino]acridine was added to a solution containing 25% plasma/75% saline to achieve a final concentration of 100 µM, while the acridine orange and the 9-amino acridine compounds were added to solutions containing 25% plasma/75% Adsol® (Baxter) Red Cell Preservation Solution to achieve a final acridine concentration of 100 µM.

The adsorbents utilized were Amberlite® XAD-16 HP (Rohm and Haas); Purolite® MN-200, and Dowex® XUS-43493 (Supelco). The water content of each adsorbent was determined by measuring the mass loss upon drying; the water content was corrected for so that the equivalent of 0.25 g dry of each adsorbent was used. Adsorbents were accurately weighed into 50 mL Falcon tubes. Thirty (30) mL of the 25% plasma/75% Adsol® solution containing 100 µM acridine was added to each tube containing adsorbent. The tubes were then placed on a rotator at room temperature, and 500 µL samples of solution were removed at various times and stored for later analysis.

Samples were analyzed by HPLC for levels of residual acridine. Each sample was diluted 2-fold with sample diluent (50% methanol, 25 mM KH₂PO₄, pH = 3.5), and proteins and other macromolecules were precipitated by incubating the samples at 4°C for 30 minutes. The samples were then centrifuged and the supernatant was filtered (0.2 µmeter) and analyzed on a C-18 reversed phase column (YMC ODS-AM 4.6 mm x 250 mm) by running a linear gradient from 75% solvent A (25 mM KH₂PO₄, pH = 3.5), 25% B (methanol) to 80% B in 20 minutes. Detection was by visible absorbance using a diode array detector set at 400 nm for 9-amino acridine, 490 nm for acridine orange, and 410 nm for 5-[(β-carboxyethyl)amino]acridine.

### B. Adsorption Kinetics

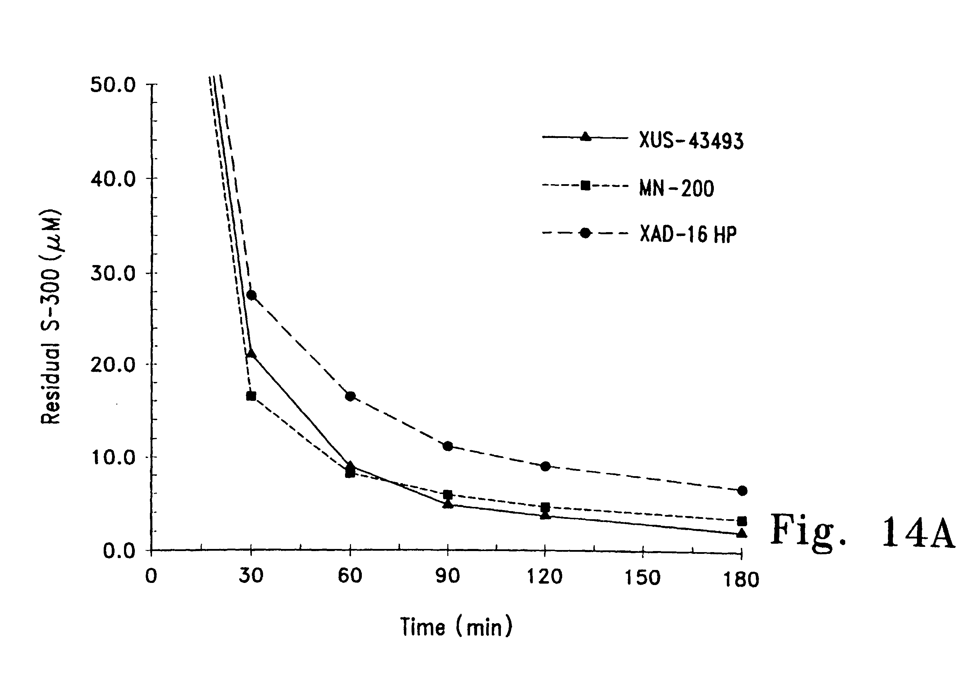

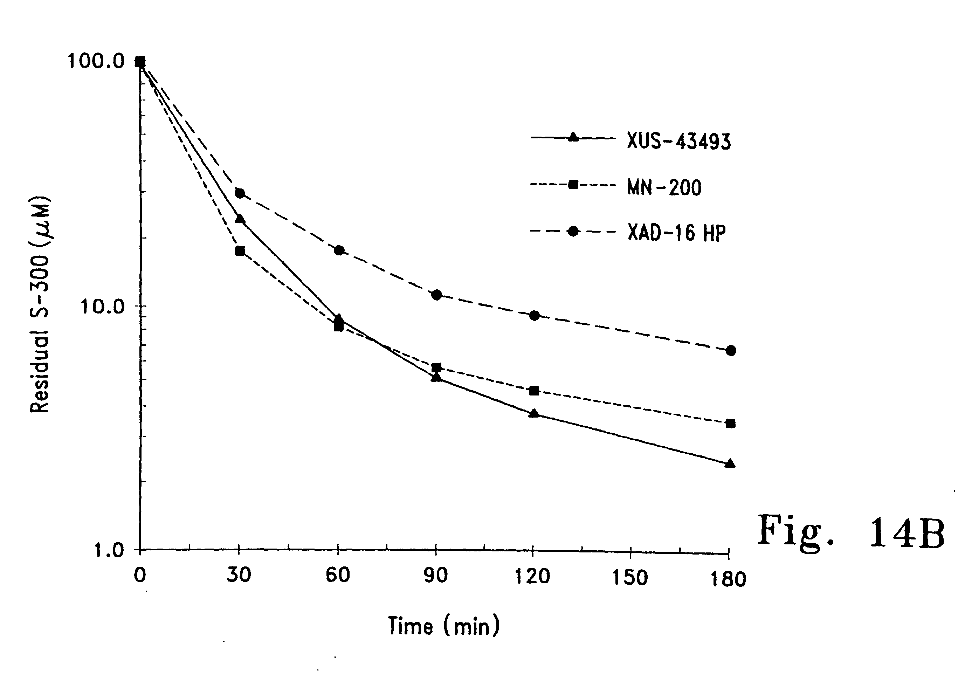

The kinetics of adsorption of 5-[(β-carboxyethyl)amino]acridine over a 3-hour period from 25% plasma/75% saline were compared for Dowex® XUS-43493, Purolite® MN-200, and Amberlite® XAD-16 HP. FIG. 14A and FIG. 14B both represent residual 5-[(β-carboxyethyl)amino]acridine as a function of time, FIG. 14B presenting the data on a logarithmic scale. In FIG. 14A and FIG. 14B, the XAD-16 HP data is represented by shaded circles connected by dashed lines, the MN-200 data is represented by shaded squares connected by dashed lines, and the XUS-43493 data is represented by shaded triangles connected by solid lines. As the data indicate, the XUS-43493 and MN-200 gave the fastest adsorption kinetics and were nearly equivalent. The XAD-16 HP appears to have a lower capacity for 5-[(β-carboxyethyl)amino]acridine.

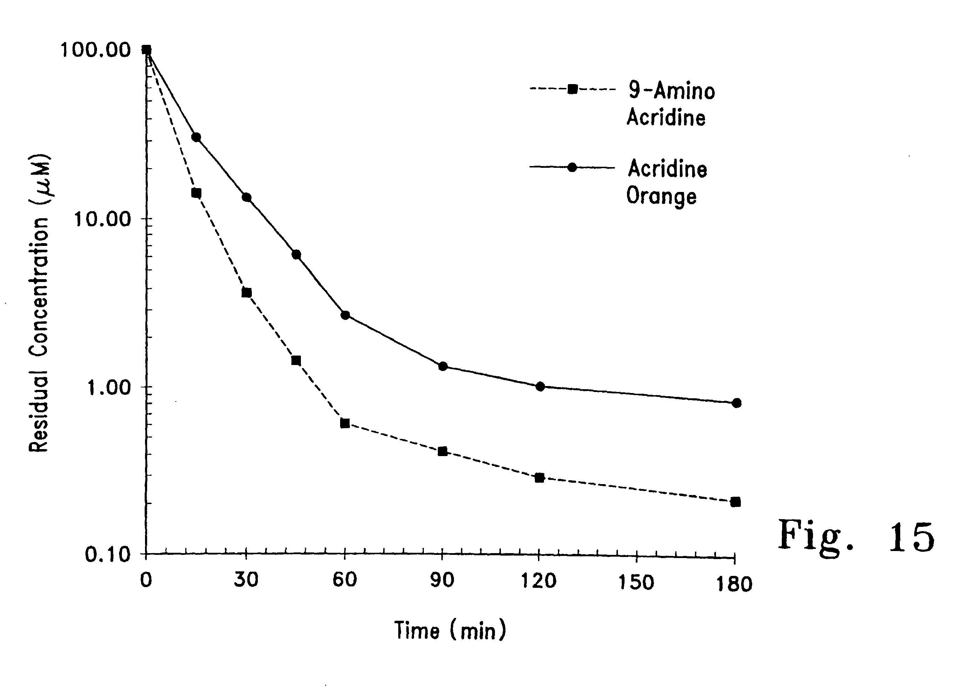

FIG. 15 compares the adsorption kinetics for removal of 9-amino acridine and acridine orange from 25% plasma/75% Adsol® with Dowex® XUS-43493. Referring to FIG. 15, the shaded squares with the dashed lines represent 9-amino acridine, and the shaded circles with the solid lines represent acridine orange. As the data indicate, levels of 9-amino acridine were undetectable beyond 3 hours. By comparison, the capacity of the Dowex® XUS-43493 for acridine orange was lower, which may be related to the presence of two tertiary amino groups on acridine orange.

The results of this study demonstrate the general ability of polystyrene-DVB adsorbents to remove acridines from blood products. It should be noted that interactions between the chemical agent (*e*.*g*., the acridine) and the cell-containing blood preparation will determine whether a batch adsorption or flow adsorption removal device will be most effective. Similarly, the hemocompatibility properties of individual adsorbents will influence the determination of which adsorbents are most effective.

It is to be understood that the invention is not to be limited to the exact details of operation or exact compounds, composition, methods, or procedures shown and described, as modifications and equivalents will be apparent to one skilled in the art. From the above, it should be clear that the methods and devices can be incorporated with apheresis systems and other devices and procedures currently used to process blood products for transfusion.

### EXAMPLE 8

This example compares the use of different types of powdered carbon as the active component in the media and demonstrates how careful choice of the active constituent is necessary to reduce the concentration of a small organic compound and preserve the fluid's biological function. The five activated carbons illustrated in Example 8 reduce the concentration of the psoralen, 4'-(4-amino-2-oxa)butyl-4,5',8-trimethyl psoralen, in plasma by approximately the same amount. Where "A Supra" is used as the adsorbent particle, however, there is substantially better retention of clotting factors relative to the other adsorbents.

4'-(4-amino-2-oxa)butyl-4,5',8-trimethyl psoralen was added to plasma for a concentration of 150 µM, and the plasma was illuminated in 325 mL batches with 3.0 J/cm2 UVA to inactivate pathogens. The residual 4'-(4-amino-2-oxa)butyl-4,5',8-trimethyl psoralen concentration was approximately 90 µM in the resulting plasma pool. 325 mL of illuminated plasma was pumped at 20 mL/min through 5 different types of 90 mm Cuno ZctaPlus carbon pads. Plasma clotting factor levels, clotting times, and 4'-(4-amino-2-oxa)butyl-4,5',8-trimethyl psoralen concentration were assayed before and after flow through the carbon pads.

| Cuno Grade | 4'-(4-amino-2-oxa)butyl-4,5',8-trimethyl psoralen | PTT | I | VIII | IX |
|---|---|---|---|---|---|
| | % Removed | Increase (s) | % Yield | % Yield | % Yield |
| R11S | 99.3 | 4.8 | 91 | 89 | 80 |
| R12S | 99.4 | 4.9 | 93 | 89 | 48 |
| R13S | 99.4 | 6.4 | 88 | 90 | 42 |
| R14S | 99.3 | 3.4 | 96 | 85 | 50 |
| A Supra | 99.4 | 1.6 | 96 | 81 | 82 |

| Activated Carbon Specifications | | | | |
|---|---|---|---|---|
| Activated Carbon | Activted Carbon | Ash Content | Activation | Special Treatment |
| R11S | Mineral | 14% | Steam | No |
| R12S | Lignite | not determined | Steam | No |
| R13S | Peat | 8% | Steam | Acid washed |
| R14S | Peat | 8% | Acid (H₂SO₄) | No |
| A Supra | Lignite | 3% | Steam | No |

The water flow rate for R10S media is 2 gallons water/min/square foot with a differential pressure of 1.5 p.s.i.

Notes: The A Supra grade was prepared to the same specifications as the R1xS series; only the carbon was changed. PTT stands for partial thromboplastin time and an increase indicates removal of clotting factors. I, VIII, and IX refer to particular clotting factors. All values are relative to post-photochemical treatment. 4'-(4-amino-2-oxa)butyl-4,5',8-trimethyl psoralen was assayed by HPLC and clotting factors and times were assayed by an automated coagulation analyzer.

### EXAMPLE 9

This example compares the use of two particle sizes and demonstrates the superiority of smaller particles in reducing the concentration of small organic compound and the tradeoff with the fluid's biological function. A particle size of less than 50 µm results in a significant increase in the percentage of psoralen removed from the plasma relative to the use of a particle size between 100 and 50 µm: 99.2% as opposed to 96.1%.

Photochemically treated plasma was similarly prepared as in Example 8. ZetaPlus-like filters were prepared containing two different particle sizes of ground Dowex Optipore L493 instead of powdered activated carbon according to the Cuno R1xS specifications. 325 mL photochemically treated plasma was pumped through each pad at 20 mL/min. 4'-(4-amino-2-oxa)butyl-4,5',8-trimethyl psoralen and clotting factors were analyzed as in Example 8.

| Particle Size | 4'-(4-amino-2-oxa)butyl-4,5',8-trimethyl psoralen | PIT | I | VIII | IX |
|---|---|---|---|---|---|
| (µm) | % Removed | Increase (s) | % Yield | % Yield | % Yield |
| 100 > > 50 | 96.1 | 0.9 | 97 | 94 | 86 |
| < 50 | 99.2 | 2.2 | 83 | 91 | 76 |

### EXAMPLE 10

This example compares the effect of changing the mass fraction of the active component on reducing the concentration of a small organic compound and the tradeoff with the fluid's biological function.

Photochemically treated plasma was similarly prepared as in Example 8. A Supra pads were prepared with the standard R1xS loading of approximately 60% Carbon and a lower loading level of 30%. Approximately 230 mL plasma was pumped through each 90 mm disc. 4'-(4-amino-2-oxa)butyl-4,5',8-trimethyl psoralen and clotting factors were analyzed as in Example 8.

| A Supra | 4'-(4-amino-2-oxa)butyl-4,5',8-trimethyl psoralen | PTT | I | VIII | IX |
|---|---|---|---|---|---|
| Loading (%) | % Removed | Increase (s) | % Retained | % Retained | % Retained |
| 61.5 | 99.4 | 1.3 | 93 | 93 | 77 |
| 30.8 | 99.4 | 0.9 | 99 | 90 | 91 |

### EXAMPLE 11

This example demonstrates that in some cases, treating the media with a hydrophilic polymer can have benefits to biological function.

Photochemically treated plasma was similarly prepared as in Example 8. One 90 mm R14S pad was flushed overnight with a 50 mg/mL solution of polyhydroxycthymethacrylate (pHEMA) in 95% ethanol dried in a 70°C oven until there was no additional weight change. The second pad was similarly flushed with 95% ethanol without pHEMA and dried. Approximately 325 mL plasma was pumped through the discs at 5 mL/min. 4'-(4-amino-2-oxa)butyl-4,5',8-trimethyl psoralen and clotting factors were analyzed as in Example 8.

| Coating | 4'-(4-amino-2-oxa)butyl-4,5',8-trimethyl psoralen | PTT | I |
|---|---|---|---|
| | % Removed | Increase (s) | % Yield |
| None | 99.6 | 4.1 | 98 |
| pHEMA | 99.1 | 2.5 | 103 |

### EXAMPLE 12

This example demonstrates that flowrate can have an effect on the reduction in concentration of the small organic compound.

Photochemically treated plasma was similarly prepared as in Example 8. A Supra pads were prepared with the standard R1xS loading of approximately 60% carbon and 325 mL of the treated plasma was pumped through each of the 47 mm diameter discs at three different flowrates. 4'-(4-amino-2-oxa)butyl-4,5',8-trimethyl psoralen and clotting factors were analyzed as in Example 8.

| Flowrate | 4'-(4-amino-2-oxa)butyl-4,5',8-trimethyl psoralen | PTT | I | VIII | IX |
|---|---|---|---|---|---|
| (mL/min) | % Removed | Increase (s) | % Yield | % Yield | % Yield |
| 10 | 99.0 | 1.3 | 98 | 83 | 80 |
| 15 | 98.8 | 1.5 | 99 | 85 | 86 |
| 20 | 98.7 | 1.4 | 97 | 89 | 86 |

### EXAMPLE 13

This example demonstrates how fluid volume can have an effect on both the reduction in concentration of the small organic compound and the biological activity of the fluid. The critical nature of the mode of contact between the biological fluid and the organic molecule reduction device is illustrated. Shown is the effect that fluid volume has on both the reduction in concentration of the small organic compound and the biological activity of the fluid. Where the plasma volume pumped through the device is increased (*e*.*g*. doubled) about the same concentration of small organic compounds, *e.g.* psoralen, is reduced from solution and there is a significant increase in the retention of clotting factors.

Photochemically treated plasma was similarly prepared as in Example 8. ZetaPlus-like filters were prepared with ground Dowex Optipore L493 with particles less than 50 µm instead of powdered activated carbon according to the Cuno R1xS specifications. Plasma was pumped through the filter at 20 mL/min and samples were taken at 180 mL and 325 mL. 4'-(4-amino-2-oxa)butyl-4,5',8-trimethyl psoralen and clotting factors were analyzed as in Example 8.

| Plasma | 4'-(4-amino-2-oxa)butyl-4,5'.8-trimethyl psoralen | PTT | I | VIII | IX |
|---|---|---|---|---|---|
| Volume (mL) | % Removed | Increase (s) | % Yield | % Yield | % Yield |
| 180 | 99.3 | 4.1 | 74 | 89 | 66 |
| 325 | 99.2 | 2.2 | 83 | 91 | 76 |

### EXAMPLE 14

This example describes the use of a sintered media.

Ninety millimeter diameter by 1/4" thick discs were fabricated by Porex. The discs contained various weight fractions of finely ground Dowex Optipore L493 with particle sizes between 20 µm and 100 µm, and small particles of ultra high molecular weight polyethylene (grade UF220), which were then sintered together. Photochemically treated plasma was similarly prepared as in Example 8 and 200 mL of plasma was pumped through each disc at 16-18 mL/min. 4'-(4-amino-2-oxa)butyl-4,5',8-trimethyl psoralen and clotting factors were analyzed as in Example 8.

| Weight | 4'-(4-amino-2-oxa)butyl-4,5',8-trimethyl psoralen | PTT | I | VIII | IX |
|---|---|---|---|---|---|
| Percent Adsorbent | % Removed | Increase (s) | % Retained | % Retained | % Retained |
| 25 | 97.8 | 0.5 | 93 | 95 | 78 |
| 35 | 99.0 | 1.2 | 90 | 94 | 74 |
| 50 | 99.3 | 1.2 | 91 | 80 | 76 |

### EXAMPLE 15

This example describes the effect of increasing the mass of adsorbent by increasing the diameter of the filter at constant thickness.

Photochemically treated plasma was similarly prepared as in Example 8. Approximately 325 mL of treated plasma was pumped through 90 and 47 mm diameter R03S grade discs at 5 mL/minute. 4'-(4-amino-2-oxa)butyl-4,5',8-trimethyl psoralen and clotting factors were analyzed as in Example 8.

| Disc | 4'-(4-amino-2-oxa)buryl-4,5',8-trimethyl psoralen | PTT | I |
|---|---|---|---|
| Diameter (mm) | % Removed | Increase (s) | % Retained |
| 47 | 99.4 | 2.1 | 96 |
| 90 | 99.6 | 3.5 | 77 |

### EXAMPLE 16

*Preparation of PRBCs Treated With a 5-[(β-carboxyethyl)amino]acridine derivative and Glutathione.* Pools of PRBC were prepared from fresh ABO-matched whole blood. The units of whole blood were centrifuged at 3800 rpm for 5 minutes using a Beckman Sorvall RC3B centrifuge. The plasma was expressed into another clean bag using an expresser. The units of PRBC were pooled into a 3.0 L size Clintec Viaflex bag if more than one unit was needed. For each unit of PRBC, 94 mL of Erythrosol was added. The percentage of Hematocrit (HCT) was measured by filling a capillary tubing with the blood sample and spinning it for 5 minutes. The hematocrit was determined to ensure that it did not decrease below 55%. For each 100 mL of PRBC, 3.3 mL of 12% glucose was added. The final percentage hematocrit was determined. PRBC (300 mL) was refilled into plastic containers PL 146 (Baxter Healthcare). To the blood bags was added 6.0 mL of 150 mM glutathione to reach a 3.0 mM final concentration and 3.0 mL of 30 mM a 5-[(β-carboxyethyl)amino]acridine derivative for a final concentration of 300 µM. The PRBC mixture was agitated for 1 minute using a wrist action shaker (manufacturer). The 5-[(β-carboxyethyl)amino]acridine derivative and glutathione treated PRBCs were allowed to incubate at room temperature overnight to allow break down of the 5-[(β-carboxyethyl)amino]acridine derivative into 5-[(β-carboxyethyl)amino]acridine.

### EXAMPLE 17

*HPLC Assay for 5-[(β-carboxyethyl)amino]acridine in PRBC and PRBC Supernatant.* The sample (100 µL) was diluted with 100 µL of saline and the resulting mixture was vortexed. To the solution was added 300 µL of 20 mM H₃PO₄ in CH3CN, and the mixture was vortexed for 15 sec. The sample was centrifuged at 13,200 rpm for 5 minutes. The supernatant (200 µL) was diluted into 800 µL of cold 0.1 M HCl and vortexed. The sample was filtered into an autosampler vial using a 0.2 µm Gelman Acrodisc filter. The HPLC conditions were as follows: (Manufacturer and part no. for column and general column.) Column = Zorbax SB-CN, 4.6 mm x 150 mm, 3.5 µm particles; guard column = (4.6 mm x. 12.5 mm, 5 µm particles (Mac Mod Analytical, Inc. (Chadds Ford, PA)); the mobile phase for A was 10 mM H₃PO₄ in HPLC water; the mobile phase for C was 10 mM H₃PO₄ in acetonitrile; temperature was 20 °C; sample volume was 100 µL; gradient conditions were as follows:

| Time | A (%) | C (%) | Flow Rate (mL/min) |
|---|---|---|---|
| 0.00 | 90.0 | 10.0 | 1.0 |
| 5.28 | 77.0 | 23.0 | 1.0 |
| 10.00 | 40.0 | 60.0 | 1.0 |
| 11.00 | 90.0 | 10.0 | 1.0 |
| 16.00 | STOP | STOP | STOP |

the DAD settings were as follows:

| Signal | Detection Wavelength | Detection Bandwidth | Reference Wavelength | Reference Bandwidth |
|---|---|---|---|---|
| A | 410 | 5 | 580 | 20 |
| B | 260 | 5 | 580 | 20 |

### EXAMPLE 18

*HPLC Assay for Glutathione in PRBC and PRBC Supernatant.* The sample was prepared as for the HPLC assay described above. The HPLC conditions were as follows: analytical column = YMC ODS-AM-303, 250 mm x 4.6 mm, 5 µm particle; guard column = Brownlee, 15 x 3.2 mm, 7 µm particle; the mobile phase for A was 10 mM H₃PO₄ in HPLC water; the mobile phase for C was 10 mM H₃PO₄ in acetonitrile; the temperature was 15°C; the sample volume was 75 µL; the gradient conditions were as follows:

| Time | A (%) | C (%) | Flow Rate (mL / min) |
|---|---|---|---|
| 0.00 | 95.0 | 5.0 | 0.5 |
| 6.00 | 95.0 | 5.0 | 0.5 |
| 8.00 | 10.0 | 90.0 | 1.0 |
| 9.00 | 95.0 | 5.0 | 1.0 |
| 20.00 | STOP | STOP | STOP |

the DAD settings were as follows:

| Signal | Detection Wavelength | Detection Bandwidth | Reference Wavelength | Reference Bandwidth |
|---|---|---|---|---|
| A | 205 | 10 | 600 | 100 |

### EXAMPLE 19

*Method of Screening Adsorbents.* A test solution containing 25% plasma and 75% Erythrosol was used to represent the supernatant from PRBCs. Erythrosol was prepared as two separate stock solution parts (solution C and solution D) that were sterilized separately. The final solutions were prepared by mixing equal volumes of solution C and solution D.

| Solution C | Solution D |
|---|---|
| 3.2 mM Adenine | 53.2 mM Na citrate 2 H₂O |
| 85 mM mannitol | 5.4 mM NaH₂PO₄ 2 H₂O |
| 100 mM glucose | 38 mM Na₂HPO₄ 2 H₂O |
| 6.2 mM HCl | |

The plasma-Erythrosol solution was spiked with 5-[(β-carboxyethyl)amino]acridine to a final concentration of 300 µM. Glutathione (reduced form, Sigma Chemical Co.) was added to the mixture to obtain a final concentration of 3 mM. Samples of adsorbents (0.2-0.8 g) were accurately weighed (± 0.001 g) into tared 7 mL polypropylene vials with screw tops. Samples of adsorbent that required pre-wetting were suspended in 70% ethanol. The adsorbent was allowed to settle and the supernatant was removed. Residual ethanol was removed by resuspending the adsorbent in distilled water, allowing the adsorbent to settle, and decanting the supernatant. Adsorbent masses were corrected for water content when adsorption capacities were calculated. A 5.0 mL aliquot of plasma/Erythrosol was added to each tube. The tubes were placed on a rotator and tumbled at room temperature for 24 hours. The vials were removed from the rotator and the adsorbent was allowed to settle. A sample of the supernatant was removed from each tube. Samples were centrifuged to remove residual adsorbent. Samples were analyzed by HPLC to determine residual levels of 5-[(β-carboxyethyl)-amino]acridine. The reversed phase assay described above was used to determine residual levels of glutathione. The results from the adsorbent screen are shown in Tables 7 and 8.

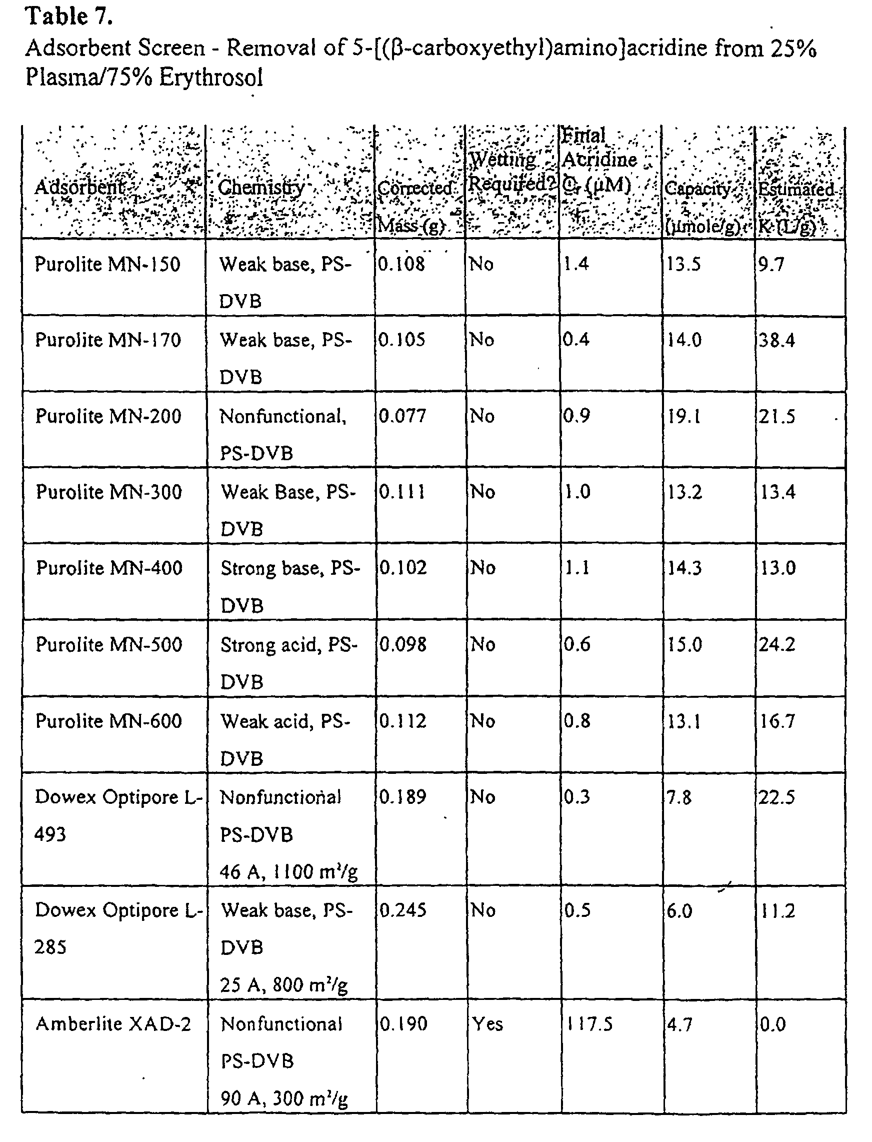

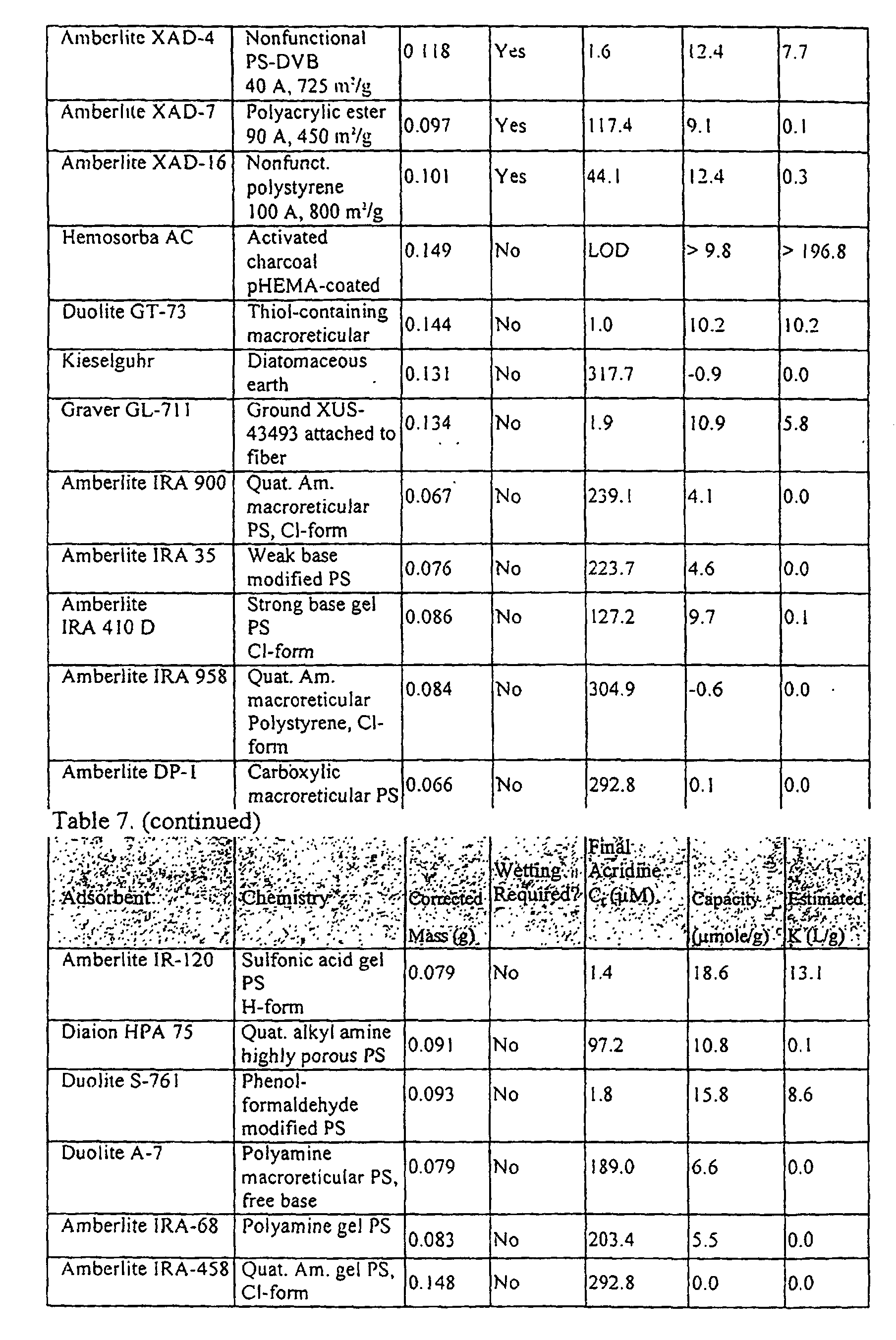

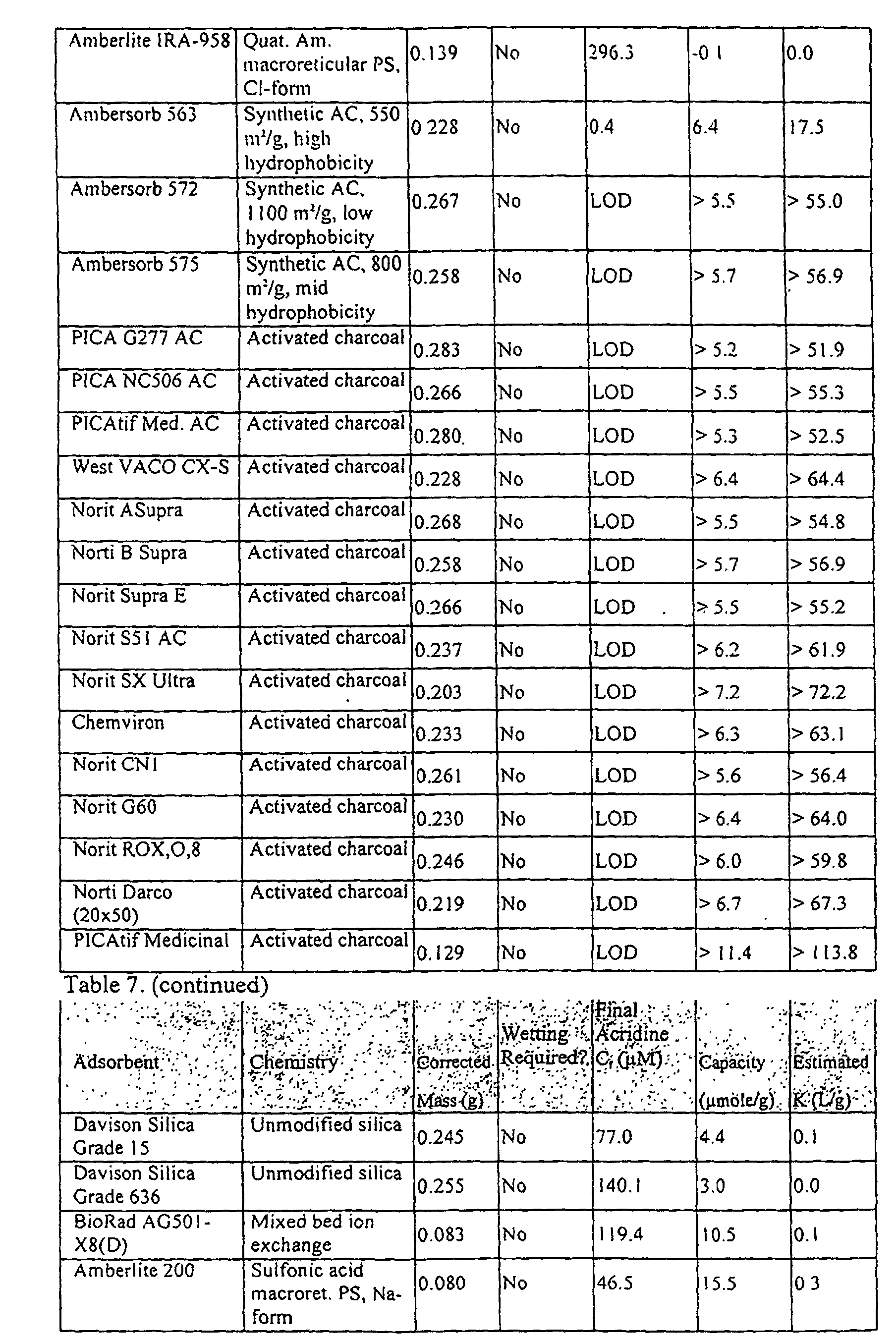

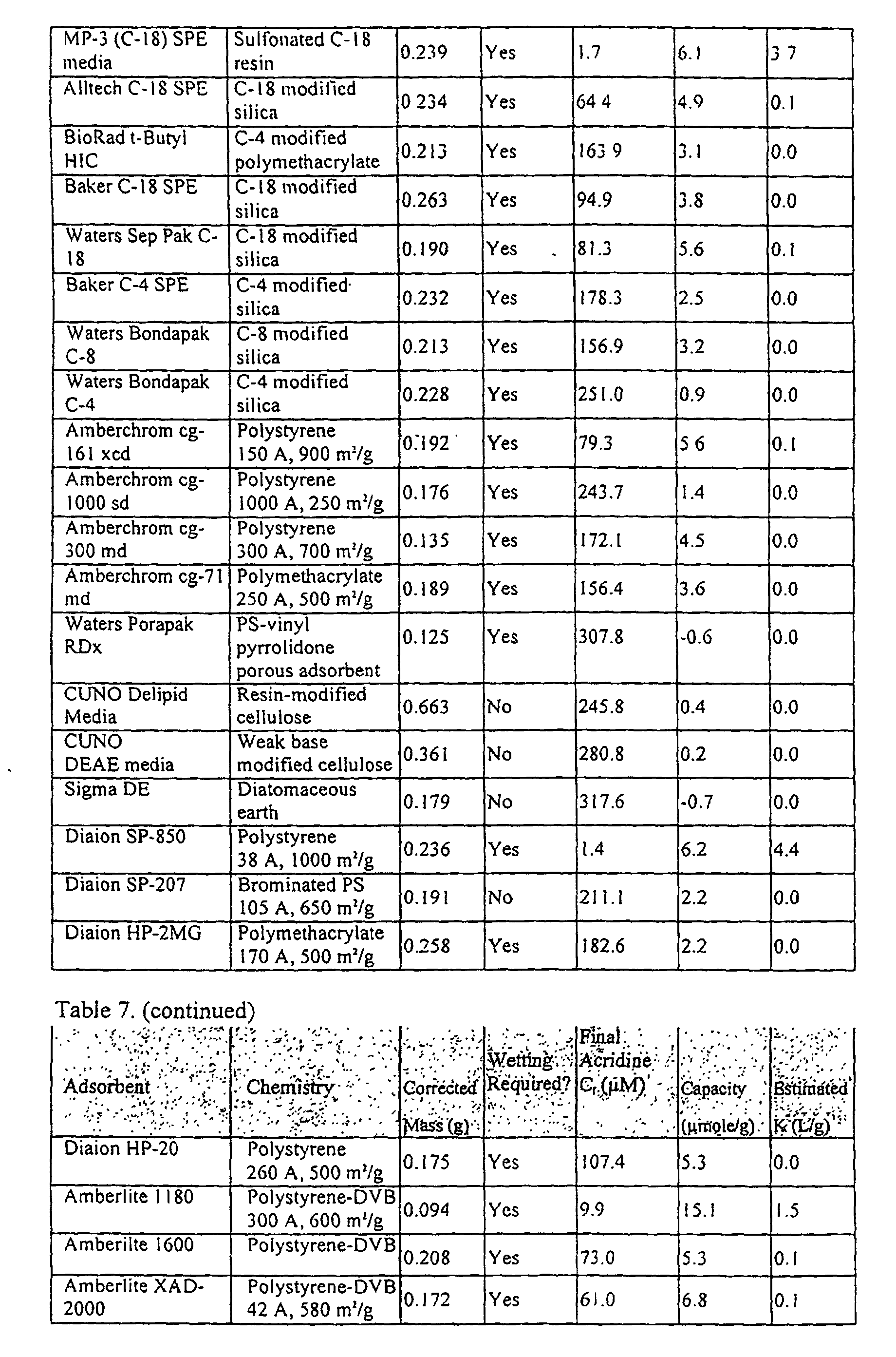

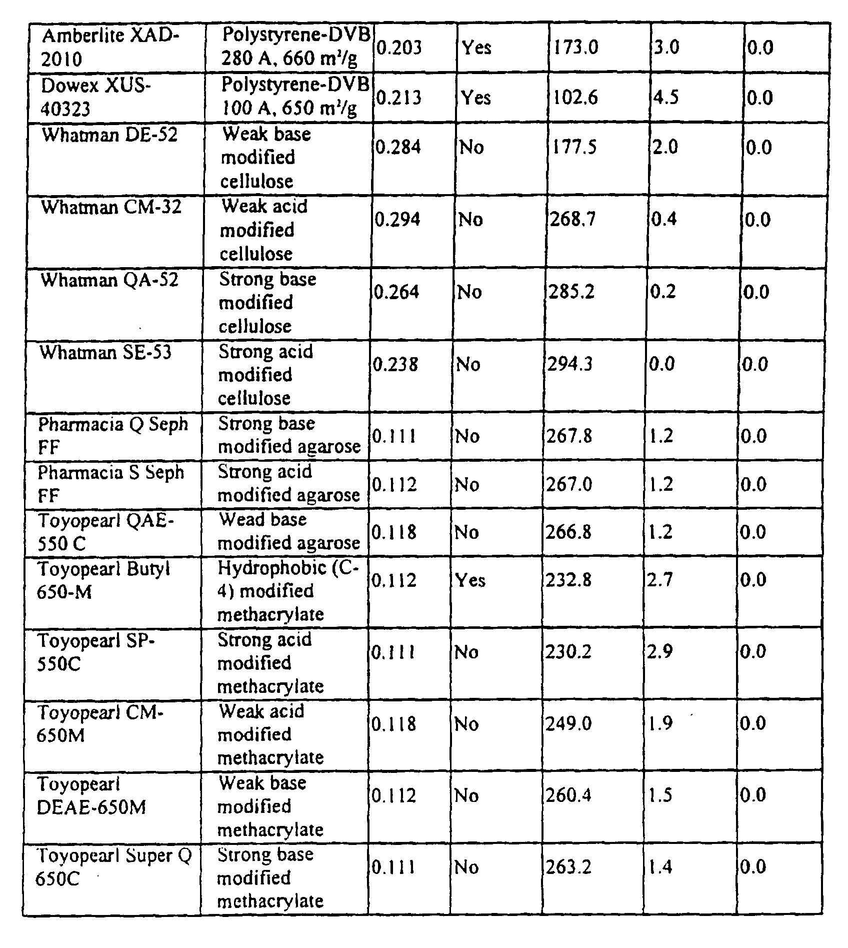

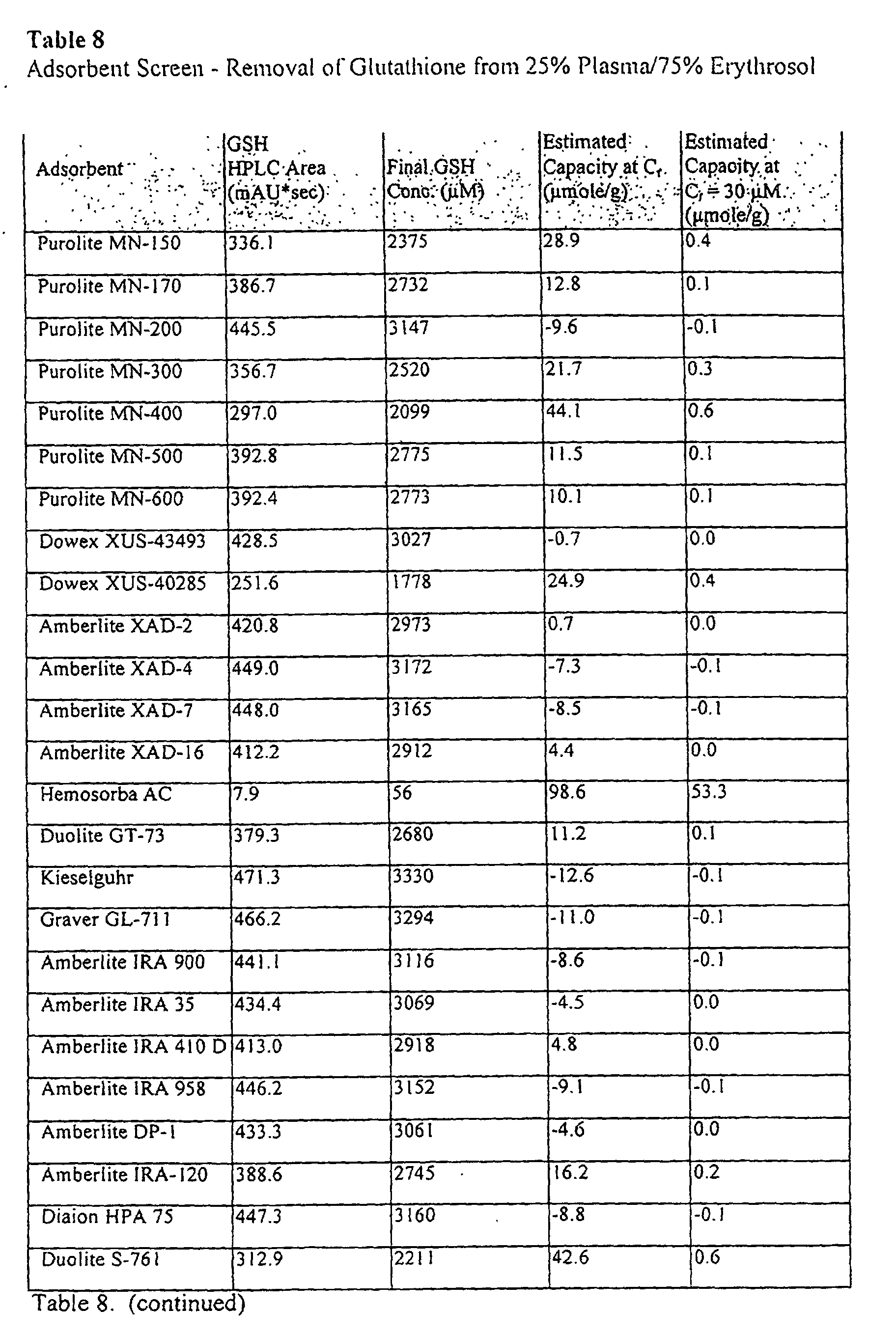

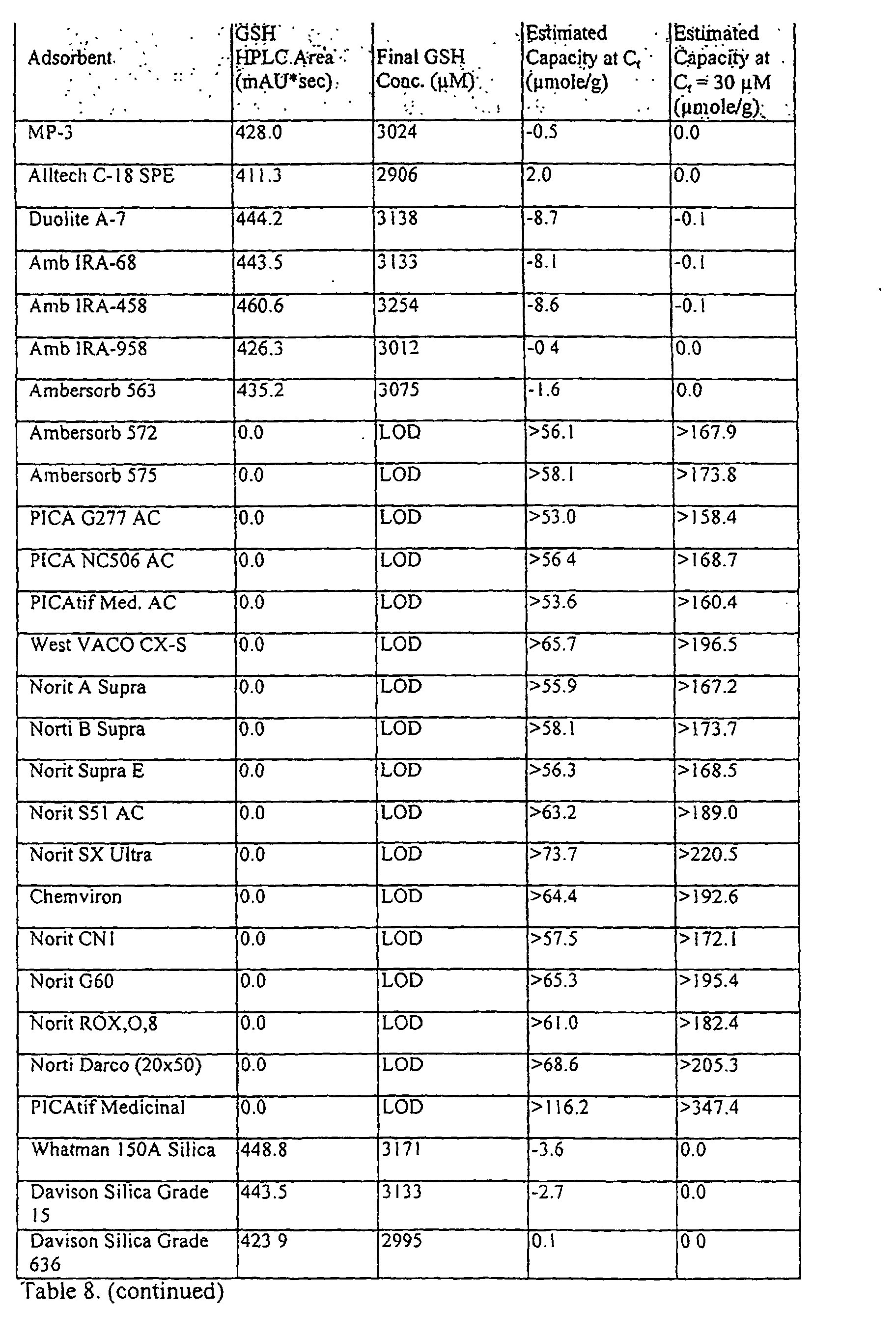

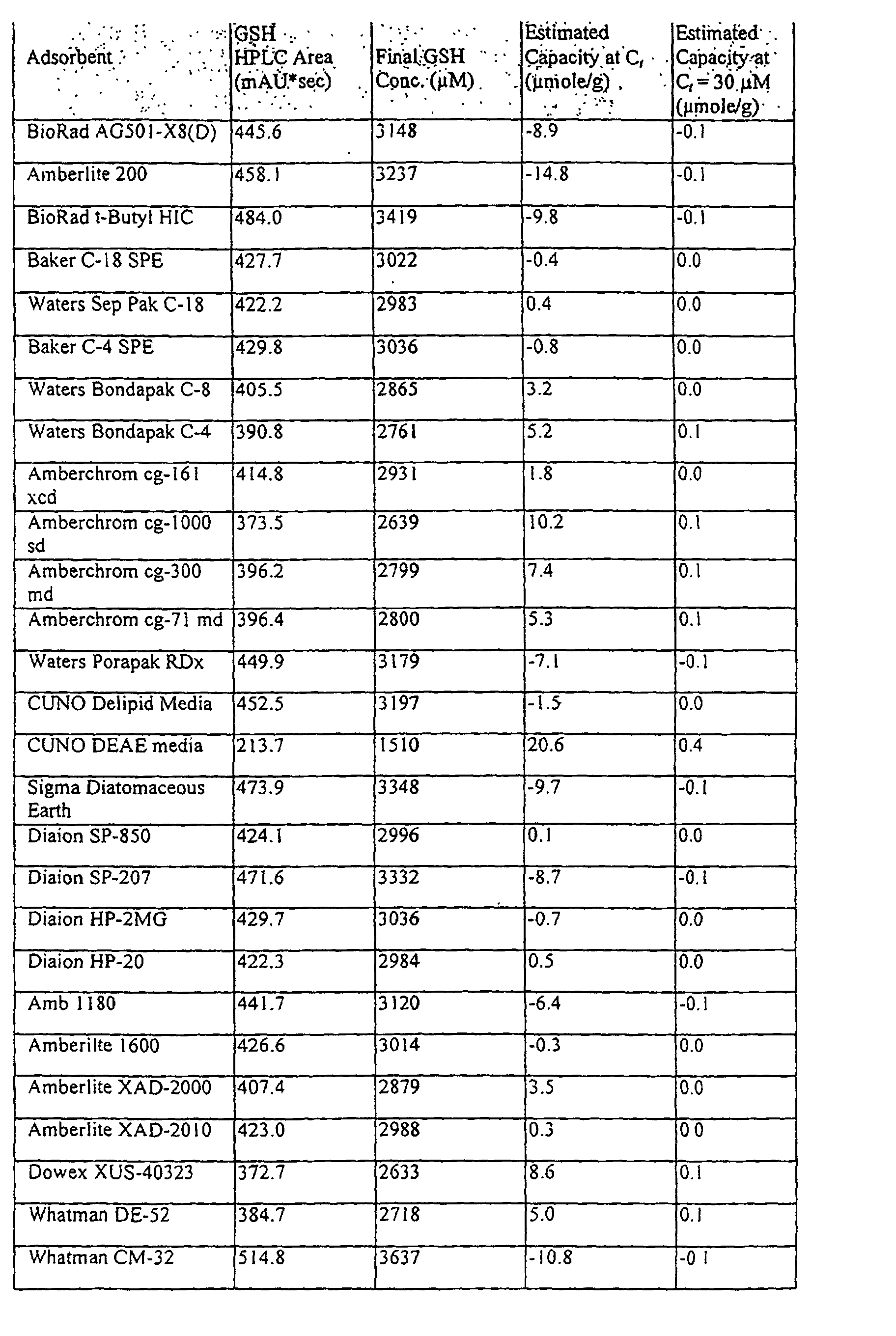

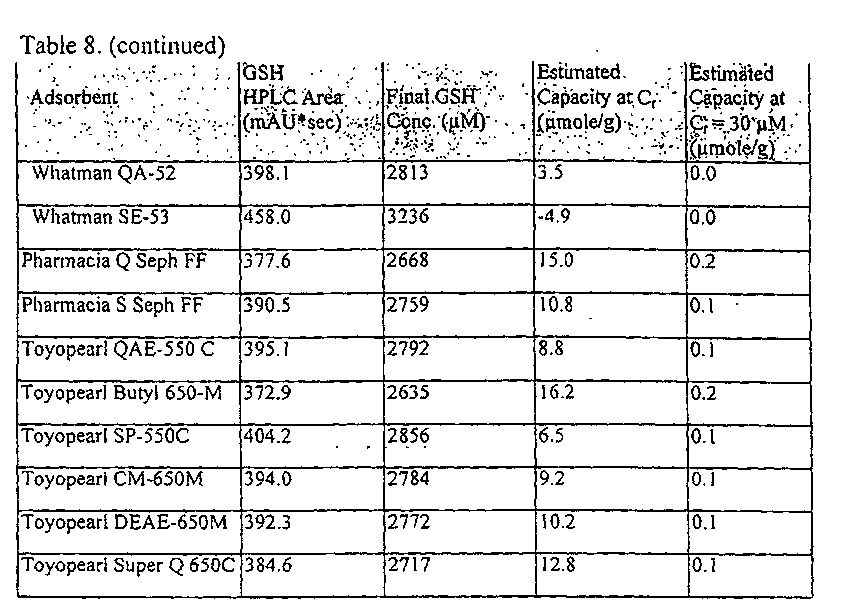

### EXAMPLE 20

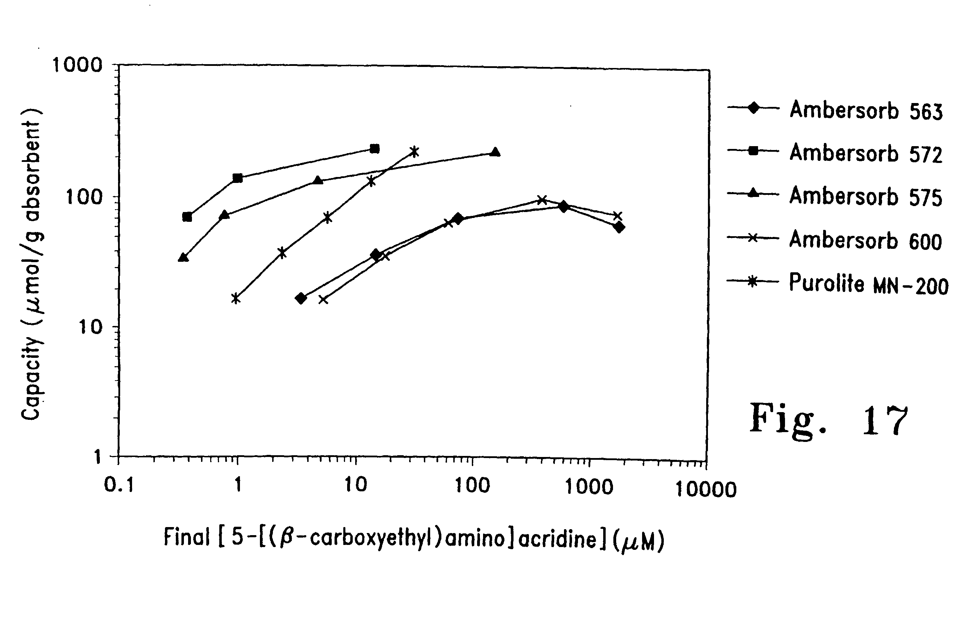

*Adsorption Capacities for Adsorbents.* Adsorption isotherm experiments were carried out to determine the adsorptive capacities (µmole 5-[(β-carboxyethyl)-amino]acridine/g adsorbent) for various types of adsorbents. Figure 17 shows adsorption isotherms obtained for several Ambersorbs as compared to the adsorption isotherm for Purolite MN-200. Adsorption studies were performed in 25% plasma/75% Erythrosol solutions containing 0.2-3 mM 5-[(β-carboxyethyl)amino]acridine and 0.6-10 mM GSH. Samples were agitated for 24 hours at room temperature.Calculations using the adsorption capacity from Table 7 (22 µmole/g) determined that approximately 4 g of Purolite MN-200 would be required to reduce the level of 5-[(β-carboxyethyl)-amino]acridine in a 300 mL unit of PRBC from 300 µM to a final level of 1 µM. Less than 1 g of Ambersorb 572 (130 µmole/g) would be required to achieve comparable removal. A similar calculation estimated that less than 1 g of Ambersorb 572 would be required to reduce the level of GSH in a 300 mL unit of PRBC from 6 mM to a final level of 500 µM in the 150 mL of supernatant (50% HCT).

### EXAMPLE 21

### Flow Studies Using Several Forms of Activated Carbon Media.

Experiments were carried out to investigate removal of 5-[(β-carboxyethyl)-amino]acridine and GSH from PRBCs using a flow device. PRBCs (Erythrosol, glucose, 63% HCT) were dosed with 300 µM a degradable 5-[(β-carboxyethyl)amino]acridine derivative and 3 mM GSH, and held at room temperature without agitation for 20 hours prior to flow through devices. The flow compound adsorption device media consisted of various forms of activated carbon, as either a composite carbon/cellulose disk, or a carbon fiber felt. The media were sealed into a 90 mm diameter polycarbonate housing (Cuno, Meridien, CT). The dosed PRBCs were pumped (Gilson Minipuls, Middleton, WI) through the media at a flow rate of 5 mL/min, and collected in a PL146 plastic container (Baxter Healthcare). The results of this study are shown in Table 9

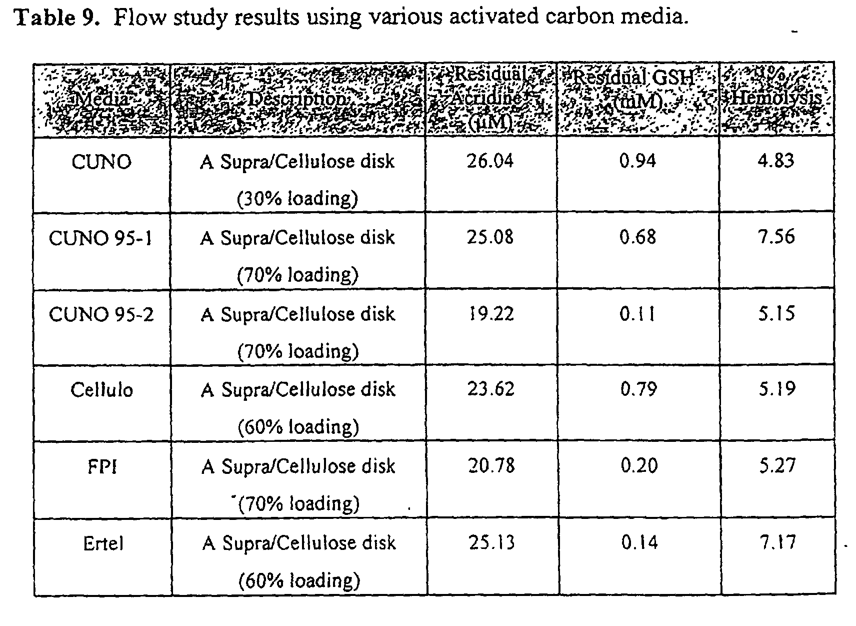

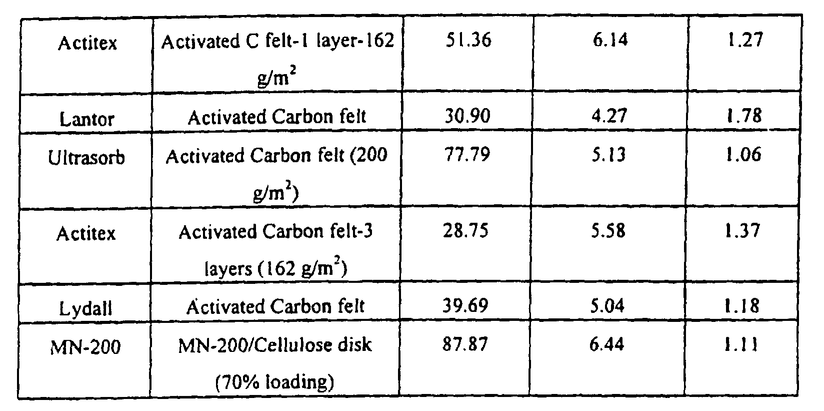

### EXAMPLE 22

*Flow Compound Adsorption Devices (CUNO media) vs. Batch Compound Adsorption Devices (AQF media).* Studies were performed which compared 5-[(β-carboxyethyl)amino]acridine and GSH removal in flow versus batch compound adsorption devices. The flow device consisted of a cellulose/Norit A Supra (Norit Americas, Inc. (Atlanta, GA)) carbon disk enclosed in a 90 mm housing. There were two separate batch devices: one consisted of fiberized Pica G277 activated carbon (AQF 500 g/m²); the other consisted of fiberized Purolite MN-200 (AQF 312 g/m²). Following dosing with 300 µM of a degradable 5-[(β-carboxyethyl)-amino]acridine derivative and 3 mM GSH, PRBCs were pumped through the cellulose/carbon media at a flow rate of 2 mL/Min, after which 5-[(β-carboxyethyl)-amino]acridine and GSH levels dropped 75 and 88%, respectively, to 24 µM and 0.71 mM in the PRBC supernatant. The flow device exposed PRBCs were then transferred to the 6 g MN-200 batch device, which decreased 5-[(β-carboxyethyl)-amino]acridine and GSH levels by an additional 5 and 1%, reaching 20 µm and 0.67 mM over 24 hours. Exposure of PRBCs to a 7 g Pica G277 batch device alone resulted in a drop in 5-[(β-carboxyethyl)-amino]acridine and GSH levels by 92 and 54% to concentrations of 8 µM and 3 mM. Therefore, one pass through the flow device was more effective in removing GSH than exposure to the carbon batch device for 24 hours. The batch device alone, however, was more effective in removing 5-[(β-carboxyethyl)-amino]acridine than the flow and MN-200 devices combined. Flow through the device did not seem to have an adverse effect on PRBC ATP concentration. K+ levels in PRBCs were lower after exposure to the flow device as compared to a 24 hour carbon batch device exposure.

### EXAMPLE 23

### Long Term Removal of Breakdown Products.

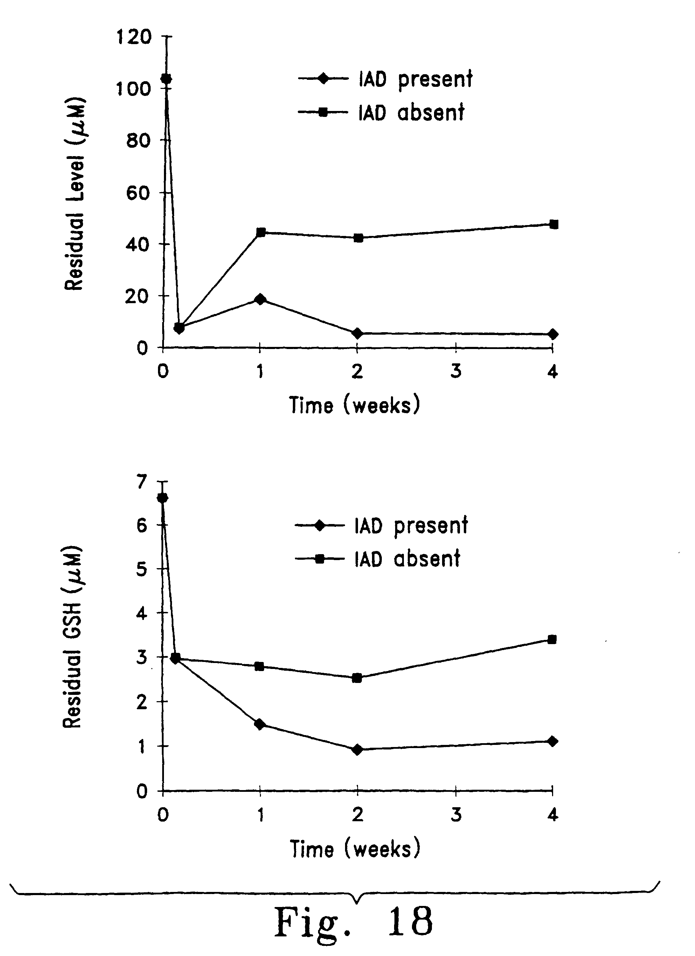

This experiment examined 5-[(β-carboxyethyl)-amino]acridine and GSH levels in PRBCs from which a fiberized PICA G-277 activated carbon device (AQF, 7.3g, 500 m²/g was removed after 24 hours of exposure. This study was conducted in parallel with studies where the PRBCs had continued device exposure. Figure 18 shows that the concentrations of 5-[(β-carboxyethyl)-amino]acridine and GSH in the supernatant samples were considerably higher in the absence of a device over storage times of 1 to 4 weeks.

The concentration of 5-[(β-carboxyethyl)-amino]acridine was reduced to 5 µM in initially shaken PRBCs after 35 days of storage in the presence of a removal device (MN-200). This indicates that 5-[(β-carboxyethyl)-amino]acridine removal does occur in static storage conditions at 4 °C.

### EXAMPLE 24

### Effect of Enclosure Material (membrane, woven, non-woven) on an IAD

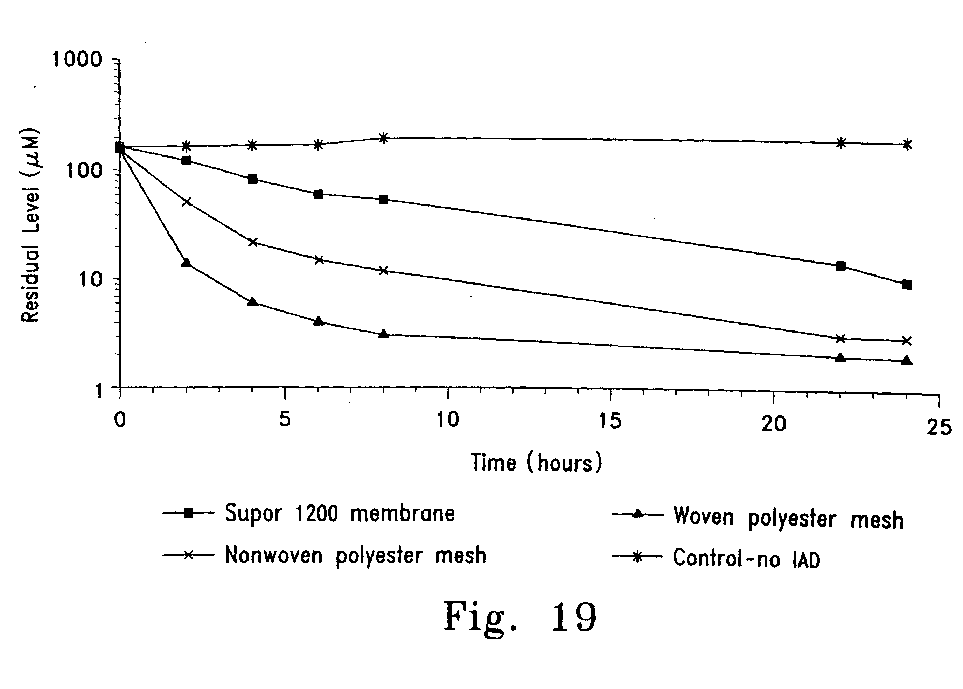

The use of an enclosure material surrounding the adsorbent media was investigated for the primary purpose of particle retention. The primary purpose is particle retention. However, membranes can enhance hemocompatibility of the devices by preventing contact between the RBCs and membranes. Membranes can easily be modified with hydrophilic polymers (PEO, PEG, HPL) to enhance hemocompatibility without altering function. Approximately 10 g of fiberized Pica G277 activated carbon media (AQF 500 g/m²) was surrounded by a heat-sealed membrane, woven polyester, or non-woven polyester material. PRBC units (300 mL) were dosed with 300 µM of a degradable 5-[(β-carboxyethyl)amino]acridine derivative and 3 mM GSH, held at room temperature for 20 hours on a platelet shaker, and then transferred to IADs. Concentration of 5-[(β-carboxyethyl)-amino]acridine was monitored over 24 hours. Figure 19 shows 5-[(β-carboxyethyl)-amino]acridine levels in the supernatant of 300 mL PRBC units exposed to [ADs consisting of fiberized Pica G277 activated carbon (500 g/m²) and enclosed by a membrane, woven, or non-woven material. PRBCs (Erythrosol, glucose, 62% HCT) were dosed with 300 µM of a degradable 5-[(β-carboxyethyl)amino]acridine derivative and 3 mM GSH, and agitated on a platelet shaker at room temperature prior to transfer to the IADs. PRBC-containing IADs were agitated at room temperature for 24 hours.

These studies indicate that the Tetko woven enclosure shows the fastest removal kinetics for 5-[(β-carboxyethyl)-amino]acridine over 24 hours. Final levels achieved for all enclosure materials after 2 weeks were similar, with 5-[(β-carboxyethyl)-amino]acridine concentrations decreasing to approximately 2 µM after 1 day, but rising back to 10 µM near day 8.

### EXAMPLE 25

### Effect of the Compound Adsorption Device on Red Blood Cell Function.

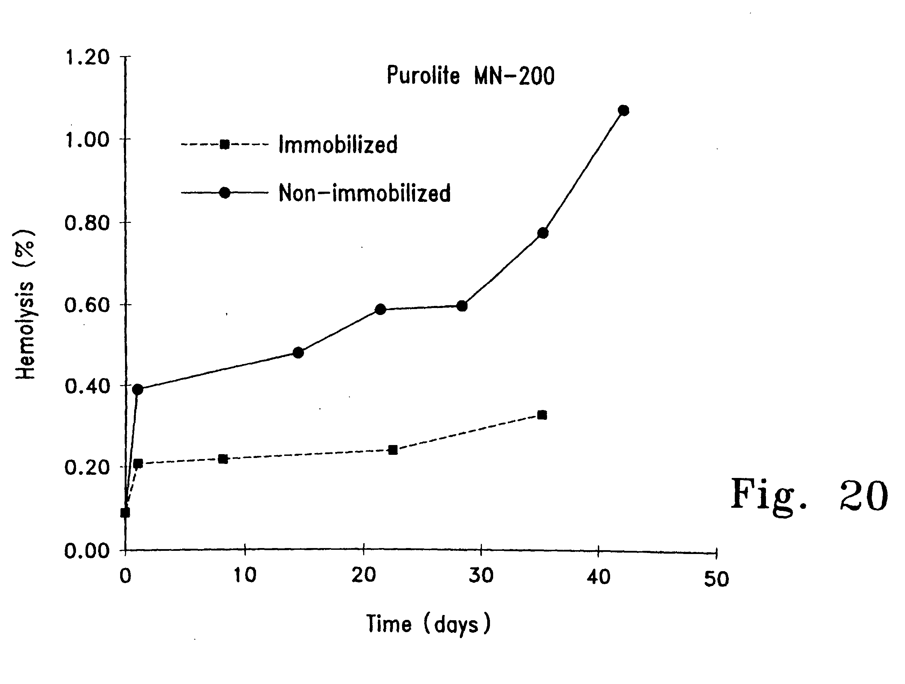

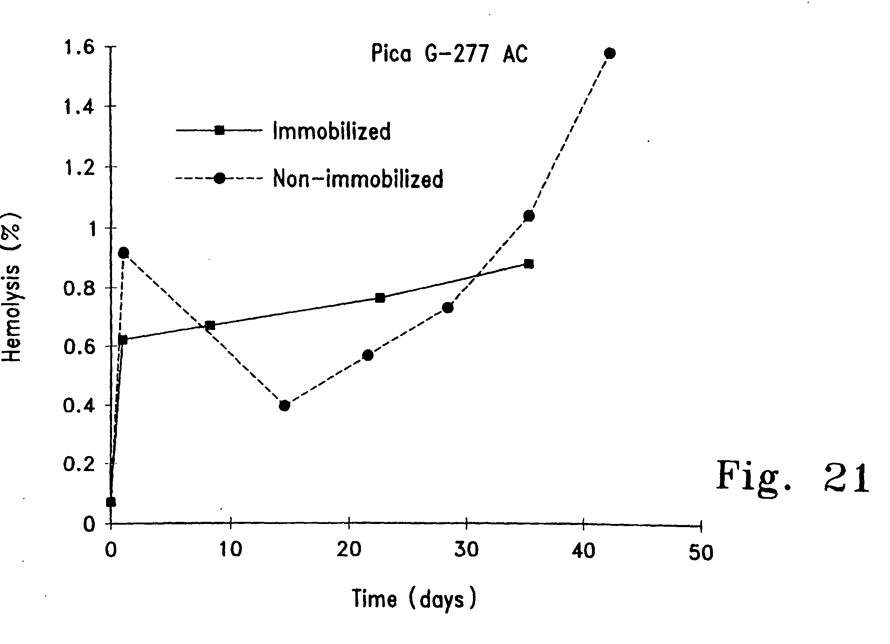

Indicators of red blood cell function were monitored over the course of 5-[(β-carboxyethyl)-amino]acridine and GSH removal experiments for various device configurations. Parameters measured included percentage lysis, ATP and K⁺ concentration. Table 10 shows that ATP concentrations were generally not affected by the presence of a compound removal device: The decrease in ATP concentrations was approximately the same for Control (no IAD) as for the MN-200 or Pica G277 devices. Levels of K⁺ in PRBCs were found to increase with time. The temperature at which removal occurred did not influence K⁺ levels in PRBC units exposed to compound removal devices over 20 days. Where the time period of exposure was extended to 35 days, however, the rate of increase of K⁺ in PRBCs varied with the type of adsorbent, with final levels achieved of 40 and 45 mmol/Lfor MN-200 and PICA G-277 devices, respectively, compared to the no device control at 39 mmol/L. The percentage of red blood cells lysed in device-exposed and no-device control PRBC units has generally been found to be between 0.1 and 1% after 24 hours. As shown in Table 11 and Table 12 and graphed in Figures 20 and 21, the % lysis varied significantly with the type of adsorbent used. Table 11 shows lysis values obtained for PRBCs exposed to two types of immobilized adsorption devices over 35 days. Table 12 shows shows lysis values obtained for PRBCs exposed to loose adsorbent particles enclosed in a woven polyester mesh over 42 days. A wrist action shaker was used in dosing all PRBC units for 1 minute, after which the PRBCs wre in a static condition for 4 hours at room temperature. The devices were held at room temperature for 24 hours on a platelet shaker, after which they were in a static condition at 4°C for the duration of the study. The immobilization MN-200 showed lower hemolysis levels than immobilized PICA G-277, while the Ambersorb synthetic carbonaceous adsorbent showed one of the lowest hemolysis levels upon comparison of the loose particle adsordents. The MN-200 IAD showed lower hemolysis than the same non-immobilized adsorbent. Similar observations have been observed for other IADs as compared to the same non-immobilized adsorbent.

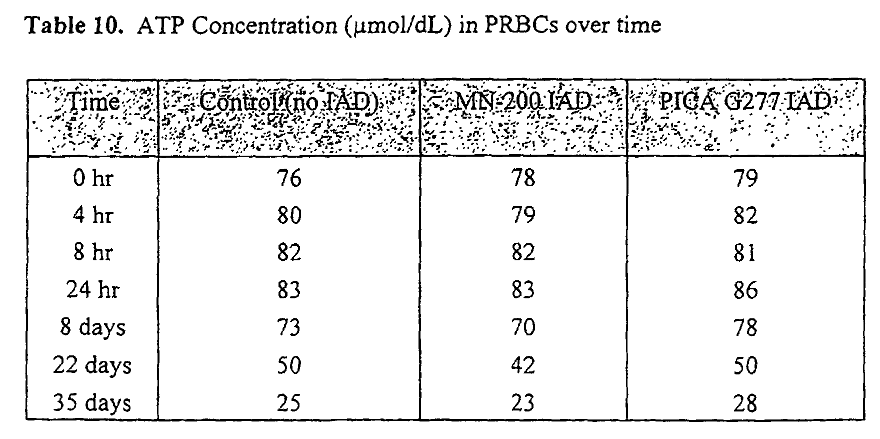

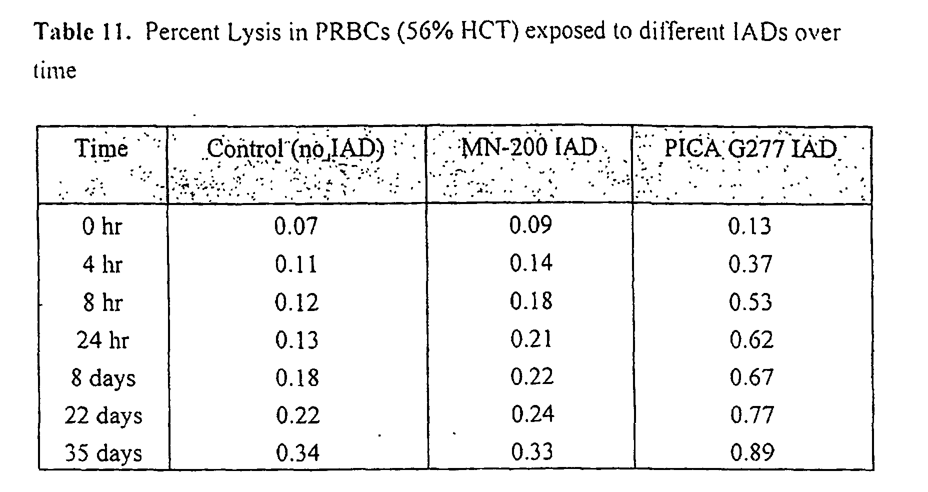

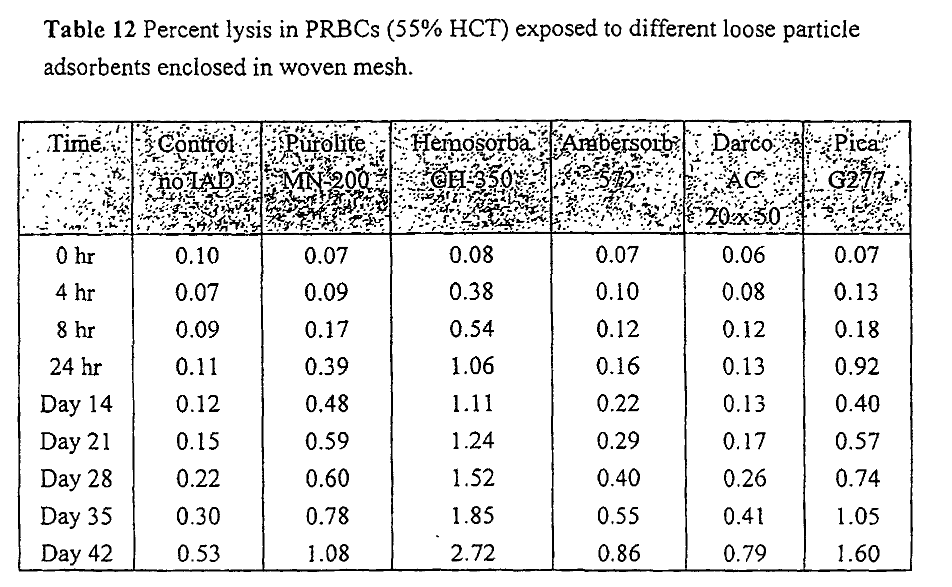

The critical nature of the adsorbent particle with respect to the maintenance of red blood cell function is illustrated. A comparative study of the effects of five different adsorbents on red blood cell hemolysis is presented. Ambersorb 572 produced only 0.16% lysis in PRBCs (55%) after a 24 hour exposure, while Darco AC (Norit Americas, Inc. (Atlanta, GA) produced 0.13% lysis. Those adsorbents were significantly better at minimizing hemolysis of red blood cells than the Purolite MN-200, Hemosorba CH-350 and Pica G277 adsorbents.

Table 13 shows a comparative study where supernatants from red blood cell samples containing glutathione and 5-[(β-carboxyethyl)amino]acridine were contacted with a number of different adsorbents. Activated carbon adsorbents were the only type of adsorbent that was capable of substantially reducing the concentrations of both 5-[(β-carboxyethyl)-amino]acridine and glutathione. Both natural activated carbons (Norit and PICA) and synthetic activated carbons (Ambersorb) proved to be effective at compound reduction.

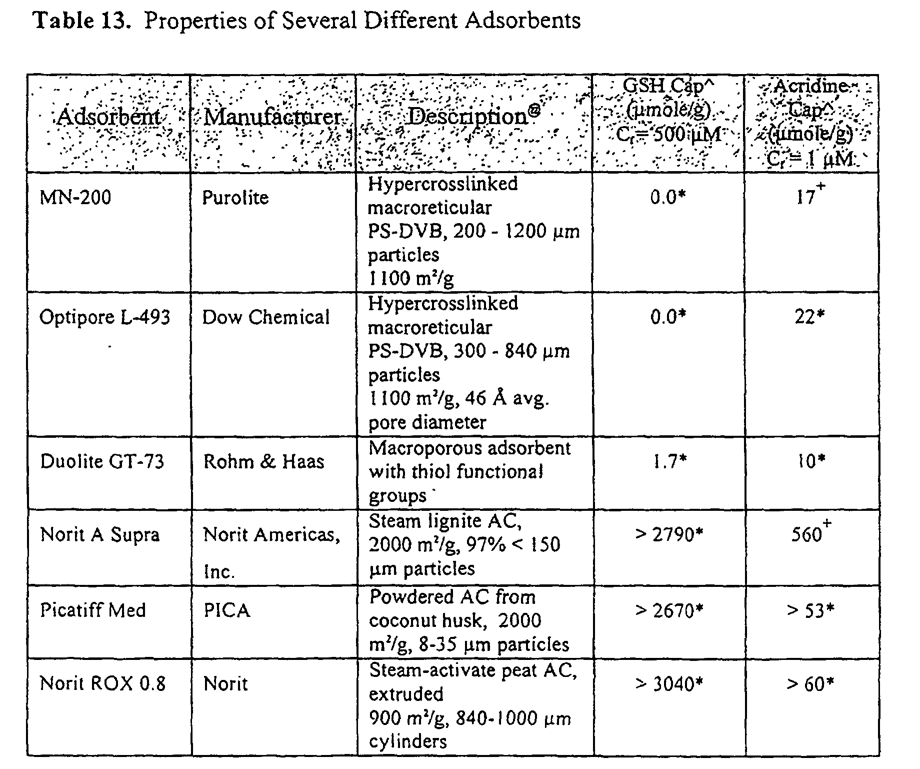

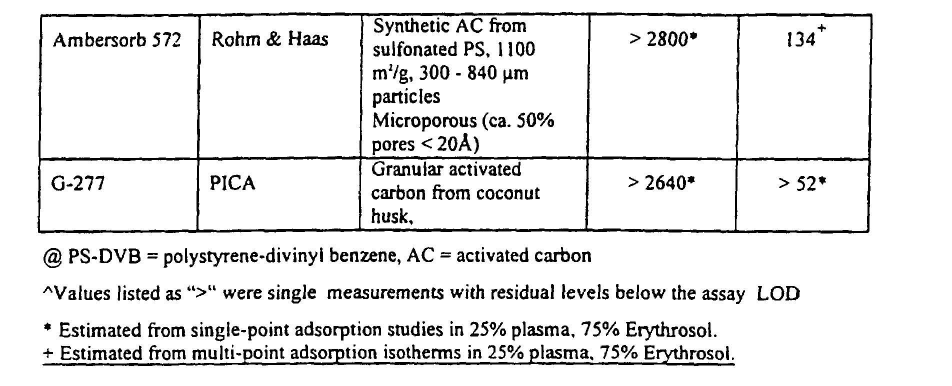

### EXAMPLE 26

This example describes the typical performance of an immobilized adsorbent device in a flow mode on plasma using adsorbent media composed of 30% Norit A Supra carbon (Norit Americas, Atlanta, GA) and manufactured by Cuno (Meriden, CT) previously described in an earlier example.

IADs were assembled using 90 mm Cuno 30% Norit A Supra impregnated R10SP media in series with 90 mm diameter Memtec hydroxypropylcellulose coated polysulfone membrane with 5 µm pores.

To complete disposable manufacture, a 1 LPL2410 bag was docked to 1/8" OD tubing and the tubing was then attached to the outlet of the IAD such that the distance from the midline of the IAD to the bag was 40cm. The same sized tubing was attached to the inlet of the SRD such that when the illumination bag is docked to it, the distance from the midline to the bag would be 30 cm.

5-[(β-carboxyethyl)-amino]acridine was added to 500 mL of plasma to a final concentration of approximately 150 µM, and the plasma was illuminated to 6.3 J/cm2 UVA to inactivate pathogens. The post-illumination 5-[(β-carboxyethyl)-amino]acridine concentration was approximately 82 µM.

Table 14 shows measures of clotting factor activity and 5-[(β-carboxyethyl)-amino]acridine levels after having been treated by the IAD relative to values beforebeing passed through the IAD. The experiment was repeated three times. Means and standard deviations are reported. Treatment time averaged 14 minutes. It is apparent that there is virtually no change in factors I, II, V, VII, X or measures of aggregate clotting activity, the prothrombin time (PT) and activated partial thromboplastin time (aPTT) and very small changes in factors XI and XII. Factors VIII and IX show somewhat larger changes but these are acceptable, especially in light of the prescription of recombinant proteins for their deficiency. The very selective nature of the device should be noted in that it retains virtually all the clotting activity while allowing on 0.9% of the 5-[(β-carboxyethyl)-amino]acridine to pass through.

**Table 14**

| | |
|---|---|
| PT(s) | 0.0 ± 0.1 |
| aPTT(s) | 0.7 ± 0.1 |
| I (%) | 98 ± 2 |
| II (%) | 99 ± 1 |
| V(%) | 101 ± 2 |
| VII(%) | 99 ± 2 |
| VIII (%) | 86 ± 1 |
| IX(%) | 82 ± 3 |
| X (%) | 103 ± 4 |
| XI (%) | 94 ± 4 |
| XII(%) | 95 ± 5 |
| S-59(%) | 0.9 ± 0.1 |

### EXAMPLE 27

Fiberized media consisting of Dowex Optipore L-493 attached to a nonwoven polyester fiber matrix (Hoechst-L493) has been manufactured by the AQF division of Hoechst Celanese (Charlotte, NC). The performance of this adsorbent media in a batch removal device for platelets was evaluated.

### Platelet Preparation

Single donor apheresis platelet units containing 3.5 - 4.5 x 10¹¹ platelets in 300 mL of 35% autologous plasma, 65% PAS III were obtained from the Sacramento Blood Bank Center. 4'-(4-Amino-2-oxa)butyl-4,5',8-trimethyl psoralen (Baxter Healthcare) was added to each platelet unit to achieve a final concentration of 150 µM. Platelet units (4-5) were pooled in a single PL-2410 plastic container and thoroughly mixed. The platelet pool was evenly divided into 4-5 l L PL2410 plastic containers each containing approximately 300 mL of the platelet mixture. Units were photochemically treated with 3.0 J/cm² UV-A and transferred into the appropriate removal device for the study. All experiments included a control platelet unit which was photochemically treated (150 µM psoralen, 3.0 J/cm² UVA) but was not contacted with a removal device.

### Device Preparation

Standard removal devices containing 2.5 g of Dowex XUS 43493 were prepared by Baxter Healthcare Corporation (Lot FX1032 D96F20042R). Experimental IADs were prepared with Hoechst-L493 media (Hoechst Celanese Corp.) that was supplied as roll stock. Media was measured and cut to give the appropriate adsorbent mass for each IAD (5.0 g, and 7.5 g). The cut media was placed in pouches constructed by impulse welding 30 µm polyester mesh (Tetko). Mesh pouches containing the media were autoclaved (121 °C, 20 min) and placed in sterile PL 2410 plastic containers. Alternatively, mesh pouches were place in PL 2410 containers, the containers were sealed, and the entire assembly was sterilized by gamma-irradiation to 2.5 MRad (SteriGenics, Corona, CA). Excess air was manually evacuated from devices using a syringe prior to transfer of the photochemically treated platelets.

### Adsorption Kinetics

Following photochemical treatment with 4'-(4-amino-2-oxa)butyl-4,5',8-trimethyl psoralen + UVA, the platelet mixtures were transferred to PL2410 plastic containers with removal devices. The devices were placed on a standard platelet incubator (Helmer) and agitated at 70 cycles/min at 22 °C. Samples of the platelet mixture were removed at 1 hour intervals for the first 8 hours of storage. These samples were stored at 4 °C and later analyzed for residual 4'-(4-amino-2-oxa)butyl-4,5',8-trimethyl psoralen by HPLC analysis. The assay involves an initial sample preparation which lyses the platelets and solubilizes the 4'-(4-amino-2-oxa)butyl-4,5',8-trimethyl psoralen and free photoproducts. The supernatant from the sample preparation is analyzed on a C-18 reverse phase column with a gradient of increasing methanol in KH₂PO₄ buffer.

### In vitro Platelet Function

Platelet mixtures were agitated in contact with the removal devices for 7 days. In one study, the platelet mixture was contacted with the IAD for 24 hours and transferred to sterile 1 L PL2410 plastic containers using a sterile tubing welder (Terumo SCD 312). Platelets were placed back in the platelet incubator and stored for the remainder of the 7 day storage period.

Following 5 or 7 days of storage, the platelet count and pH were determined for each platelet unit. The pH and pO2/pCO2 was measured using a CIBA-Corning model 238 Blood Gas Analyzer. The platelet count of each sample was determined using a Baker 9118+ Hematology analyzer.

Several assays were performed to evaluate in vitro platelet function. The shape change of platelet samples was monitored using a Chrono-Log model 500 VS whole blood aggregometer. Shape change was quantified as the ratio of maximum change in light transmission following addition of ADP relative to the change in light transmission following addition of EDTA.

The response of platelets to hypotonic stress was evaluated by the Hypotonic Shock Response (HSR) assay. The change in light transmission following addition of a hypotonic solution was measured using a Chrono-Log model 500 VS whole blood aggregometer. Data is reported as percent recovery from the hypotonic stress two minutes after absorbance reached its minimum value.

The ability of platelets to aggregate in response to ADP/collagen agonist was indicated by change in optical transmission as measured by a Chrono-Log model 500 VS whole blood aggregometer.

The status of the platelets was evaluated by scoring the platelet. Samples were blinded and morphology scores of 100 platelets were totaled for each sample. The highest possible score is 400 (Disc = 4, intermediate = 3, sphere = 2, dendrite = 1, balloon = 0).

Platelet activation as indicated by expression of p-selectin (Granular Membrane Protein, GMP-140) was measured. CD62 antibody was used for the test sample, mouse control IgG1 was used for the negative control, and CD62 antibody with phorbal myristate acetate (PMA) was used for the positive control. Samples were analyzed on a FACScan (Becton Dickinson). The percent of activation that is reported is relative to the positive control and is the difference between the test value and negative control value.

### Adsorption Kinetics

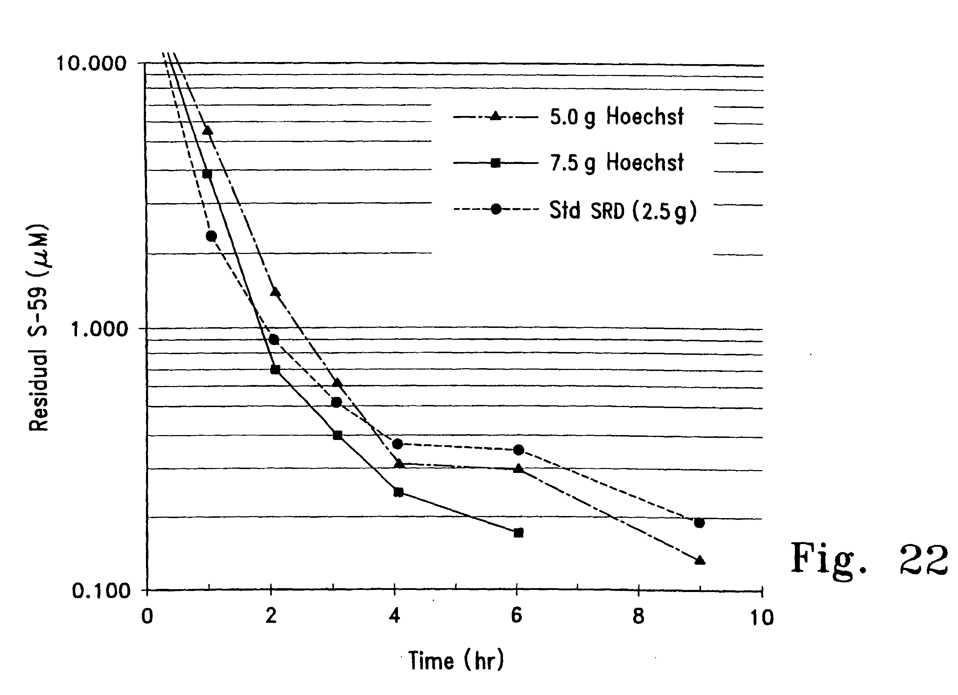

The impact of incorporating the adsorbent beads into a fiber matrix was investigated. Platelet units containing 4.0 x 10¹¹ platelets in 300 mL of 35% plasma, 65% PAS III were treated with 150 µM 4'-(4-amino-2-oxa)butyl-4,5',8-trimethyl psoralen + 3.0 J/cm² UVA. IADs were prepared from Hoechst-L493 with an adsorbent loading of 450 g/m². The IADs were sterilized by gamma irradiation. Following treatment with psoralen + UVA, the platelets were transferred to the removal devices. Samples were removed at 1 hour intervals and analyzed for residual 4'-(4-amino-2-oxa)butyl-4,5',8-trimethyl psoralen by HPLC. Figure 22 compares the kinetics for removal of 4'-(4-amino-2-oxa)butyl-4,5',8-trimethyl psoralen for IADs containing 5.0 g and 7.5 g Hoechst-L493 media to that of a standard removal device containing 2.5 g of loose beads. IADs contained either 5.0 g Hoechst-L493 (triangles), 7.5 g of Hoechst-L493 media (squares), or 2.5 g of loose Dowex L493 adsorbent beads (circles). The Hoechst-L493 media contained adsorbent beads at a loading of 450 g/m².

The kinetics of 4'-(4-amino-2-oxa)butyl-4,5',8-trimethyl psoralen adsorption were slower for the Hoechst media when compared to an equal mass of loose adsorbent beads. The removal device containing 2.5 g of loose beads achieved the lowest levels of residual 4'-(4-amino-2-oxa)butyl-4,5',8-trimethyl psoralen at short times (1 hr). However, at longer times, the IADs containing 7.5 g and 5.0 g of Hoechst-L493 media performed better. In this study the adsorption kinetics were relatively rapid with all three removal devices achieving the target of < 0.5 µM residual 4'-(4-amino-2-oxa)butyt-4,5',8-trimethyl psoralen in under 4 hours of contact.

Note that at long times (6-8 hr) the Hoechst-L493 IADs which contained a higher mass of adsorbent achieved lower levels of residual 4'-(4-amino-2-oxa)butyl-4,5',8-trimethyl psoralen. This observation suggests that the slower adsorption kinetics for the Hoechst-L493 IADs is a result of mass transport limitations (fluid flow vs. diffusion) and is not a result of loss of functional adsorption area due to fiber attachment. Additional studies indicate that Hoechst-L493 media with lower levels of adsorbent loading (200-300 g/m²) allows fluid to penetrate the media more readily resulting in faster kinetics of adsorption. Previous studies (data not shown) with Hoechst media containing Amberlite XAD-16 adsorbent at a loading of 160 g/m² indicated that adsorption kinetics were not affected by incorporation into a fiber matrix at a low level of loading.

### Platelet Yield and In Vitro Platelet Function

Data from two separate studies are presented in this section. The platelet units within each study were derived from a single pool so that the effect of the IAD media format, adsorbent mass, and contact time could be clearly evaluated.

The first study evaluated the effect of fiberization (Hoechst media) on yield and function of the platelets following extended contact (5 and 7 days). A total of four platelet units that were derived from a single pool were used in this study. The no-IAD control unit was treated with psoraten + UVA. Two of the platelet units were contacted with 5.0 g Hoechst-L493. One unit was contacted with the IAD for 24 hours before being transferred to an empty PL 2410 storage container. The other unit remained in contact with the IAD for the duration of the study. The Hoechst-L493 IADs were sterilized with steam (120 °C, 20 min). The standard removal device (2.5 g loose Dowex XUS-43493), which was obtained from Baxter. was sterilized by gamma-irradiation. Note that the platelets were not transferred away from the standard removal device following 8-16 hour contact as is typically the practice with the device that utilizes loose adsorbent particles. Results from platelet counts and in vitro function following 5 and 7 days of storage are summarized in Table 15A and Table 15B respectively.

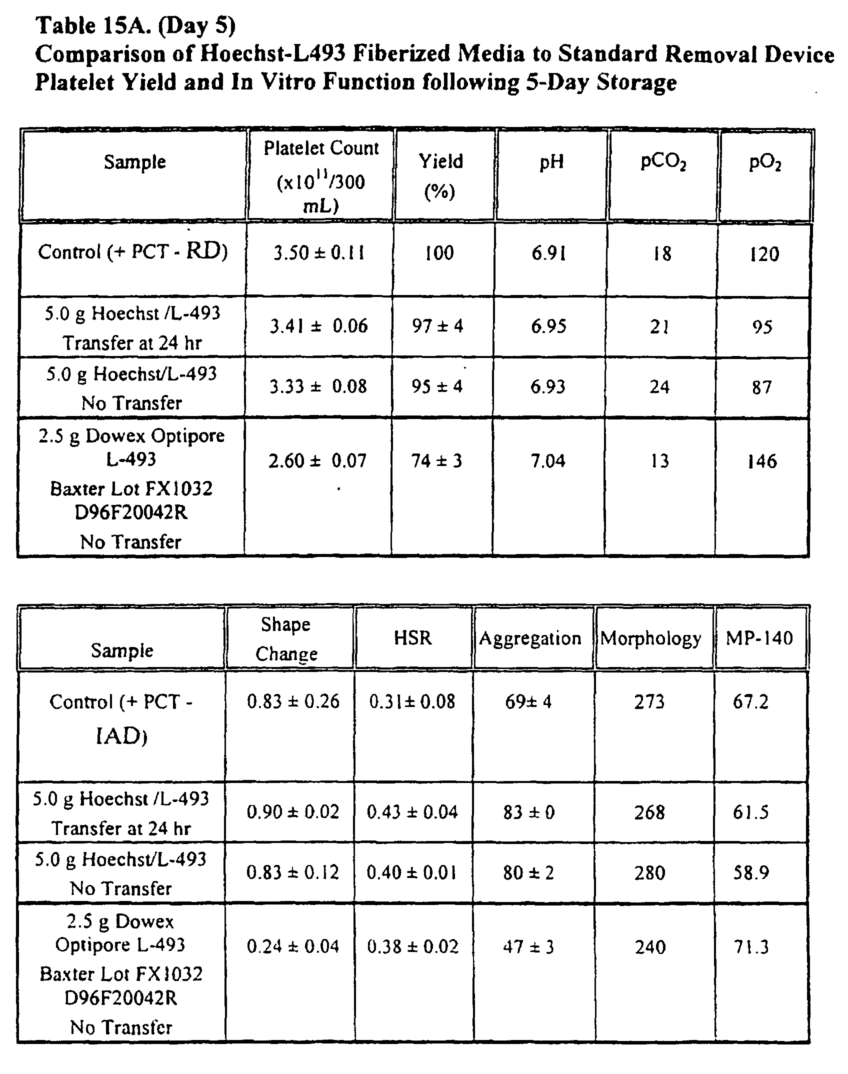

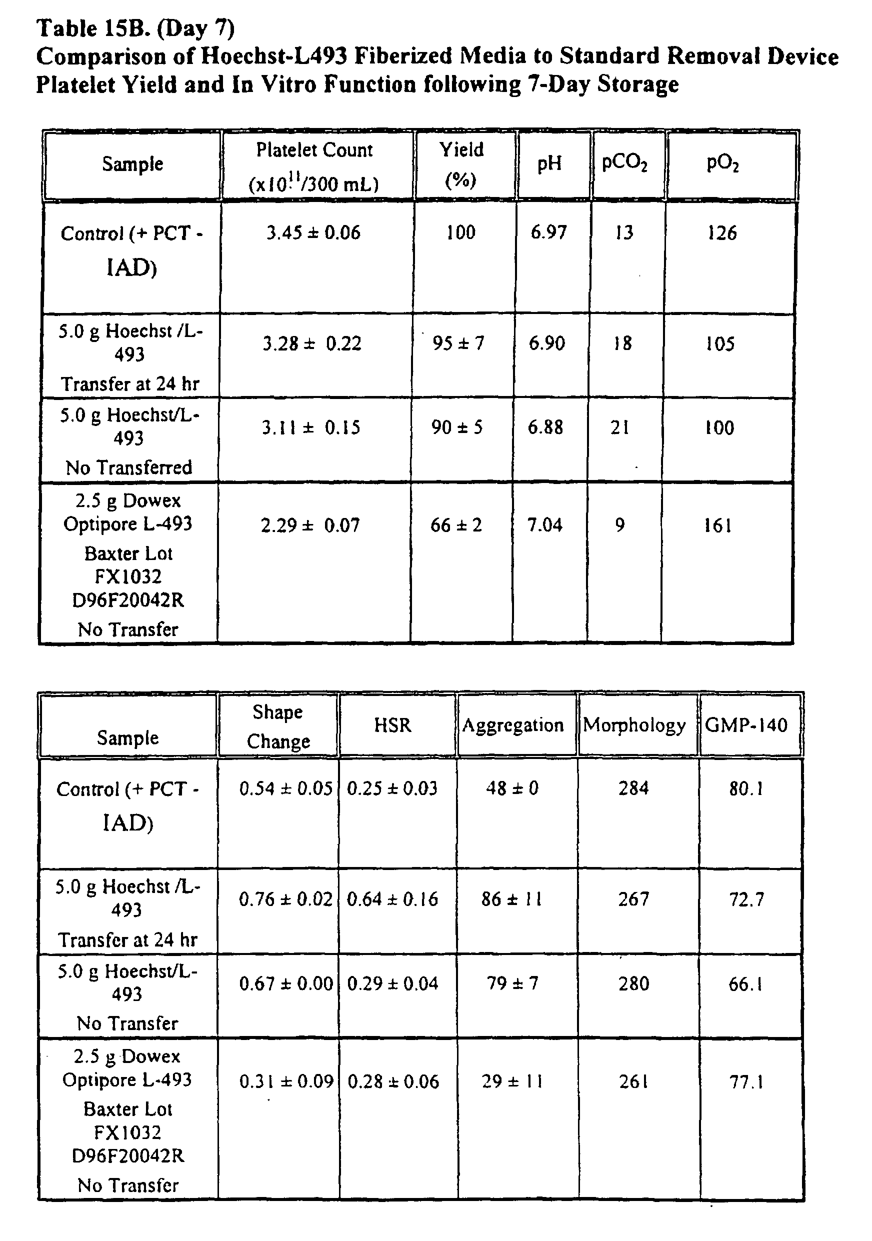

The platelet yields for the Hoechst-L493 IADs (5.0 g) were substantially better than the yield for the standard removal device (2.5 g). Losses of < 10% were achieved for 7 days of storage in continuous contact with the Hoechst-L493 IAD (5.0 g). Both platelet units that were treated with the Hoechst-L493 IADs displayed better performance in the shape change, aggregation, and GMP-140 assays than the no-IAD control. The platelets that remained in contact with the Hoechst-L493 IAD (5.0 g) for the entire 5 days showed comparable in vitro function to the platelets that were transferred after 24 hours of contact. Interestingly, the platelets that remained in contact with the Hoechst-L493 IAD performed better in the GMP-140 assay. The difference in performance between the two was even larger after 7 days. This observation suggests that contact with the IAD decreases the rate of p-selectin expression by platelets during storage.

The second study looked at IADs containing 5.0 g and 7.5 g of Hoechst-L493 media to determine if there was a significant decrease in platelet yield or in vitro function with a higher mass of media. In this study, the Hoechst-L493 IADs were sterilized by gamma irradiation. A standard removal device (2.5 g Dowex XUS-43493) was included in the study. Platelets remained in contact with the removal devices for the entire duration of the study. Results from platelet counts and in vitro function following 5 and 7 days of storage are summarized in Table 16A and Table 16B respectively.

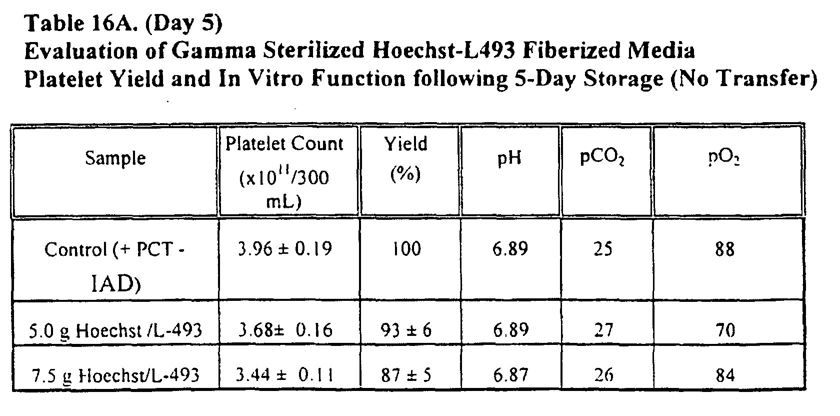

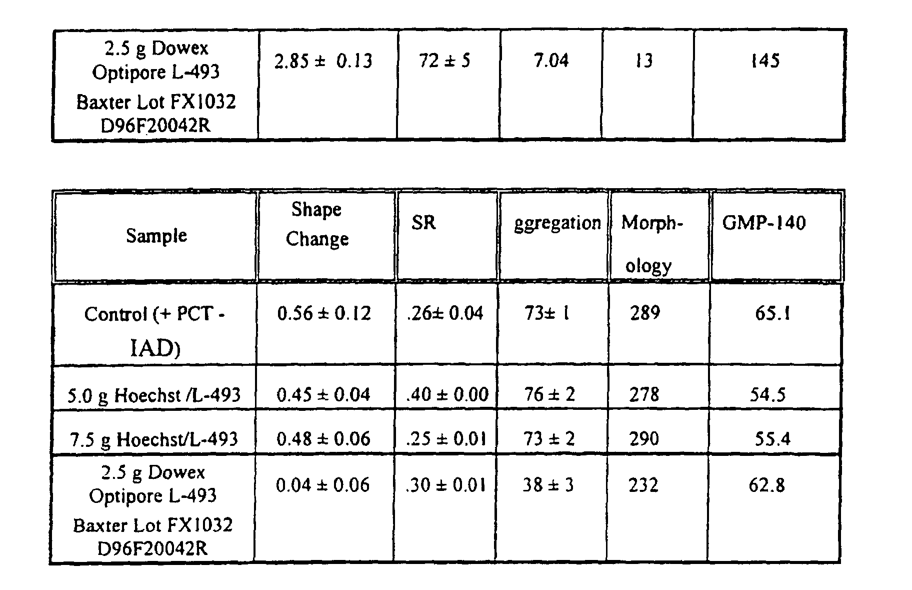

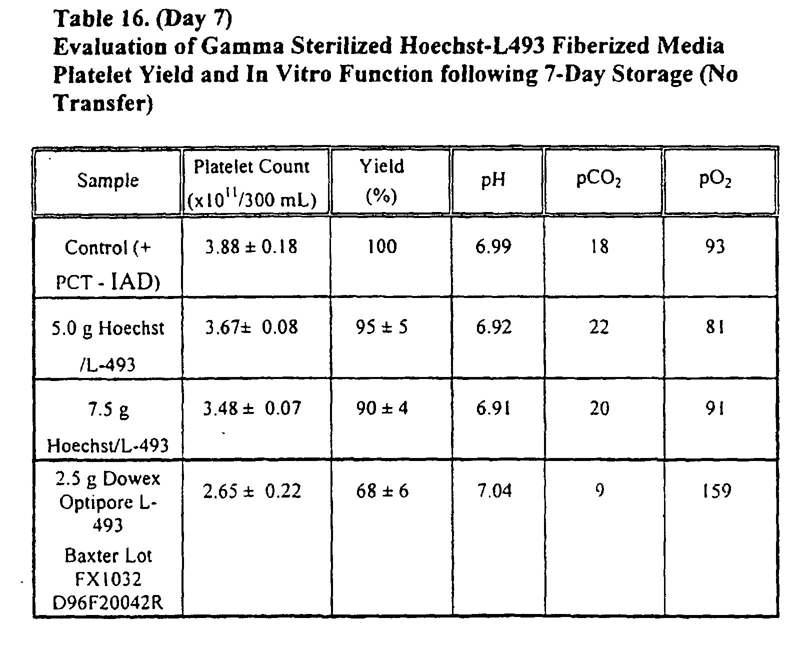

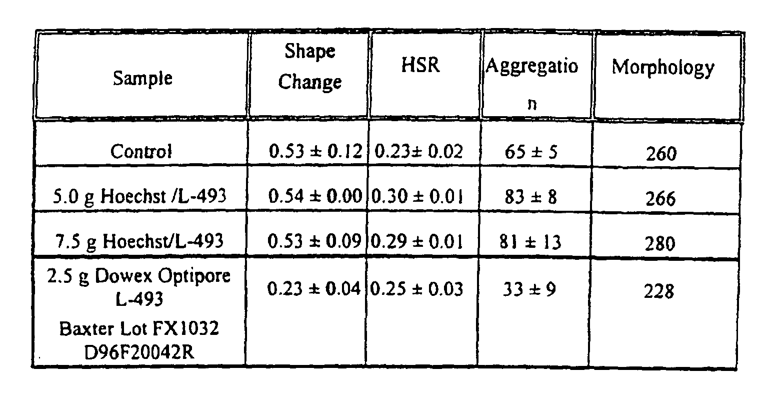

The results that are summarized in Tables 16A and 16B are similar to the results that were observed in the first study. Platelets that were contacted with the Hoechst-L493 IAD had significantly higher yields than platelets that were contacted with the standard removal device. Platelet yield was slightly lower for the 7.5 g Hoechst-L493 IAD relative to the 5.0 g IAD. Platelets that were treated with the Hoechst-L493 IADs performed comparably to the control platelets in all in vitro assays. Platelets that were contacted with the Hoechst-L493 IADs showed improved performance in the aggregation assay relative to the no-IAD control on day 7. The GMP-140 assay was not performed on day 7.

IADs that were prepared with Hoechst-L493 media (5.0, 7.0 g) resulted in superior in vitro function when compared to standard removal devices (2.5 g loose XUS-43493) stored in contact with the platelets for 5 days. Moreover, platelets that were treated with 150 µM 4'-(4-amino-2-oxa)butyl-4,5',8-trimethyl psoralen + 3.0 J/cm² UVA showed improved in vitro function as indicated by shape change, aggregation, and GMP-140 assays when contacted with Hoechst-L493 IADs (5.0 g) for 5 and 7 days. An additional study that compared 5-7 day storage for platelets (no psoralen/UVA) with and without IAD (50.g Hoechst-L493) demonstrated that storage with an IAD may improve platelet performance as indicated by in vitro function assays.

## Claims

1. An adsorbent medium for reducing the concentration of a small organic compound in a blood product while substantially maintaining a desired biological activity of the blood product comprising (1) highly porous adsorbent particles capable of adsorbing the small organic compound, and (2) a sintered polymeric inert matrix in which the adsorbent particles are immobilized.

2. An adsorbent medium according to claim 1, wherein the matrix comprises a polyolefin.

3. An adsorbent medium according to claim 1 or 2, wherein the small organic compound comprises a pathogen inactivating compound.

4. An adsorbent medium according to claim 1, 2 or 3, wherein said small organic compound is selected from psoralens or its photo activation products, psoralen derivatives, thiazine dyes, acridines, acridine derivatives and xanthene dyes.

5. An adsorbent medium according to claim 4, wherein said small organic compound is selected from the group consisting of 4'-aminomethy-4, 5', 8 trimethylpsoralen, 4'-(4-amino-2-oxa)butyl-4,5', 8-trimethyl psoralen, 8-methoxypsoralen, halogenated psoralens, isopsoralens and psoralens linked to quaternary amines, 5'-bromomethyl-4, 4',8-trimethylpsoralen, 4'-brornomethyl-4,5',8-trimethylpsoralen, 4'-(4-ainino-2-aza)butyl-4,5',8-trimethylpsoralen, 4'-(2-aminoethyl)-4,5',8-trimethylpsoralen, 4'-(5-amino-2-oxa)pentyl-4,5',8-trimethylpsoralen, 4'-(5-amino-2-aza)pentyl-4,5',8-trimethylpsoralen, 4'-(6-amino-2-aza)hexyl-4,5',8-trimethylpsoralen, 4'-(7-amino-2,5-oxa)heptyl-4,5',8-trimethylpsoralen, 4'-(12-amino-8-aza-2,5-dioxa)dodecyl-4,5',8-trimethylpsoralen, 4'-(13-amino-2-aza-6,11-dioxa)tridecyl-4,5',8-trimethylpsoralen, 4'-(7-amino-2-aza)heptyl-4,5',8-trimethylpsoralen, 4'-(7-amino-2-aza-5-oxa)heptyl-4,5',8-trimethylpsoralen, 4'-(9-amino-2,6-diaza)nonyl-4,5',8-trimethylpsoralen, 4'-(8-amino-5-aza-2-oxa)octyl-4,5',8-trimethylpsoralen, 4'-(9-amino-5-aza-2-oxa)nonyl-4,5',8-trimethylpsoralen, 4'-(14-amino-2,6,11-triaza)tetradecyl-4,5',8-trimethylpsoralen, 5'-(4-amino-2-aza)butyl-4,4',8-trimethylpsoralen, 5'-(6-amino-2-aza)hexyl-4,4',8-trimethylpsoralen and 5'-(4-amino-2-oxa)butyl-4,4',8-trimethylpsoralen.

6. An adsorbent medium according to claim 4 wherein the small organic molecule comprises N-(9-acridinyl)-β-alanine.

7. An adsorbent medium according to any of claims 1-6, wherein the adsorbent particles are synthetic polymeric adsorbent particles.

8. An adsorbent medium according to claim 7, wherein the adsorbent particles comprise a hydrophobic resin.

9. An adsorbent medium according to claim 7 or 8, wherein the adsorbent particles comprise a macroreticular resin.

10. An adsorbent medium according to any of claims 7 to 9, wherein the adsorbent particles comprise a polyaromatic resin.

11. An adsorbent medium according to claim 10, wherein the polyaromatic resin comprises a hypercrosslinked resin.

12. An adsorbent medium according to any of claims 7 to 11, wherein the resin has a pore size of the particles is between about 25 and about 800Å.

13. An adsorbent medium according to any of claims 1-12, wherein the adsorbent particles have an internal surface area between about 300 and 1100 m²/g.

14. An adsorbent medium according to any of claims 1-13, wherein the adsorbent medium forms a flow-through device and wherein the adsorbent particles are between about 10 micron and about 200 micron in diameter.

15. An adsorbent medium according to claim 14, wherein the amount of absorbent particles per area is between about 300 g/m² and about 1100 g/m².

16. An adsorbent medium according to claim 14, wherein the adsorbent medium contains about 20-70% of the adsorbent particles by weight.

17. An adsorbent medium according to any of claims 1-13, wherein the adsorbent medium forms a batch device, and wherein the adsorbent particles have a diameter between about 300 and about 1200 micron.

18. An adsorbent medium according to claim 17, wherein the amount of absorbent particles per area is between about 100 g/m² and about 500 g/m².

19. An adsorbent medium according to claim 17 wherein the adsorbent medium contains about 25-85% adsorbent particles by weight.

20. An adsorbent medium according to any of claims 17-19 wherein the device comprises a blood bag.

21. An adsorbent medium according to any of claims 17-20 and further comprising a mesh enclosure, wherein the adsorbent medium is contained within the mesh enclosure.

22. A device comprising an adsorbent medium according to any of the preceding claims and a blood product containing the small organic compound and wherein the adsorbent particles are in contact with the blood product containing the small organic compound.

23. A device according to claim 22 wherein the small organic compound comprises at least one compound selected from the group consisting of psoralens and psoralen derivatives.

24. A device according to claim 22 wherein the small organic compound comprises 4'-(4 -amino-2-oxa)butyl-4,5',8-trimethylpsoralen.

## Patentansprüche

1. Adsorptionsmedium zur Verminderung der Konzentration einer kleinen organischen Verbindung in einem Blutprodukt, wobei eine gewünschte biologischen Aktivität des Blutprodukts im wesentlichen aufrechterhalten bleibt, welches umfasst: (1) hochporöse Absorbenspartikel, die zum Adsorbieren der kleinen organischen Verbindung fähig sind, und (2) eine gesinterte polymere inerte Matrix, in welcher die Adsorbenspartikel immobilisiert sind.

2. Adsorptionsmedium nach Anspruch 1, worin die Matrix ein Polyolefin umfasst.

3. Adsorptionsmedium nach Anspruch 1 oder 2, worin die kleine organische Verbindung eine Pathogen-inaktivierende Verbindung umfasst.

4. Adsorptionsmedium nach Anspruch 1, 2 oder 3, worin die kleine organische Verbindung ausgewählt ist aus Psoralenen oder seinen Photoaktivierungsprodukten, Psoralen-Derivaten, Thiazin-Farbstoffen, Acridinen, Acridin-Derivaten und Xanthen-Farbstoffen.

5. Adsorptionsmedium nach Anspruch 4, worin die kleine organische Verbindung ausgewählt ist aus der Gruppe, bestehend aus 4'-Aminomethyl-4,5'-8-trimethylpsoralen, 4'-(4-Amino-2-oxa)butyl-4,5',8-Trimethylpsoralen, 8-Methoxypsoralen, halogenierten Psoralenen, Isopsoralenen und an quaternäre Amine geknüpften Psoralenen, 5'-Brommethyl-4,4'-8-trimethylpsoralen, 4'-Brommethyl-4,5',8-trimethylpsoralen, 4'-(4-Amino-2-aza)butyl-4,5',8-trimethylpsoralen, 4'-(2-Aminoethyl)-4,5',8-trimethylpsoralen, 4'-(5-Amino-2-oxa)pentyl-4,5',8-trimethylpsoralen, 4'-(5-Amino-2-aza)pentyl-4,5',8-trimethylpsoralen, 4'-(6-Amino-2-aza)hexyl-4,5',8-trimethylpsoralen, 4'-(7-Amino-2,5-oxa)heptyl-4,5',8-trimethylpsoralen, 4'-(12-Amino-8-aza-2,5-dioxa)dodecyl-4,5',8-trimethylpsoralen, 4'-(13-Amino-2-aza-6,11-dioxa)tridecyl-4,5',8-trimethylpsoralen, 4'-(7-Amino-2-aza)heptyl-4,5',8-trimethylpsoralen, 4'-(7-Amino-2-aza-5-oxa)heptyl-4,5',8-trimethylpsoralen, 4'-(9-Amino-2,6-diaza)nonyl-4,5',8-trimethylpsoralen, 4'-(8-Amino-5-aza-2-oxa)octyl-4,5',8-trimethylpsoralen, 4'-(9-Amino-5-aza-2-oxa)nonyl-4,5',8-trimethylpsoralen, 4'-(14-Amino-2,6,11-triaza)tetradecyl-4,5',8-trimethylpsoralen, 5'-(4-Amino-2-aza)butyl-4,4',8-trimethylpsoralen, 5'-(6-Amino-2-aza)hexyl-4,4',8-trimethylpsoralen und 5'-(4-Amino-2-oxa)butyl-4,4',8-trimethylpsoralen.

6. Adsorptionsmedium nach Anspruch 4, worin das kleine organische Molekül N-(9-Acridinyl)-β-alanin umfasst.

7. Adsorptionsmedium nach einem der Ansprüche 1-6, worin die Adsorbenspartikel synthetische polymere Adsorbenspartikel sind.

8. Adsorptionsmedium nach Anspruch 7, worin die Adsorbenspartikel ein hydrophobes Harz umfassen.

9. Adsorptionsmedium nach Anspruch 7 oder 8, worin die Adsorbenspartikel ein makroporöses Harz umfassen.

10. Adsorptionsmedium nach einem der Ansprüche 7 bis 9, worin die Adsorbenspartikel ein polyaromatisches Harz umfassen.

11. Adsorptionsmedium nach Anspruch 10, worin das polyaromatische Harz ein hypervernetztes Harz umfasst.

12. Adsorptionsmedium nach einem der Ansprüche 7 bis 11, worin das Harz eine Porengröße der Partikel von zwischen etwa 25 und etwa 800A aufweist.

13. Adsorptionsmedium nach einem der Ansprüche 1-12, worin die Adsorbenspartikel einen inneren Oberflächenbereich von zwischen etwa 300 und 1100 m²/g aufweisen.

14. Adsorptionsmedium nach einem der Ansprüche 1-13, worin das Adsorptionsmedium ein Durchflussgerät bildet und worin die Adsorbenspartikel einen Durchmesser von zwischen etwa 10 µm und etwa 200 µm aufweisen.

15. Adsorptionsmedium nach Anspruch 14, worin die Menge der Adsorbenspartikel pro Fläche zwischen etwa 300 g/m² und etwa 1100 g/m² beträgt.

16. Adsorptionsmedium nach Anspruch 14, worin das Adsorptionsmedium etwa 20-70 Gew.-% an Adsorbenspartikeln enthält.

17. Adsorptionsmedium nach einem der Ansprüche 1-13, worin das Adsorptionsmedium ein Chargengerät bildet, und worin die Adsorbenspartikel einen Durchmesser von zwischen etwa 300 und etwa 1200 µm aufweisen.

18. Adsorptionsmedium nach Anspruch 17, worin die Menge an Adsorbenspartikeln pro Fläche zwischen etwa 100 g/m² und etwa 500 g/m² beträgt.

19. Adsorptionsmedium nach Anspruch 17, worin das Adsorptionsmedium etwa 25-85 Gew.-% an Adsorbenspartikeln enthält.

20. Adsorptionsmedium nach einem der Ansprüche 17-19, worin das Gerät einen Blutbeutel umfasst.

21. Adsorptionsmedium nach einem der Ansprüche 17-20, das außerdem eine Maschenumhüllung umfasst, wobei das Adsorptionsmedium innerhalb der Maschenumhüllung enthalten ist.

22. Gerät, umfassend ein Adsorptionsmedium nach einem der vorangehenden Ansprüche und ein Blutprodukt, das die kleine organische Verbindung enthält, und worin die Adsorbenspartikel in Kontakt mit dem Blutprodukt sind, das die kleine organische Verbindung enthält.

23. Gerät nach Anspruch 22, worin die kleine organische Verbindung mindestens eine Verbindung umfasst, gewählt aus der Gruppe, bestehend aus Psoralenen und Psoralen-Derivaten.

24. Gerät nach Anspruch 22, worin die kleine organische Verbindung 4'-(4-Amino-2-oxa)butyl-4,5'-8-trimethylpsoralen umfasst.

## Revendications

1. Milieu adsorbant pour réduire la concentration d'un petit composé organique dans un produit sanguin, tout en maintenant sensiblement une activité biologique souhaitée du produit sanguin, comprenant (1) des particules adsorbantes à forte porosité, capables d'adsorber le petit composé organique, et (2) une matrice inerte polymère frittée, dans laquelle les particules adsorbantes sont immobilisées.

2. Milieu adsorbant selon la revendication 1, dans lequel la matrice comprend une polyoléfine.

3. Milieu adsorbant selon la revendication 1 ou 2, dans lequel le petit composé organique comprend un composé inactivant les agents pathogènes.

4. Milieu adsorbant selon la revendication 1, 2 ou 3, dans lequel ledit petit composé organique est choisi parmi les psoralènes ou leurs produits de photoactivation, les dérivés du psoralène, les colorants thiaziniques, les acridines, les dérivés de l'acridine et les colorants xanthéniques.

5. Milieu adsorbant selon la revendication 4, dans lequel ledit petit composé organique est choisi dans l'ensemble consistant en le 4'-aminométhyl-4,5',8-triméthylpsoralène, le 4'-(4-amino-2-oxa)butyl-4,5',8-triméthylpsoralène, le 8-méthoxypsoralène, les psoralènes halogénés, les isopsoralènes et les psoralènes liés à des amines quaternaires, le 5'-bromométhyl-4,4',8-triméthylpsoralène, le 4'-bromométhyl-4,5',8-triméthylpsoralène, le 4'-(4-amino-2-aza)butyl-4,5',8-triméthylpsoralène, le 4'-(2-aminoéthyl)-4,5',8-triméthylpsoralène, le 4'-(5-amino-2-oxa)pentyl-4,5',8-triméthylpsoralène, le 4'-(5-amino-2-aza)pentyl-4,5',8-triméthylpsoralène, le 4'-(6-amino-2-aza)hexyl-4,5',8-triméthylpsoralène, le 4'-(7-amino-2,5-oxa)heptyl)-4,5',8-triméthylpsoralène, le 4'-(12-amino-8-aza-2,5-dioxa)dodécyl-4,5',8-triméthylpsoralène, le 4'-(13-amino-2-aza-6,11-dioxa)tridécyl-4,5',8-triméthylpsoralène, le 4'-(7-amino-2-aza)heptyl-4,5',8-triméthylpsoralène, le 4'-(7-amino-2-aza-5-oxa)heptyl-4,5',8-triméthylpsoralène, le 4'-(9-amino-2,6-diaza)nonyl-4,5',8-triméthylpsoralène, le 4'-(8-amino-5-aza-2-oxa)octyl-4,5',8-triméthylpsoralène, le 4'-(9-amino-5-aza-2-oxa)nonyl-4,5',8-triméthylpsoralène, le 4'-(14-amino-2,6,11-triaza)tétradécyl-4,5',8-triméthylpsoralène, le 5'-(4-amino-2-aza)butyl-4,4',8-triméthylpsoralène, le 5'-(6-amino-2-aza)hexyl-4,4',8-triméthylpsoralène et le 5'-(4-amino-2-oxa)butyl)-4,4',8-triméthylpsoralène.

6. Milieu adsorbant selon la revendication 4, dans lequel la petite molécule organique comprend la N-(9-acridinyl)-β-alanine.

7. Milieu adsorbant selon l'une quelconque des revendications 1 à 6, dans lequel les particules adsorbantes sont des particules adsorbantes polymères synthétiques.

8. Milieu adsorbant selon la revendication 7, dans lequel les particules adsorbantes comprennent une résine hydrophobe.

9. Milieu adsorbant selon la revendication 7 ou 8, dans lequel les particules adsorbantes comprennent une résine macroréticulaire.

10. Milieu adsorbant selon l'une quelconque des revendications 7 à 9, dans lequel les particules adsorbantes comprennent une résine polyaromatique.

11. Milieu adsorbant selon la revendication 10, dans lequel la résine polyaromatique comprend une résine hyperréticulée.

12. Milieu adsorbant selon l'une quelconque des revendications 7 à 11, dans lequel la résine a des particules dont le diamètre des pores est compris entre environ 25 et environ 800 Å.

13. Milieu adsorbant selon l'une quelconque des revendications 1 à 12, dans lequel les particules adsorbantes ont une aire spécifique interne comprise entre environ 300 et 1100 m²/g.

14. Milieu adsorbant selon l'une quelconque des revendications 1 à 13, lequel milieu adsorbant formant un dispositif à passage direct, où les particules adsorbantes ont un diamètre compris entre 10 et environ 200 micromètres.

15. Milieu adsorbant selon la revendication 14, dans lequel la quantité de particules adsorbantes par unité d'aire est comprise entre environ 300 et environ 1100 g/m².

16. Milieu adsorbant selon la revendication 14, lequel milieu adsorbant contenant environ 20-70 % en poids de particules adsorbantes.

17. Milieu adsorbant selon l'une quelconque des revendication 1 à 13, lequel milieu adsorbant formant un dispositif à lot discontinu, et où les particules adsorbantes ont un diamètre compris entre environ 300 et environ 1200 micromètres.

18. Milieu adsorbant selon la revendication 17, dans lequel la quantité de particules adsorbantes par unité d'aire est comprise entre environ 100 et environ 500 g/m².

19. Milieu adsorbant selon la revendication 17, lequel milieu adsorbant contenant environ 25-85 % en poids de particules adsorbantes.

20. Milieu adsorbant selon l'une quelconque des revendications 17 à 19, dans lequel le dispositif comprend une poche à sang.

21. Milieu adsorbant selon l'une quelconque des revendications 17 à 20, et comprenant en outre une enveloppe maillée, le milieu adsorbant étant confiné à l'intérieur de l'enveloppe maillée.

22. Dispositif comprenant un milieu adsorbant selon l'une quelconque des revendications précédentes et un produit sanguin contenant le petit composé organique, les particules adsorbantes étant en contact avec le produit sanguin contenant le petit composé organique.

23. Dispositif selon la revendication 22, dans lequel le petit composé organique comprend au moins un composé choisi dans l'ensemble consistant en les psoralènes et les dérivés du psoralène.

24. Dispositif selon la revendication 22, dans lequel le petit composé organique comprend du 4'-(4-amino-2-oxa)butyl-4,5',8-triméthylpsoralène.
